# EUROPEAN PATENT APPLICATION

(11) **EP 2 377 952 A1**
(43) Date of publication of application: **19.10.2011**
(21) Application number: 11162375.7
(22) Date of filing: 25.04.2008
(51) Int. Cl.: C12Q 1/68

(54) **Methods for diagnosing and treating astrocytomas**

(30) Priority: 26.04.2007 US 926263 P
(62) Divisional of application: 08826602.8
(71) Applicant: LUDWIG INSTITUTE FOR CANCER RESEARCH, New York, New York 10158 (US); Universidade De São Paulo - Usp, 05.508-900 São Paulo - SP (BR); Sociedade Beneficente Israelita Brasileira Hospital Albert Einstein, 05651-901 Sao Paulo (BR)
(72) Inventor: Caballero, Otavia, New York, 10021 (US); Marie Suely, Kazue Nagahashi, 01246-903, Sao Paulo (BR); Oba Shinjo, Sueli Mieko, 01246-903, Sao Paulo (BR); Okamoto, Oswaldo Keith, 04023-900, Sao Paulo (BR)
(74) Representative: Gill, Siân Victoria

(57) **Abstract**

The invention relates to the identification of astrocytoma markers, astrocytoma stem cells and markers of such stem cells, and diagnostic, prognostic and therapeutic methods based on an understanding of the markers and cells.

## Description

### Related Applications

This application claims the benefit under 35 U.S.C. § 119(e) of United States provisional patent application serial number 60/926,263, filed April 26, 2007, the content of which is incorporated herein by reference in its entirety.

### Field of the Invention

The invention relates to the identification of genes that are upregulated in astrocytomas, which can be used in diagnosis, staging of disease, treatment and monitoring of treatment. The invention also relates to the identification of genes that are found to be upregulated or downregulated in astrocytoma stem cells, which also can be used in diagnosis, staging of disease, treatment and monitoring of treatment.

### Background of the Invention

Astrocytomas are gliomas of astrocytic origin and constitute the most common type of primary brain tumor. They are classified, according to the World Health Organization (WHO), into pilocytic astrocytomas (PA) (grade I, GI), low grade astrocytomas (grade II), anaplastic astrocytomas (grade III) and glioblastoma multiforme (GBM) (grade IV)^{1, 2}, PA · (GI), the most common glioma in children and young adults, has a good prognosis due to it being more circumscribed and thus curable by total surgical resection. In contrast, astrocytomas grade II to grade IV are highly infiltrating and are hence named diffuse astrocytomas. These are generally not curable by resection. Despite efforts to develop novel therapies, the median survival rate of patients with GBM rarely exceeds 12 months³, reflecting the resistance of these tumors not only to surgical approaches but also chemotherapy and radiation therapy. Although multiple genetic alterations including chromosomal abnormalities, oncogene activation and tumor suppressor gene inactivation⁴ have already been identified in astrocytomas, the dismal prospects for patients with this disease render the identification of additional therapeutic targets an important objective.

Gene expression patterns provide insight into the nature of the molecular alterations that lead to the establishment and progression of tumors. Genome-wide technologies such as cDNA microarrays, large scale EST sequencing⁵, serial analysis of gene expression⁶ (SAGE) and massive parallel signature sequencing^{7, 8} (MPSS) have become powerful tools for investigating the molecular biology of malignancy.

Despite recent advances in cancer research, the overall survival rate of patients with gliomas, the most common central nervous system tumors, has remained minimal. This scenario is particularly distressing for patients with glioblastoma multiforme (GBM), in which median survival stands below 16 months even with the combined use of resection and radiotherapy⁴³. Currently, there is no effective treatment for this highly aggressive and infiltrative tumor, and relatively little is known about the molecular processes leading to its formation. Progress in clinical management of malignant gliomas is critical and relies on the development of alternative therapeutic approaches. Such development, however, can not be achieved without understanding the genetic and molecular events leading to oncogenic activation. With the advent of genomic technologies such as Serial Analysis of Gene Expression and DNA microarrays, the study of genes and pathways related to cancer pathogenesis has been facilitated. Current studies have successfully identified tumor-associated molecular signatures in gliomas^{44, 45, 46, 47}, with potential clinical exploitation.

In addition, comparative analysis of cancer stem cells with their neoplastic and non-neoplastic counterparts should provide a better understand of the underlying molecular events leading to transformation and tumor dissemination.

A need therefore exists for better identification of changes in gene expression of cells that give rise to and/or propagate astrocytoma to facilitate improved detection and therapy of astrocytoma.

### Summary of the Invention

We have performed cDNA microarray analyses to identify gene expression differences between highly invasive glioblastoma multiforme and typically benign pilocytic astrocytomas. In this study, we used oligonucleotide microarrays to compare the expression pattern of 10,000 genes in grade IV (GBM) and grade I (PA) astrocytic tumors in order to identify genes associated with invasion and proliferation. Amongst the most highly over-expressed genes in GBM, was *MELK* which has been reported to be up-regulated in several cancer types relative to normal tissues and which regulates multipotent neural progenitors^{9, 10}. Thus, to investigate the potential oncogenic role of *MELK* in astrocytomas, we studied mechanisms of *MELK* over-expression by gene amplification, promoter methylation analysis, and the biological consequences of *MELK* down-regulation for tumor cell proliferation, anchorage independent growth, motility and apoptosis.

We then extended the identification to additional genes and determined the relative expression of such genes in non-neoplastic tissue (N), astrocytoma tissues (grades I-IV) and glioblastoma cell lines. The genes identified all are markers for tumorigenesis (e.g., Grade IV/N is higher than Grade I/N) and certain genes (e.g., LOX, HOXA5, KIAA0101, Melk and ASPM) are clearly markers for the progression of the disease (their expression levels gradually increase in relation with the progression of the malignancy).

In addition, we report here a molecular signature comprised by genes with exclusive aberrant expression in CD133+ cells, a reported subpopulation of tumorigenic stem-like cells, isolated from human glioblastomas. Microarrays covering 55,000 transcripts were used to compare gene expression profiles in purified subpopulations of CD133+ and CD133- GBM cells. Sixteen genes, many of which not previously associated with astrocytomas, were found aberrantly expressed in CD133+ cells, but not in CD133-, when compared with corresponding non-neoplastic controls. Up-regulation of three of such genes, *E2F2*, *CD99* and *HOXC9*, was detected in a set of 54 astrocytomas of different grades and significantly correlated with malignancy. Due to their distinctive expression in CD 133+ cells, the use of *CD99, E2F2* and *HOXC9* as therapeutic targets for tumor eradication is suggested.

According to one aspect of the invention, methods of diagnosing an astrocytoma in a subject are provided. The methods include determining a level of a KIAA0101, LOX, HOXA5, PLP2, DKFZp762E1312, CD99, HOXC9 and/or E2F2 gene product in a biological sample isolated from the subject, and comparing the level in the sample to a reference/control level. If the level in the sample is higher than the reference/control level, then the subject is indicated as having an astrocytoma. In some embodiments, the astrocytoma is a pilocytic astrocytoma (PA; grade I), low grade astrocytoma (grade II), anaplastic astrocytoma (grade III) or glioblastoma multiforme (GBM; grade IV).

In some embodiments, the gene product is a mRNA. In further embodiments, the level of gene product in the sample is determined by nucleic acid amplification; preferably, the nucleic acid amplification is polymerase chain reaction (PCR), reverse transcribed PCR (RT-PCR), real-time RT-PCR, or quantitative real-time RT-PCR. In other embodiments, the level of gene product in the sample is determined by a nucleic acid hybridization method; preferably, the nucleic acid hybridization method is a microarray or a membrane hybridization method, or is performed using a nucleic acid probe or primer, or the nucleic acid probe or primer is detectably labeled.

In some embodiments, the gene product is a polypeptide. In further embodiments, the level of gene product in the sample is determined by a polypeptide binding method, preferably an immunological detection method. In some preferred embodiments, the immunological detection method uses an antibody or an antigen binding fragment thereof; optionally the antibody or an antigen binding fragment thereof is detectably labeled.

In still other embodiments, the difference between the level of the gene product in the sample and the reference/control level is at least about 1%, 2%, 3%, 5%, 10%, 15%, 20%, 25%, 30%, 35%, 40%, 50%, 60%, 70%, 80%, 90%, 100%, 110%, 120%, 130%, 140%, 150%, 175%, 200%, 250%, 300%, 350%, 400%, 450%, 500%, 600%, 700%, 800%, 900%, or 1000%.

In preferred embodiments, the gene product is a KIAA0101 gene product, a LOX gene product, a HOXA5 gene product, a PLP2 gene product, a DKFZp762E1312 gene product, a CD99 gene product, a HOXC9 gene product, or a E2F2 gene product.

In some embodiments, the sample is a body fluid, a body effusion, cell or a tissue, preferably a brain cell or tissue.

According to another aspect of the invention, methods of staging an astrocytoma tumor in a subject are provided. The methods include determining a level of a KIAA0101, ASPM, LOX, HOXA5, COL6A2, PLP2, DKFZp762E1312, MELK, HOXC9 and/or E2F2 gene product in a sample of the astrocytoma tumor isolated from the subject and comparing the level of the gene product in the sample to a reference/control level to determine that the tumor is an astrocytoma of a particular stage.

In some embodiments, the gene product is a mRNA. In further embodiments, the level of gene product in the sample is determined by nucleic acid amplification; preferably, the nucleic acid amplification is polymerase chain reaction (PCR), reverse transcribed PCR (RT-PCR), real-time RT-PCR, or quantitative real-time RT-PCR. In other embodiments, the level of gene product in the sample is determined by a nucleic acid hybridization method; preferably, the nucleic acid hybridization method is a microarray or a membrane hybridization method, or is performed using a nucleic acid probe or primer, or the nucleic acid probe or primer is detectably labeled.

In some embodiments, the gene product is a polypeptide. In further embodiments, the level of gene product in the sample is determined by a polypeptide binding method, preferably an immunological detection method. In some preferred embodiments, the immunological detection method uses an antibody or an antigen binding fragment thereof; optionally the antibody or an antigen binding fragment thereof is detectably labeled.

In still other embodiments, the difference between the level of the gene product in the sample and the reference/control level is at least about 1%, 2%, 3%, 5%, 10%, 15%, 20%, 25%, 30%, 35%, 40%, 50%, 60%, 70%, 80%, 90%, 100%, 110%, 120%, 130%, 140%, 150%, 175%, 200%, 250%, 300%, 350%, 400%, 450%, 500%, 600%, 700%, 800%, 900%, or 1000%.

In preferred embodiments, the gene product is a KIAA0101 gene product, a ASPM gene product, a LOX gene product, a HOXA5 gene product, a COL6A2 gene product, a PLP2 gene product, a DKFZp762E 1312 gene product, a CD99 gene product, a MELK gene product, a HOXC9 gene product, or a E2F2 gene product.

In some embodiments, the sample is a body fluid, a body effusion, cell or a tissue, preferably a brain cell or tissue.

In some embodiments, the astrocytoma is a pilocytic astrocytoma (PA; grade I), low grade astrocytoma (grade II), anaplastic astrocytoma (grade III) or glioblastoma multiforme (GBM; grade IV).

In another aspect of the invention, methods for monitoring treatment in a subject having an astrocytoma. The methods include determining a level of a KIAA0101, ASPM, LOX, HOXA5, COL6A2, PLP2, DKFZp762E1312, MELK, CD99, HOXC9 and/or E2F2 gene product in a first biological sample of an astrocytoma isolated from the subject, determining a level of the same gene product as in the first biological sample; in a second biological sample of an astrocytoma isolated from the subject at a second, later time than the first sample, and comparing the level of the gene product in the first sample and the level of the gene product in the second sample to determine if the astrocytoma in the second sample is of a different grade that the astrocytoma in the first sample. In some embodiments, if the grade of the astrocytoma in the second sample is different than in the first sample, then a treatment of the subject is changed to address the grade of the astrocytoma. IN other embodiments, the level of gene product in the first sample and level of gene product in the second sample indicates that the first sample and the second sample independently is pilocytic astrocytoma (PA; grade I), low grade astrocytoma (grade II), anaplastic astrocytoma (grade III) or glioblastoma multiforme (GBM; grade IV).

In some embodiments, the gene product is a mRNA. In further embodiments, the level of gene product in the sample is determined by nucleic acid amplification; preferably, the nucleic acid amplification is polymerase chain reaction (PCR), reverse transcribed PCR (RT-PCR), real-time RT-PCR, or quantitative real-time RT-PCR. In other embodiments, the level of gene product in the sample is determined by a nucleic acid hybridization method; preferably, the nucleic acid hybridization method is a microarray or a membrane hybridization method, or is performed using a nucleic acid probe or primer, or the nucleic acid probe or primer is detectably labeled.

In some embodiments, the gene product is a polypeptide. In further embodiments, the level of gene product in the sample is determined by a polypeptide binding method, preferably an immunological detection method. In some preferred embodiments, the immunological detection method uses an antibody or an antigen binding fragment thereof; optionally the antibody or an antigen binding fragment thereof is detectably labeled.

In still other embodiments, the difference between the level of the gene product in the sample and the reference/control level is at least about 1%, 2%, 3%, 5%, 10%, 15%, 20%, 25%, 30%, 35%, 40%, 50%, 60%, 70%, 80%, 90%, 100%, 110%, 120%, 130%, 140%, 150%, 175%, 200%, 250%, 300%, 350%, 400%, 450%, 500%, 600%, 700%, 800%, 900%, or 1000%.

In preferred embodiments, the gene product is a KIAA0101 gene product, a ASPM gene product, a LOX gene product, a HOXA5 gene product, a COL6A2 gene product, a PLP2 gene product, a DKFZp762E1312 gene product, a CD99 gene product, a MELK gene product, a HOXC9 gene product, or a E2F2 gene product.

In some embodiments, the sample is a body fluid, a body effusion, cell or a tissue, preferably a brain cell or tissue.

According to yet another aspect of the invention, methods of treating astrocytoma are provided. The methods include administering to a subject who has or is suspected or having astrocytoma a compound or composition, which reduces the level or biological activity of a KIAA0101, LOX, , Melk, ASPM, HOXA5, PLP2, DKFZp762E1312, CD99, HOXC9 and/or E2F2 gene product, in an amount effective to treat the astrocytoma.

In some embodiments, the astrocytoma is a pilocytic astrocytoma (PA; grade I), low grade astrocytoma (grade II), anaplastic astrocytoma (grade III) and glioblastoma multiforme (GBM; grade IV). In other embodiments, the compound or composition is a RNAi molecule or antisense molecule.

In preferred embodiments, the gene product is a KIAA0101 gene product, a ASPM gene product, a Melk gene product, a LOX gene product, a HOXA5 gene product, a PLP2 gene product, a DKFZp762E1312 gene product, a CD99 gene product, a HOXC9 gene product, or a E2F2 gene product.

In some embodiments, the gene product is a mRNA or a polypeptide.

In a further aspect of the invention, methods of treating astrocytoma are provided. The methods include administering to a subject who has or is suspected or having astrocytoma a compound or composition, which binds a LOX, KIAA0101, HOXA5, DKFZp762E1312, HOXC9, MELK, ASPM, E2F2, CD99 or PLP2 polypeptide, in an amount effective to treat the astrocytoma. In some embodiments, the astrocytoma is a pilocytic astrocytoma (PA; grade I), low grade astrocytoma (grade II), anaplastic astrocytoma (grade III) and glioblastoma multiforme (GBM; grade IV). In other embodiments, the compound or composition is an antibody or an antigen-binding fragment thereof. Molecules that induce. RNA interferrence, such as siRNA molecules also can be used. In preferred embodiments, the compound or composition reduces the activity or function of the polypeptide.

According to another aspect of the invention, methods for identifying the presence of astrocytoma stem cells in a cell sample or tissue sample are provided. The methods include contacting the cell sample or tissue sample with a detectably labeled molecule that binds to CD133 and a gene product of a gene upregulated in astrocytoma stem cells.

In some embodiments, the agent is an antibody or antigen-binding fragment thereof that binds CD133 3 and a gene product of a gene upregulated in astrocytoma stem cells. In certain embodiments, the agent is a bispecific or multispecific antibody that binds CD133 and at least one gene product of a gene upregulated in astrocytoma stem cells.

In preferred embodiments, the at least one gene product is NXPH1, PCDHB14, TOD1, HOXC9, E2F2, LOC151261 (BX439624) or CD99.

In some embodiments, the detectable label is a fluorescent molecule. In other embodiments, the cell sample or tissue sample is an astrocytoma cell sample or tissue sample.

According to a further aspect of the invention, methods for identifying the presence of astrocytoma stem cells in a cell sample or tissue sample are provided. The methods include contacting the cell sample or tissue sample with a detectably labeled molecule that binds to CD133 to isolate a CD133+ subpopulation of cells, and determining the expression of at least one gene product of a gene upregulated or downregulated in astrocytoma stem cells in the CD133+ subpopulation of cells. The upregulation or downregulation of the gene product indicates the presence of astrocytoma stem cells in the cell sample or tissue sample.

In preferred embodiments, the at least one gene product of a gene upregulated or downregulated in astrocytoma stem cells is NXPH1, PCDHB14, TMOD1, HOXC9, E2F2, LOC151261 (BX439624), CD99, PDE1A, PPFIBP2, BAIAP2L1, THBS1, SLC45A1, DAAM2, AI272963 (Hs.149361), T03803 (Hs.654945), HSD3B7 and/or FLJ10489 (AK001351).

According to a further aspect of the invention, methods for identifying the presence of astrocytoma stem cells in an animal are provided. The methods include administering to the animal a detectably labeled agent that binds to CD133 and a gene product of a gene upregulated in astrocytoma stem cells. In some embodiments, the agent is one or more antibodies or antigen-binding fragments thereof that binds CD133 and a gene product of a gene upregulated in astrocytoma stem cells. In certain embodiments, the agent is a bispecific or multispecific antibody that binds CD133 and at least one gene product of a gene upregulated in astrocytoma stem cells. In preferred embodiments, the at least one gene product is CD99. In other embodiments, the detectable label is a contrast agent.

According to a further aspect of the invention, isolated cell populations are provided. The isolated cell populations include astrocytoma stem cells that express CD133 and an amount of at least one gene product of genes upregulated or downregulated in astrocytoma stem cells that is different from the amount of gene product expressed by a reference/control cell. In some embodiments, the CD133 is CD133-1 or a splice variant thereof; preferably the splice variant is CD133-2.

In other embodiments, the reference/control cell is an astrocytoma cell that is not an astrocytoma stem cell. Preferably, the astrocytoma stem cells express one or more of CD99, HOXC9 and E2F2 at a higher level than in astrocytoma cells. (of the same origin) that are not astrocytoma stem cells. In certain embodiments, the astrocytoma cells that are not astrocytoma stem cells do not express CD133. In still other embodiments, the expression of one or more of CD99, HOXC9 and/or E2F2 is determined by immunohistochemistry or PCR.

In further embodiments, the astrocytoma stem cells also express one or more markers for stem cells; preferably the one or more markers for stem cells are the SSEAs, ABCG2 and/or Sca-1.

In other embodiments, the astrocytoma stem cells are isolated by contacting a population of astrocytoma cells with an antibody that binds CD133 and the contacted cells are isolated by flow cytometry. Preferably the antibody is detectably labeled. Preferably, the contacted cells are further contacted with a detectably labeled molecule that binds to the antibody.

In still other embodiments, the astrocytoma stem cells are isolated by contacting a population of astrocytoma cells with immunomagnetic particles comprising an antibody that binds to CD133, and isolating the immunomagnetic particles.

In preferred embodiments, the astrocytoma stem cells are enriched in the cell population by at least 2-fold, at least 5-fold, at least 10-fold, 20-fold relative, or 50-fold relative to an unenriched cell population.

In another aspect of the invention, methods for treating cancer are provided. The methods include administering to a subject an effective amount of an agent or combination of agents selectively targeted to astrocytoma stem cells of the population of astrocytoma cells, wherein the agent or combination of agents kills the astrocytoma stem cells or inhibits the proliferation of astrocytoma stem cells. Preferably the population of astrocytoma cells is a tumor. In some preferred embodiments, the at least one gene product is one or more of CD99.

In some embodiments, the agent is one or more antibodies or antigen-binding fragments thereof that binds CD133 and a gene product of genes upregulated in astrocytoma stem cells such as a bispecific or multispecific antibody that binds CD133 and at least one gene product of genes upregulated in astrocytoma stem cells. In some embodiments, the agent is labeled with a cytotoxic agent or a cytostatic agent; preferably the cytotoxic agent is a radionuclide.

In other embodiments, the agent reduces expression of one or more gene products of genes upregulated in astrocytoma stem cells. Preferably the agent comprises one or more small interfering RNA molecules (siRNA) or other nucleic acid molecules that reduce expression of the one or more gene products of genes upregulated in astrocytoma stem cells by RNA interference. Preferably the agent comprises one or more double stranded RNA molecules.

In other embodiments, the combination of agents comprises an antibody or antigen-binding fragment thereof that binds CD133 and one or more small interfering RNA molecules (siRNA) or other nucleic acid molecules that reduce expression of the one or more gene products of genes upregulated in astrocytoma stem cells by RNA interference. In some of the foregoing embodiments, the gene is CD99, HOXC9 or E2F2.

According to another aspect of the invention, methods for killing or inhibiting the proliferation of astrocytoma stem cells are provided. The methods include contacting a population of astrocytoma cells with an agent or combination of agents selectively targeted to astrocytoma stem cells of the population of astrocytoma cells, wherein the agent or combination of agents kills the astrocytoma stem cells or inhibits the proliferation of astrocytoma stem cells. Preferably the population of astrocytoma cells is a tumor.

In some embodiments, the agent is one or more antibodies or antigen-binding fragments thereof that binds CD133 and a gene product of genes upregulated in astrocytoma stem cells such as a bispecific or multispecific antibody that binds CD133 and at least one gene product of genes upregulated in astrocytoma stem cells. In some embodiments, the agent is labeled with a cytotoxic agent or a cytostatic agent; preferably the cytotoxic agent is a radionuclide.

In other embodiments, the agent reduces expression of one or more gene products of genes upregulated in astrocytoma stem cells. Preferably the agent comprises one or more small interfering RNA molecules (siRNA) or other nucleic acid molecules that reduce expression of the one or more gene products of genes upregulated in astrocytoma stem cells by RNA interference. Preferably the agent comprises one or more double stranded RNA molecules.

In other embodiments, the combination of agents comprises an antibody or antigen-binding fragment thereof that binds CD133 and one or more small interfering RNA molecules (siRNA) or other nucleic acid molecules that reduce expression of the one or more gene products of genes upregulated in astrocytoma stem cells by RNA interference. In some of the foregoing embodiments, the gene product of a gene upregulated in astrocytoma stem cells is CD99, HOXC9 or E2F2.

In yet another aspect of the invention, methods for isolating astrocytoma stem cells are provided. The methods include contacting a population of astrocytoma cells with one or more labeled antibodies that bind to CD133 and a gene product of a gene upregulated in astrocytoma stem cells and isolating the astrocytoma stem cells based on the binding of the labeled antibodies to the astrocytoma stem cells. In certain embodiments, the population of astrocytoma cells is obtained from a tumor sample or a cell line. In other embodiments, the labeled antibodies are labeled with fluorescent molecules. In some embodiments, the step of isolating is performed by fluorescence-activated cell sorting or magnetic activated cell sorting.

In additional embodiments, the step of contacting the astrocytoma cells is carried out by contacting the cells with labeled antibodies that bind to CD133 first followed by contacting the cells with labeled antibodies that bind to a gene product of a gene upregulated in astrocytoma stem cells.

In additional embodiments, the step of contacting the astrocytoma cells is carried out by contacting the cells with labeled antibodies that bind to a gene product of a gene upregulated in astrocytoma stem cells first followed by contacting the cells with labeled antibodies that bind to CD133.

In additional embodiments, the step of contacting the astrocytoma cells is carried out by simultaneously contacting the cells with labeled antibodies that bind to CD133 and with labeled antibodies that bind to a gene product of a gene upregulated or downregulated in astrocytoma stem cells.

The use of the foregoing compositions in the preparation of medicaments for treatment of disease, particularly cancer, also is provided in accordance with the invention.

These and other aspects of the invention, as well as various embodiments thereof, will become more apparent in reference to the drawings and detailed description of the invention.

### Brief Description of the Drawings

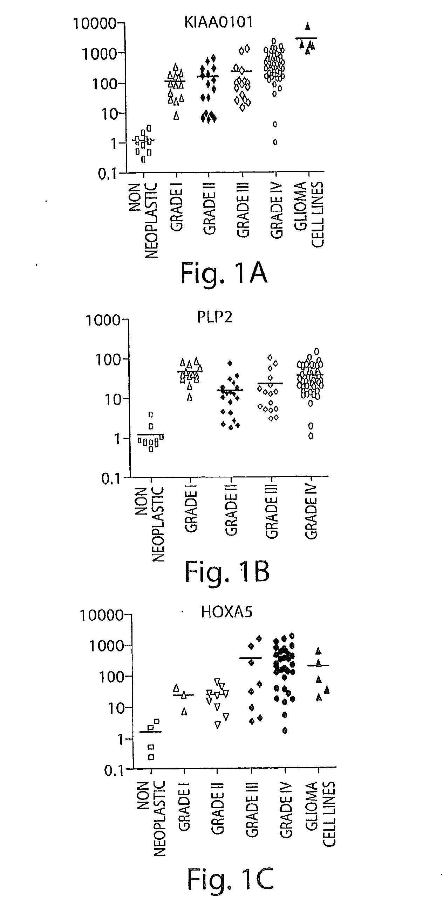

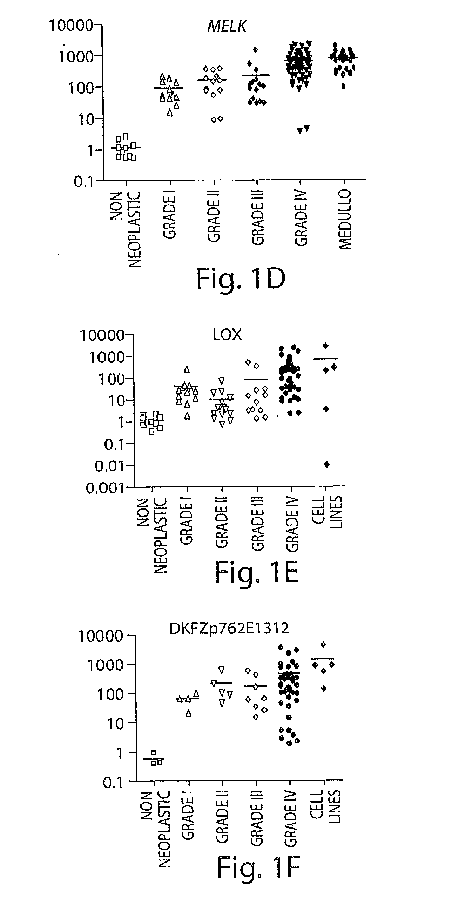

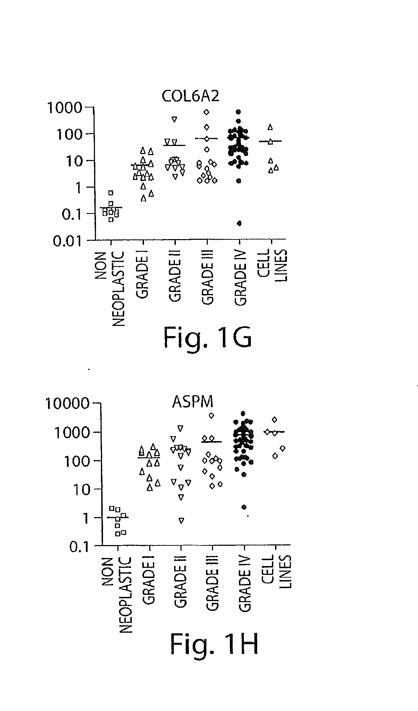

**Figure 1**. Relative expression of genes in non-neoplastic tissue (non neopl), grade I astrocytoma tissue (pilocytic astrocytomas, grade I), grade II astrocytoma tissue (low grade astrocytomas, grade II), grade III astrocytoma tissue (anaplastic astrocytomas, grade III), grade IV astrocytoma tissue (glioblastomas, grade IV) and glioblastoma cell lines (cell lines) was determined by QT-PCR and is shown on a log scale of relative expression (log₁₀^{2-ΔΔt}) calculated based on HPRT expression levels of each sample. The horizontal bar shows the median of each group and the statistical p values according to Mann-Whitney test are shown at each comparison. The results for the genes are depicted as follows: KIAA0101 (Fig. 1A); PLP2 (Fig. 1B); HOXA5(Fig. 1C); MELK (Fig. 1D); LOX (Fig. 1E); DKFZp762E1312 (Fig. 1F); COL6A2 (Fig. 1G); ASPM (Fig. 1H).

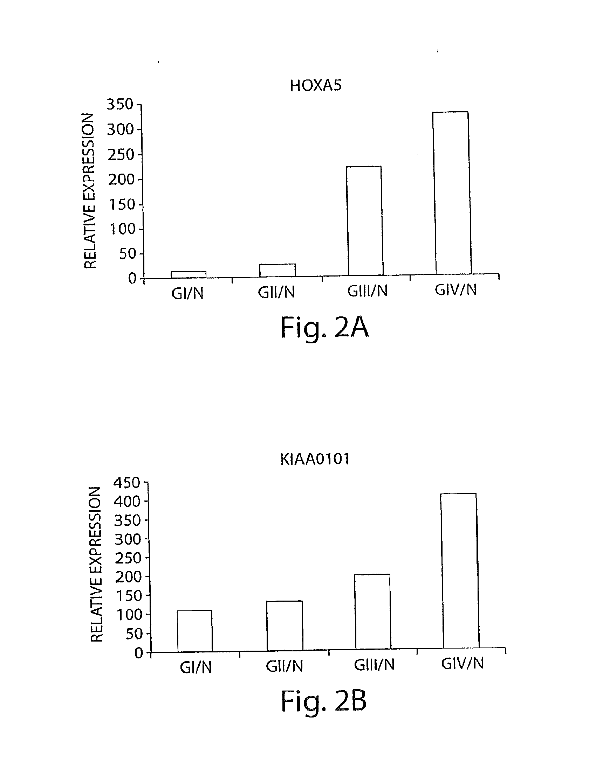

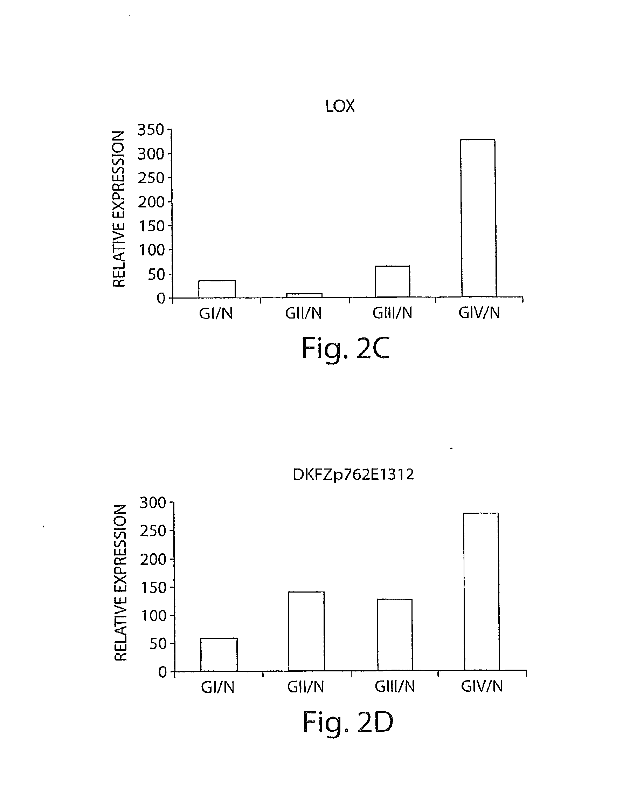

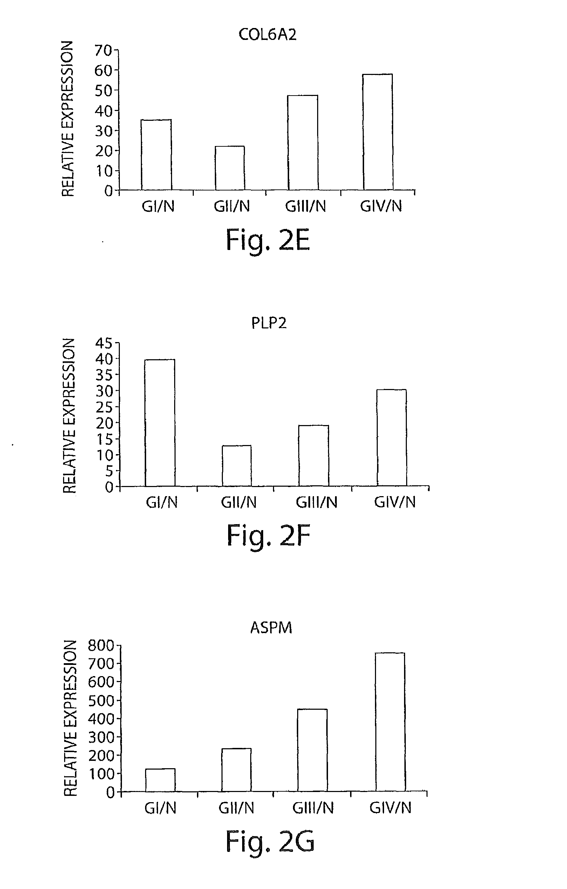

**Figure 2**. Relative expression of genes in non-neoplastic tissue and astrocytoma tissues determined by QT-PCR. Comparisons of non-neoplastic tissue (N) with grade I astrocytoma tissue (GI), grade II astrocytoma tissue (GII), grade III astrocytoma tissue (GIII), or grade IV astrocytoma tissue (GIV; glioblastoma multiforme) are shown on a linear scale of relative expression. The results for the genes are depicted as follows: HOXA5 (Fig. 2A); KIAA0101 (Fig. 2B); LOX (Fig. 2C); DKFZp762E1312 (Fig. 2D); COL6A2 (Fig. 2E); PLP2 (Fig. 2F); ASPM (Fig. 2G).

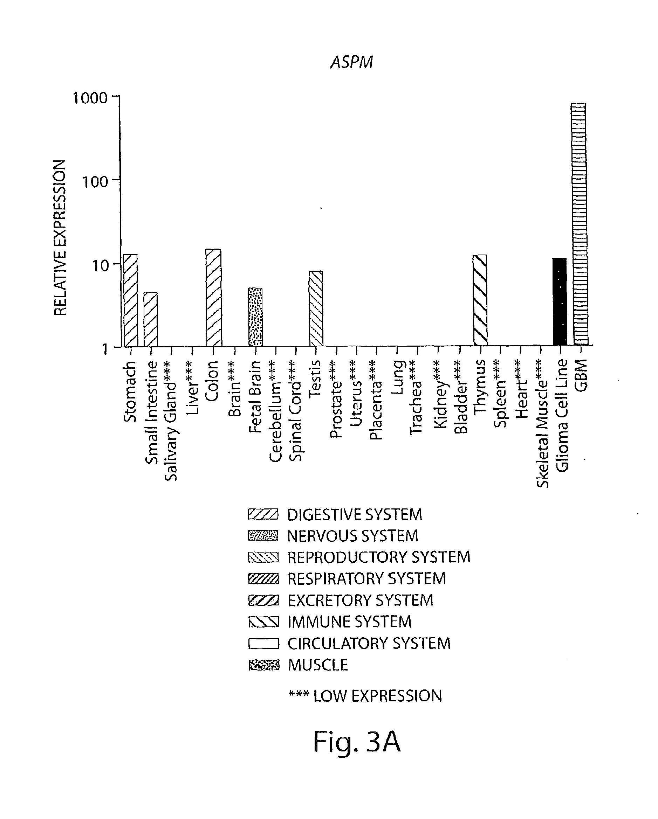

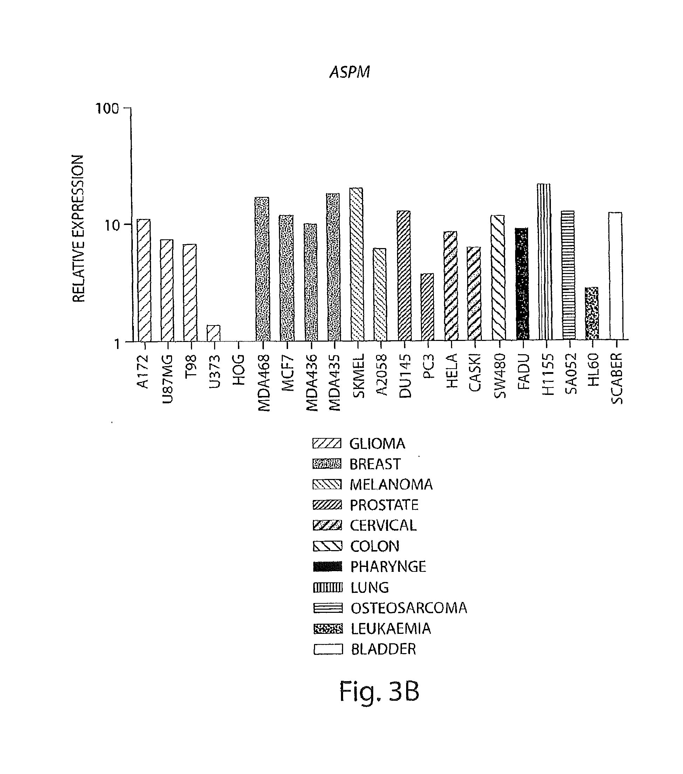

**Figure 3****.** Relative expression levels for *ASPM* analyzed by QT-PCR. A) panel of 21 normal tissues and B) panel of cancer cell lines.

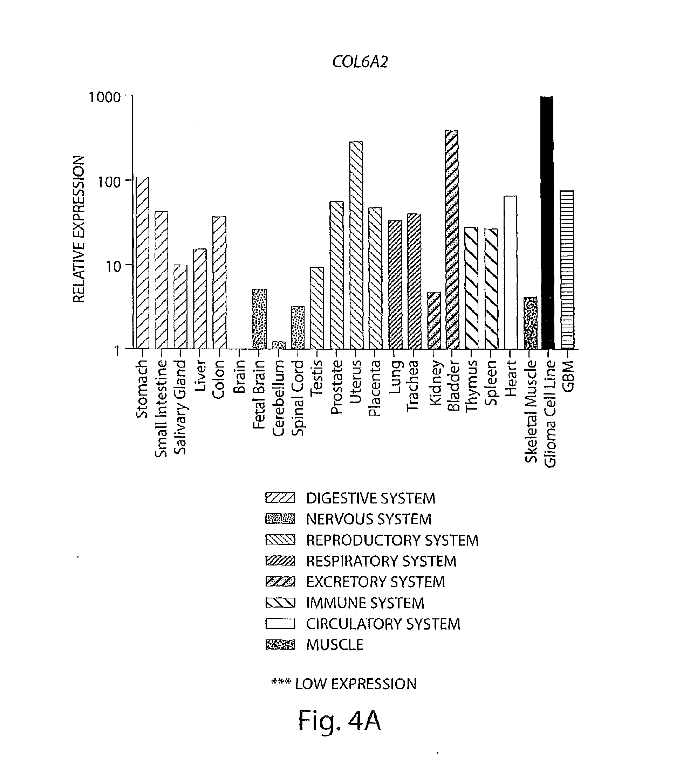

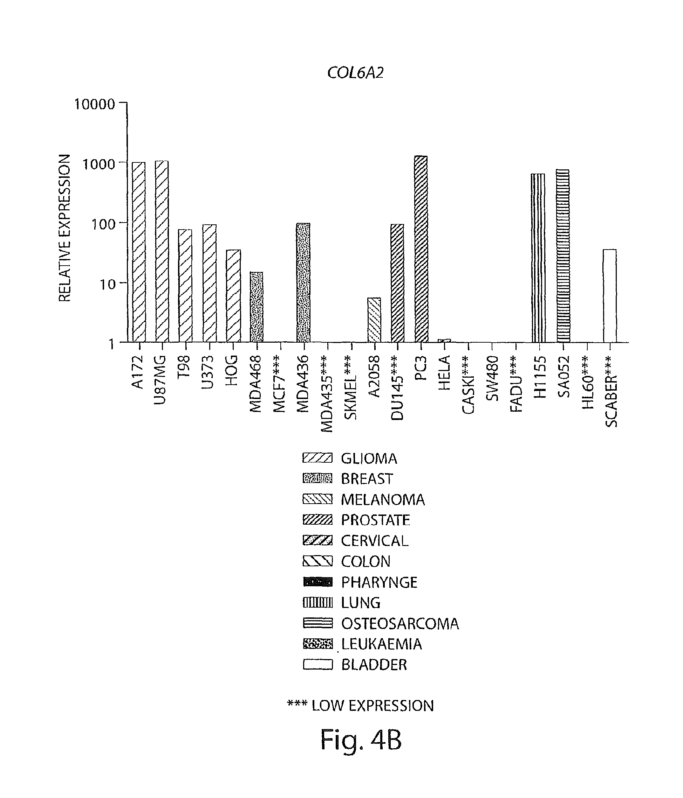

**Figure 4****.** Relative expression levels for *COL6A2* analyzed by QT-PCR. A) panel of 21 normal tissues and B) panel of cancer cell lines.

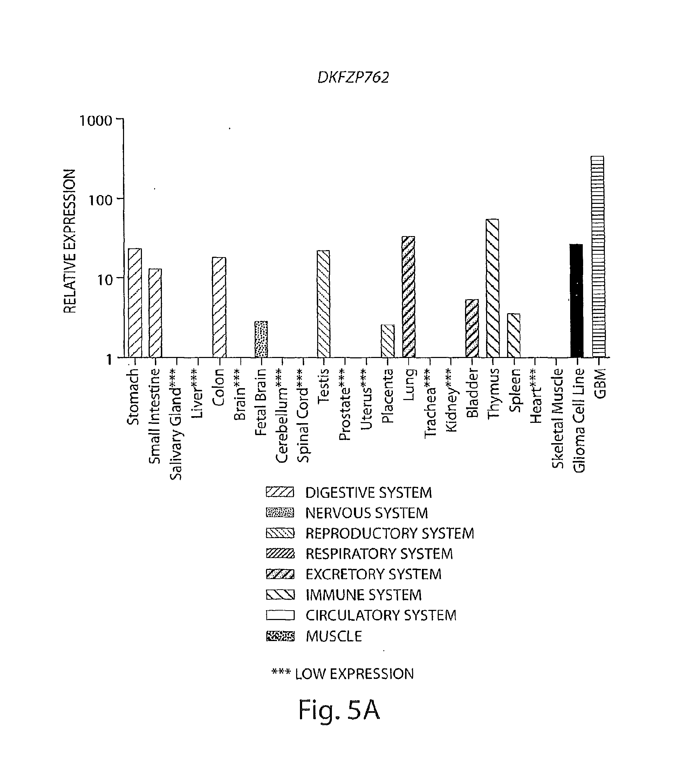

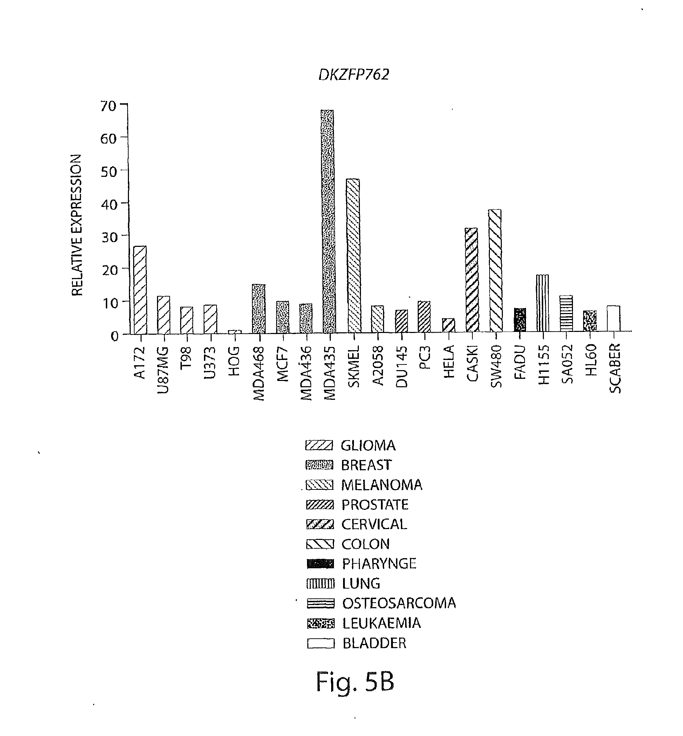

**Figure 5****.** Relative expression levels for *DKFZp762E1312* analyzed by QT-PCR. A) panel of 21 normal tissues and B) panel of cancer cell lines.

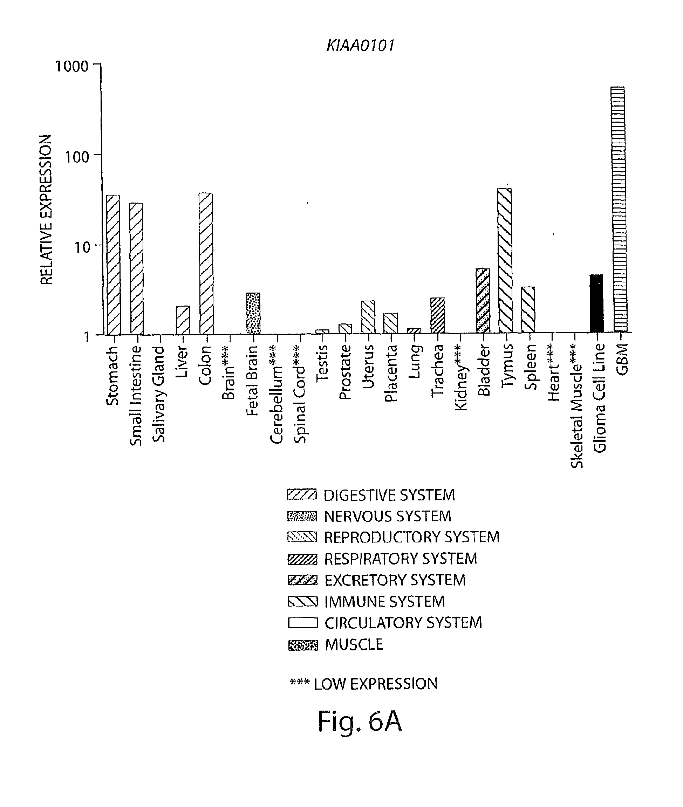

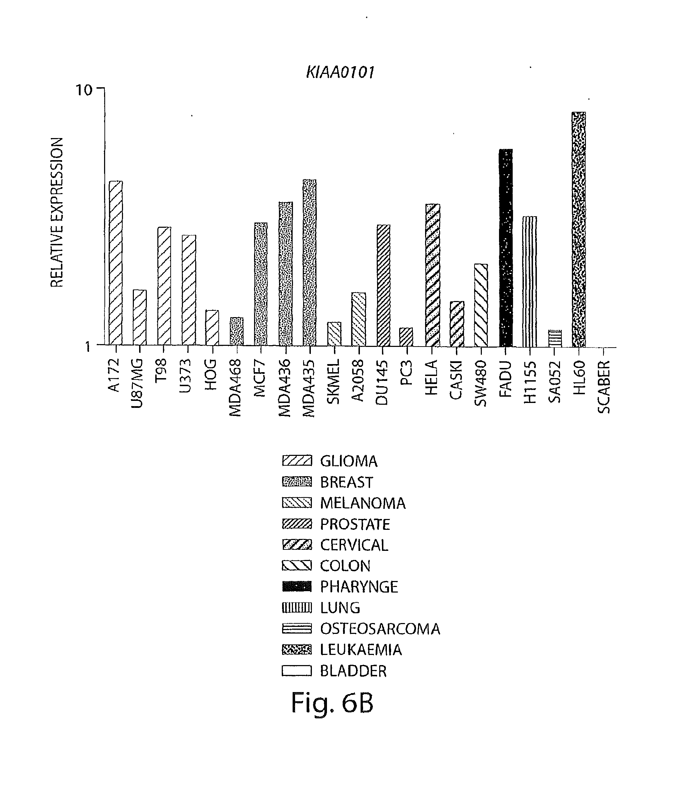

**Figure 6****.** Relative expression levels for *KIAA0101* analyzed by QT-PCR. A) panel of 21 normal tissues and B) panel of cancer cell lines.

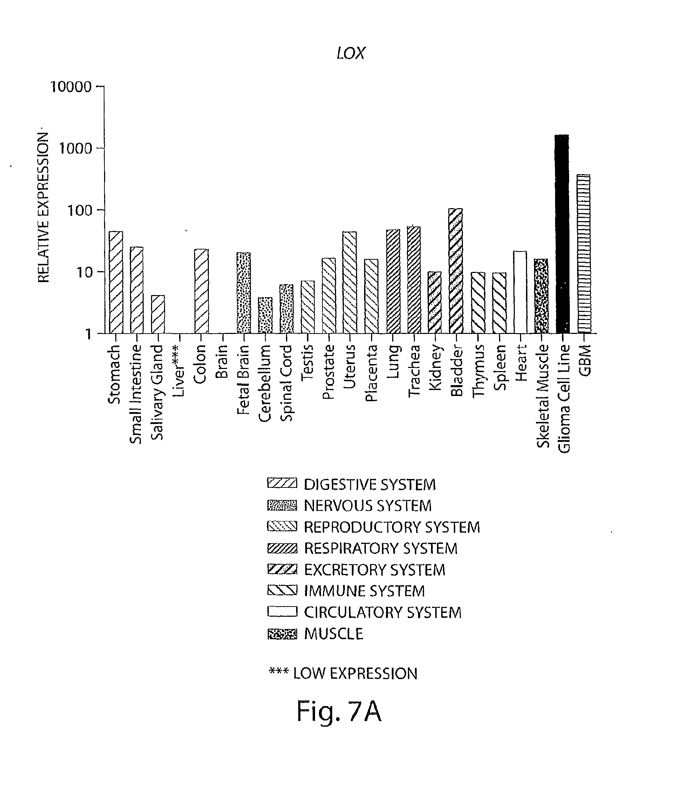

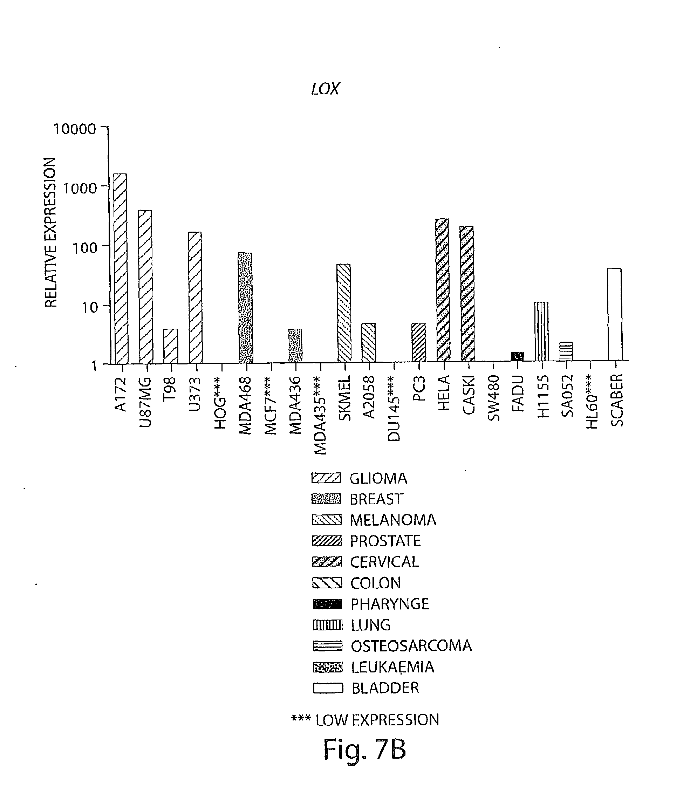

**Figure 7****.** Relative expression levels for *LOX* analyzed by QT-PCR. A) panel of 21 normal tissues and B) panel of cancer cell lines.

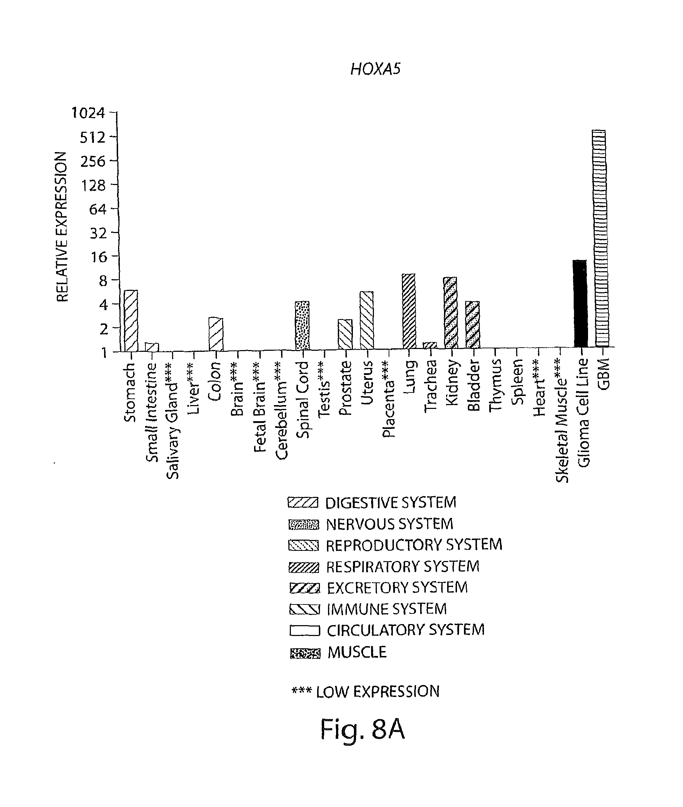

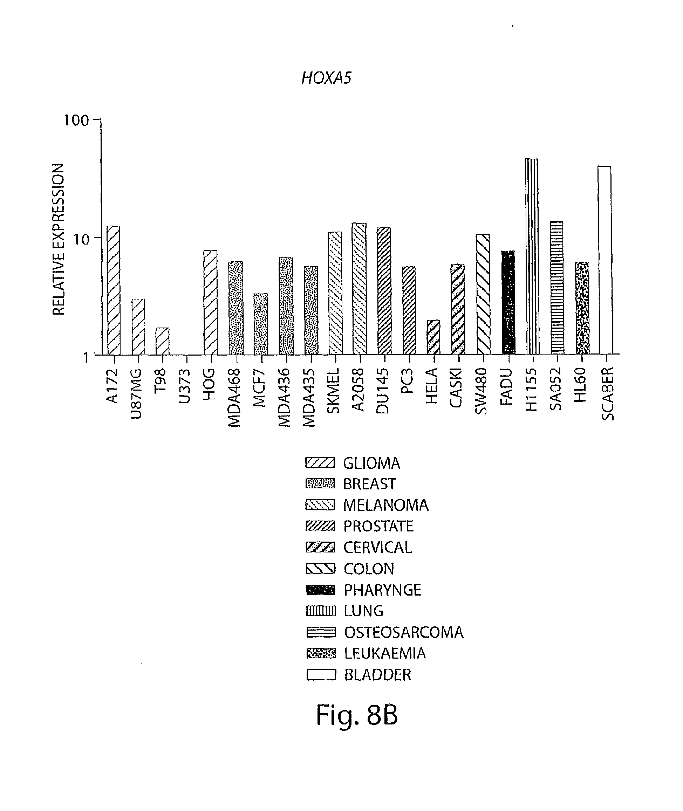

**Figure 8****.** Relative expression levels for *HOXA5* analyzed by QT-PCR. A) panel of 21 normal tissues and B) panel of cancer cell lines.

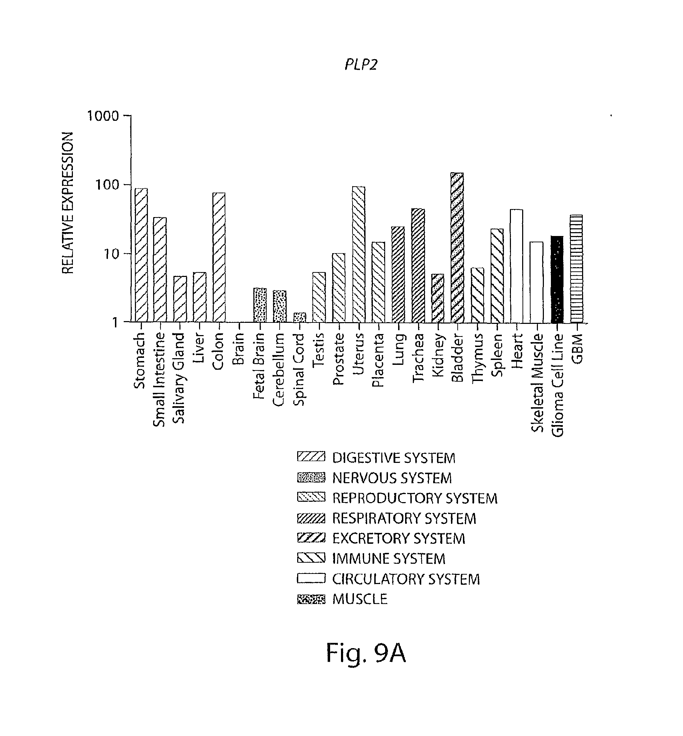

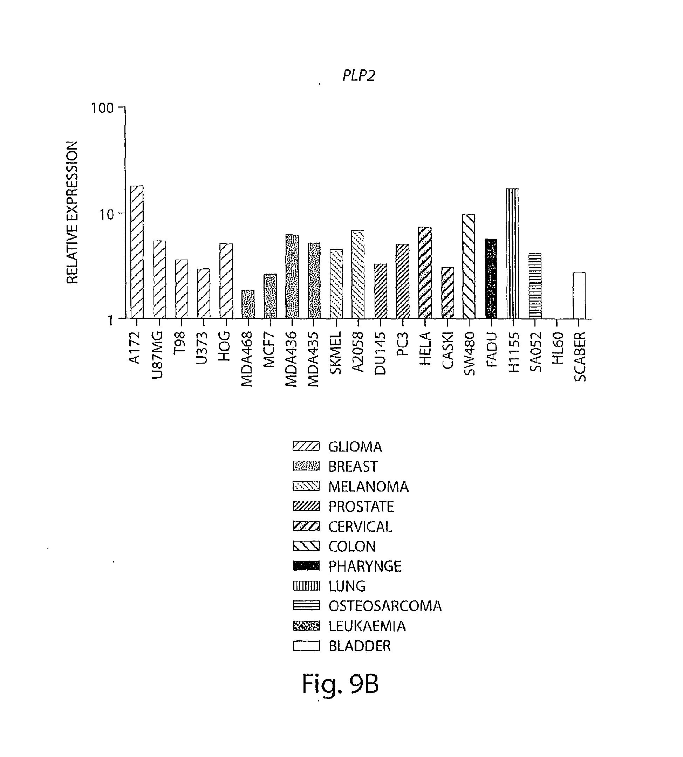

**Figure 9****.** Relative expression levels for *PLP2* analyzed by QT-PCR. A) panel of 21 normal tissues and B) panel of cancer cell lines.

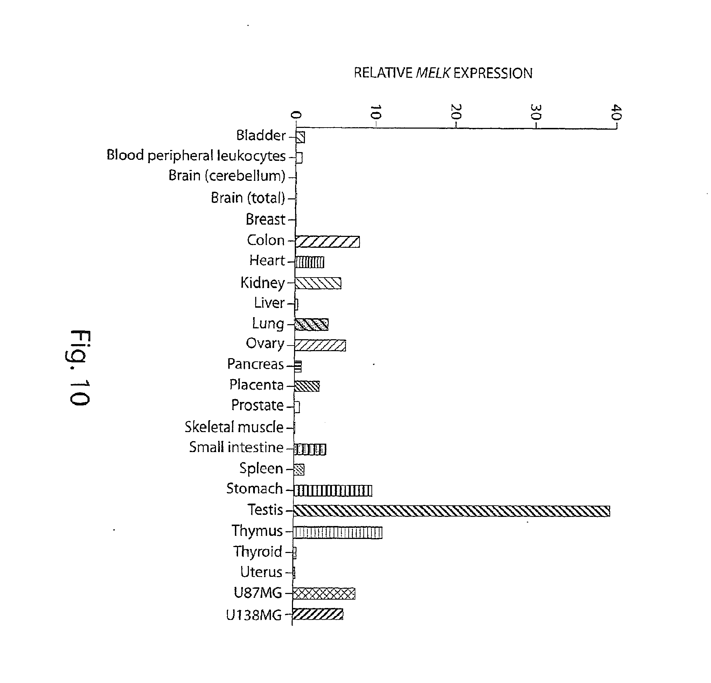

**Figure 10****.** MELK expression in normal tissues. Quantitative RT reverse transcription PCRs were performed with c-DNAs samples prepared from RNAs from several normal tissues for MELK and ACTB as endogenous control gene. The cDNA templates used were normalized on the basis of ACTB amplification.

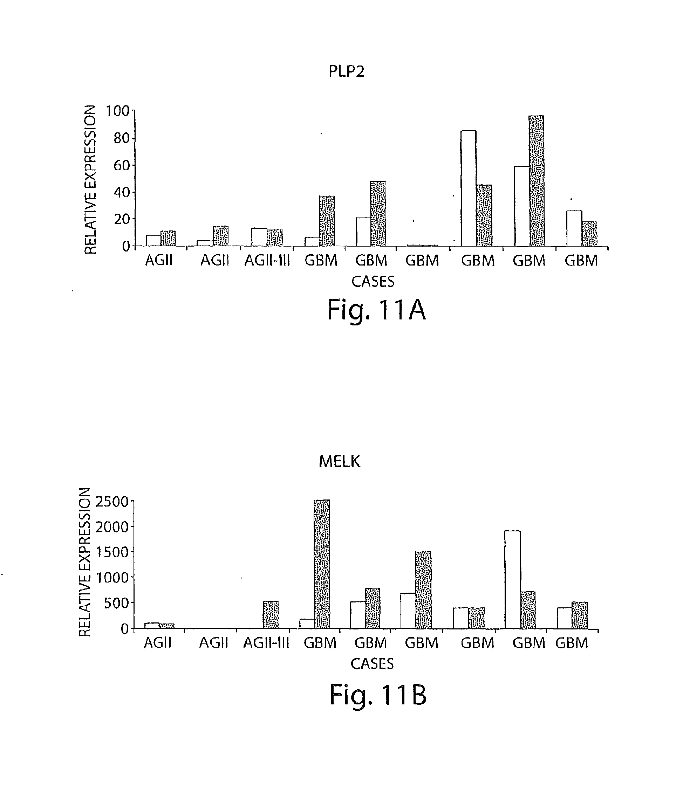

**Figure 11****.** Gene expression level in recurrence measured by QT-PCR. A) PLP2 and B) MELK relative expression in sequential samples of nine patients who were submitted to two surgeries. The first surgery was diagnostic and therapeutical (white) and the second intervention was because of recurrence of tumor (black). The expression level of each gene was compared to the gene expression in non-neoplastic brain samples. Two patients presented low grade astrocytoma (GII), one patient presented low grade astrocytoma in the first sample and anaplastic astrocytoma (GIII) in the second sample. Six patients presented reccurence of GBM (GIV).

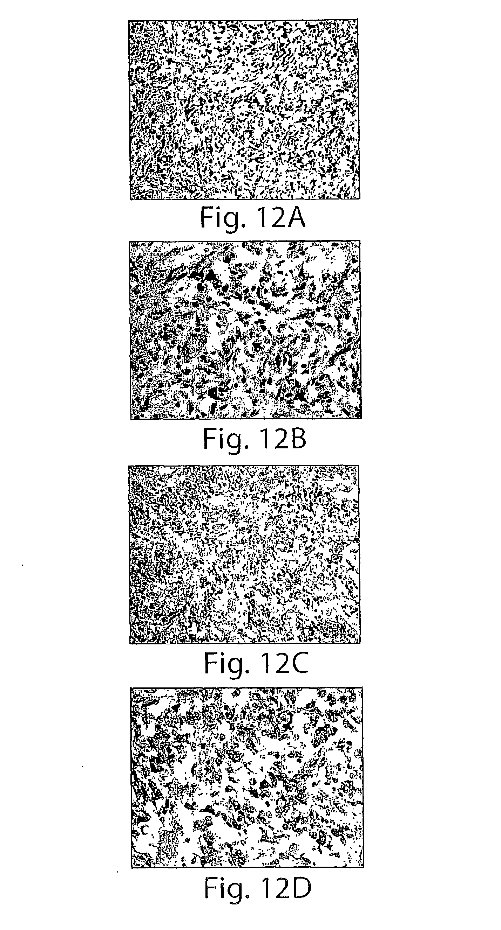

**Figure 12****.** MELK expression level in recurrence. Immunohistochemistry of a GIV case with polyclonal anti-MELK antibody developed by Jean-Pierre Tassan [iii]. Panels A & B: tumor sample from the first surgery, and Panels C & D: tumor sample from the second surgery. Panels C and D show an increase of MELK positive staining in the recurence of the disease. Interval of four months between the two surgeries. Amplification: A &C:100x, B & D: 200X.

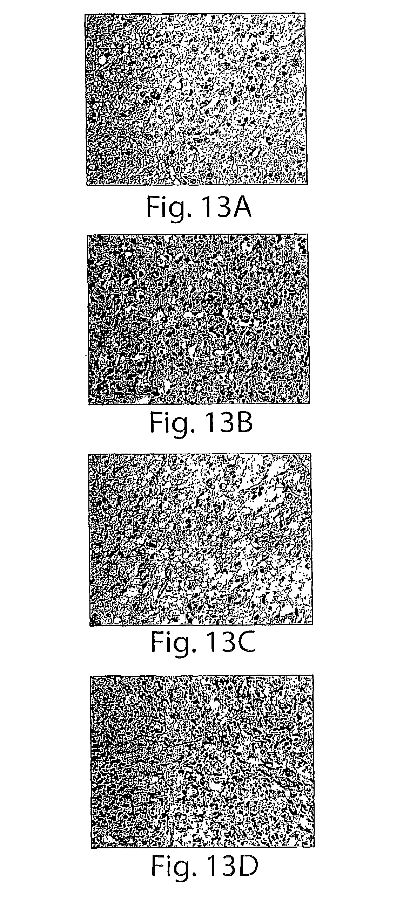

**Figure 13****.** PLP2 expression level in recurrence. Immunohistochemistry of a GIV case with polyclonal anti-PLP2 antibody. Figures A and B tumor sample from the first surgery, and Figures C and D from the second surgery. Samples A and C are from border zone of the tumor whereas B and D are from the bulk of the tumor. An increase of PLP2 positive staining can be observed in the sample from the border zone of the tumor extracted in the second surgery, C. Interval of three months between the two surgeries. Amplification: 200X.

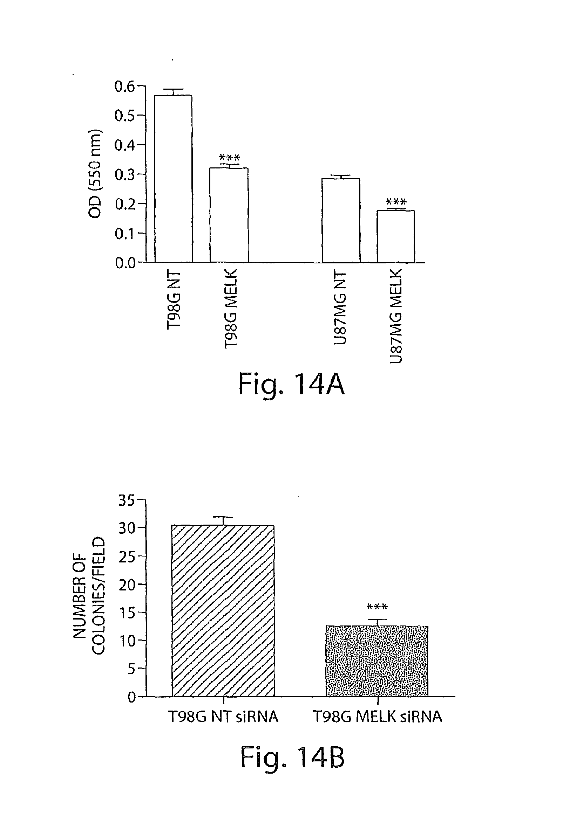

**Figure 14****.** Effect of MELK downregulation on in vitro behavior of malignant astrocytoma cell lines. Fig. 14A shows the effect on cell proliferation as measured by MTT assay on the fourth day after transfection with non-targeting (NT) or MELK-specific siRNA pools (21-mer pool siRNA) on T98G and U87MG cell lines. Fig. 14B shows the effect of MELK knock-down on anchorage-independent growth as measured by colony formation assays. T98G cell line transfected with MELK-specific siRNA pool (21-mer pool siRNA)showed reduced capacity to form colonies in soft agar compared with negative controls. The mean and standard error of the mean (SEM) were calculated from six replicates for the MTT assay and from 20 fields in two separate wells for the colony formation assay. Statistically significant differences from control are indicated as *** (P < 0.001). Similar results were obtained from two independent experiments.

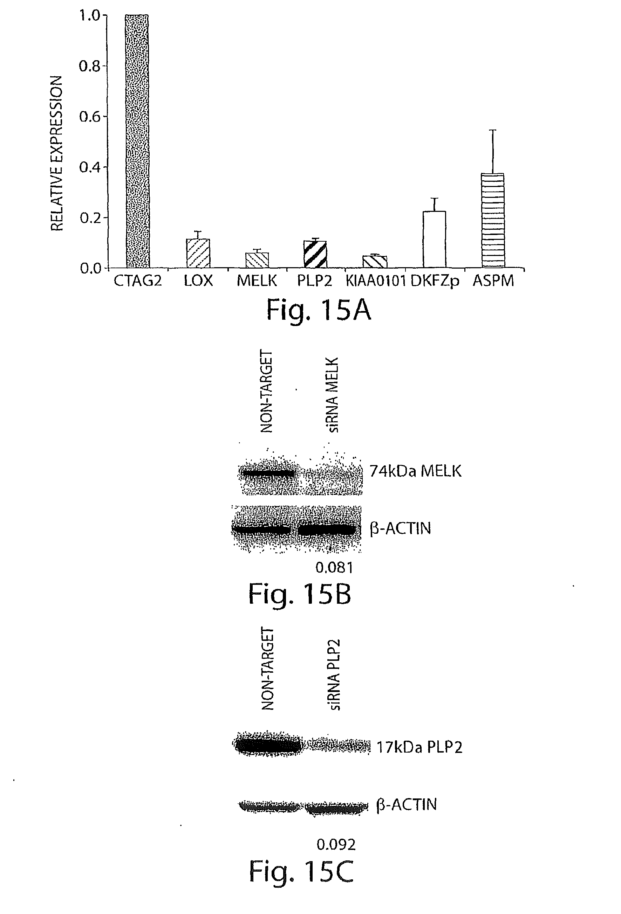

**Figure 15****.** Use of 27 mer-siRNA to deplete gene expression in U251MG glioma cell line. A) Relative gene expression measured by QT-PCR after treating the cells with siRNAs targeting *ASPM*, *Melk*, *DKFZp762E1312*, *KIAA0101*, *LOX* and *PLP2.* Control siRNA used CTAG2. Columns, mean of two independent experiment; bars, SD. B) Western blot analysis of U251MG cell line treated with non-target and *MELK* siRNAs. The number represents the relative fold *MELK* mRNA expression in comparison to the control CTAG2. C) Western blot analysis of U251MG cell line treated with non-target and *PLP2* siRNAs. The number represents the relative fold *PLP2* mRNA expression in comparison to the control CTAG2.

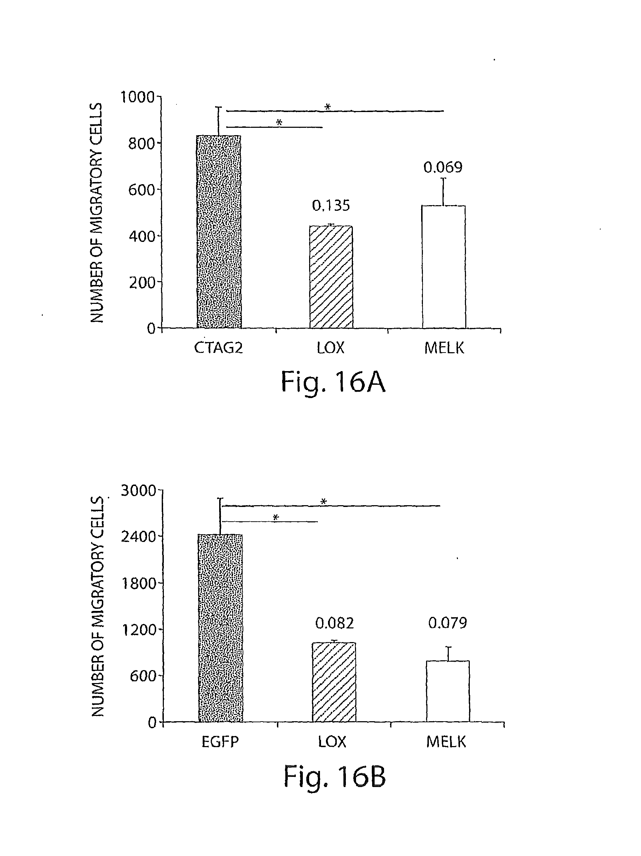

**Figure 16****.** Effect of targeted genes knock-down on migration using A) U251MG and B) U87MG glioma cell lines. Analysis and quantification of the *in vitro* migratory phenotype of U251MG and U87MG glioma cells transfected with each targeted gene or non-targeting siRNA. Experiments were performed in duplicates. The numbers represent the relative fold target genes mRNA expression in comparison to the control CTAG2. Colums, mean of two wells; bars, SD. Statistically significant differences from controls (CTAG2, and EGFP) are indicated as * (p<0.001).

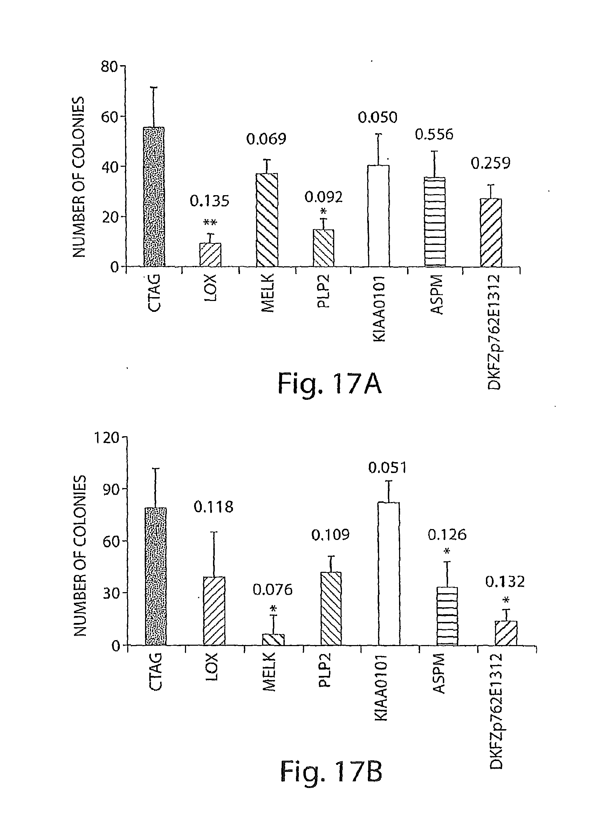

**Figure 17****.** Effect of targeted genes knock-down on colony formation using A) U251MG and B) A172 glioma cell lines. Analysis and quantification of the colony formation of U251MG and U87MG glioma cells transfected with each targeted gene or non-targeting siRNA. Columns, mean of three wells; bars, SD. Statistically significant differences from control. (CTAG2) are indicated as *(p<0.05) and ** (p<0.005). The numbers represent the relative fold target genes mRNA expression in comparison to the control CTAG2.

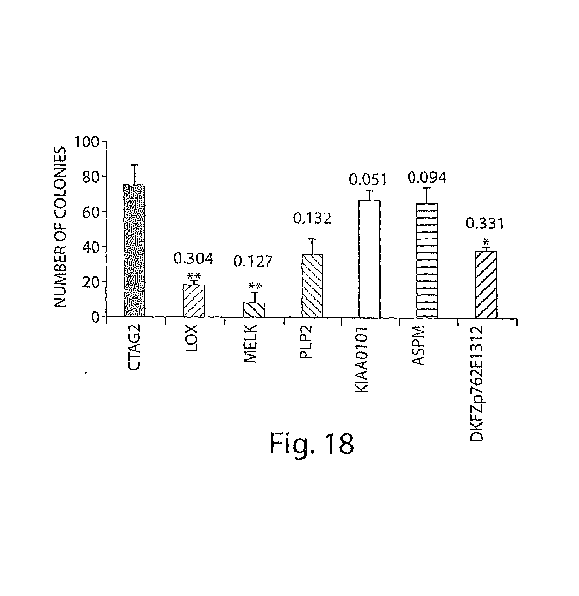

**Figure 18****.** Effect of targeted genes knock-down on anchorage independent growth in soft-agar assay in U87MG glioma cell line. The number and size of colonies were registered, in triplicate. Columns, mean of the triplicates; bars, SD. Statistically significant differences from control (CTAG2) are indicated as * (p<0.05) and ** (p<0.005). The numbers represent the relative fold target genes mRNA expression in comparison to the control CTAG2.

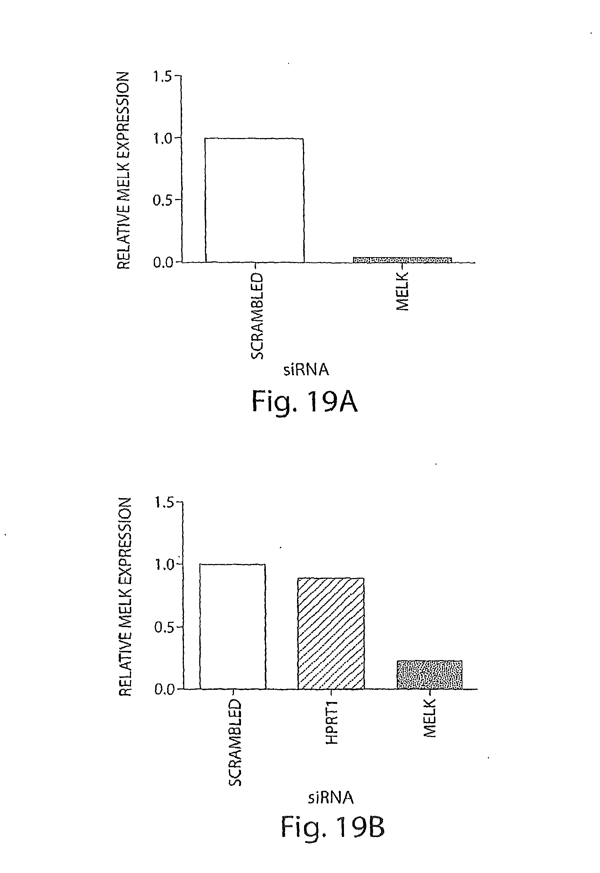

**Figure 19****.** Use of 27 mer-siRNA to deplete Melk expression in A) SK-MEL-37 and B) LNCaP cell lines (melanoma and prostate cancer cell lines, respectively). Relative Melk expression measured by QT-PCR after treating the cells with siRNA, Melk (1). A scrambled universal negative control RNA duplex (Scrambled) which is absent in human, mouse, and rat genomes, and a positive control Dicer-Substrate RNA duplex (HPRT1) which targets a site in the *HPRT* (hypoxanthine guanine phosphoribosyltransferase 1) and is prevalidated to give >90% knockdown of *HPRT* when transfected at 10 nM concentration were used.

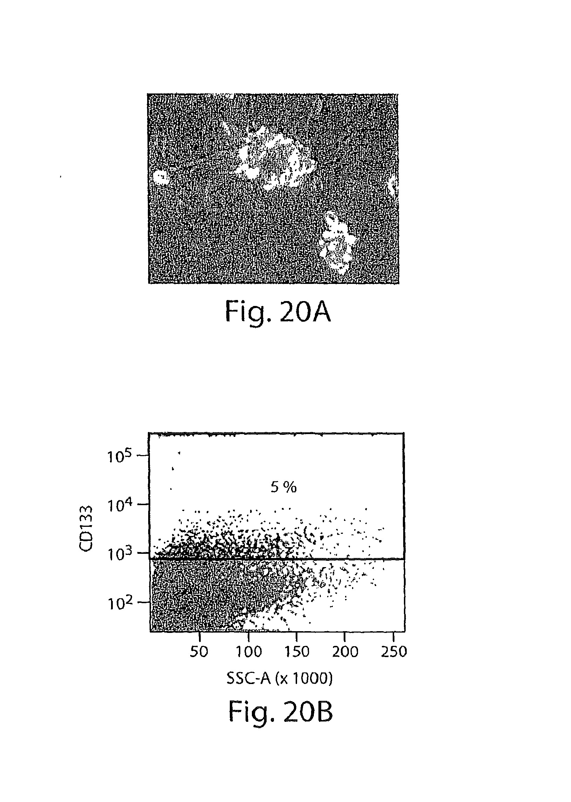

**Figure 20****.** CD133+ cells in primary glioblastomas. Fig. 20A shows a cluster of non-adherent cells (oncospheres) maintained in culture medium after dissociation of glioblastoma (GBM) specimens. Fig. 20B depicts the relative amount of CD133-expressing cells in suspensions of dissociated GBM.

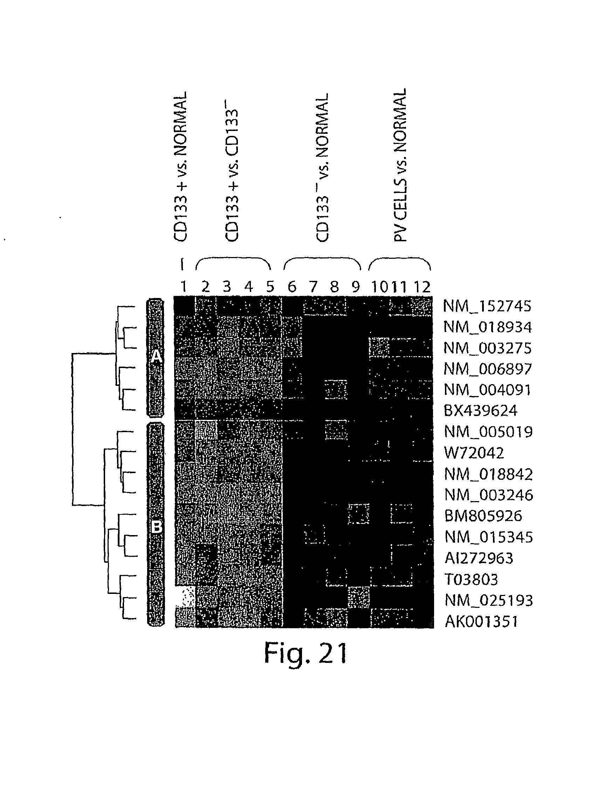

**Figure 21****.** Gene expression profiling of CD133+ and CD133- cells from glioblastomas. The transcriptomes of CD133+ and CD133- GBM cells were directly compared against each other. Individual comparisons of CD133+, CD133-, or non-neoplastic cells recovered from the periventricular region (PV), with control brain tissue (normal) were also included in the analysis. Twelve meaningful hybridization comparisons were performed and results expressed as fold change. Genes are clustered based on similarity in expression pattern across the 12 hybridizations. Clusters A is comprised by genes consistently hyper-expressed in CD133+ cells, when compared either with CD133- cells or control brain tissue. Likewise, cluster B genes show a consistent hypo-expression in CD133+ cells. None of these 16 genes in clusters A and B, however, shows a uniform pattern of differential expression in CD133-and PV cells, compared with control brain tissue.

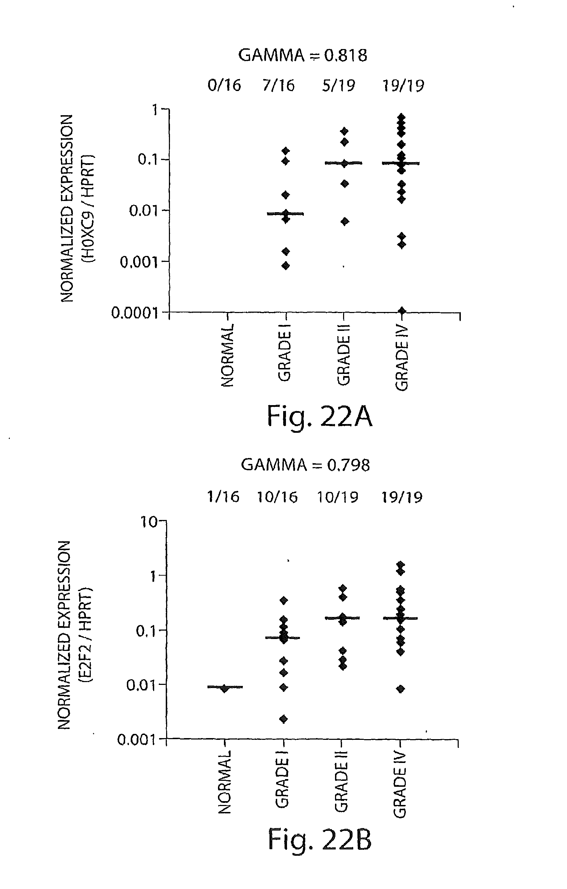

**Figure 22****.** Up-regulation of *HOXC9* (A) and *E2F2* (B) in astrocytomas. The horizontal bars indicate median expression values. The proportion of samples with detectable *HOXC9* or *E2F2* expression is indicated on top of each plot, respectively. The Goodman and Kruskal's gamma values indicate association between the expression of each of these genes with malignancy of human astrocytomas.

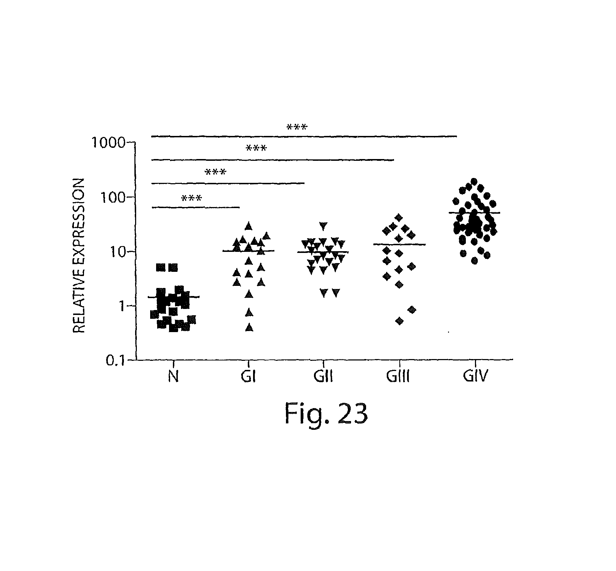

**Figure 23****.** Relative expression of *CD99* in astrocytomas. A total of 19 non-neoplastic brain tissue (N), and 97 astrocytic tumor samples were used (18 pilocytic astrocytomas (GI), 18 low grade astrocytomas (GII), 18 anaplastic astrocytomas (GIII), and 46 Glioblastomas (GIV). QT-PCR assays were carried out in duplicates by the Sybr-Green approach, and normalization of quantitative data was based on the housekeeping genes: hypoxanthine guanine phosphoribosyl transferase gene (*HPRT*), glucuronidase beta gene (*GUSB*), and breast cancer resistance protein gene (*BCRP*). The horizontal bars show the median of each group, and the statistical analysis according to Mann-Whitney test are shown for each comparison, *** (p<0.0001).

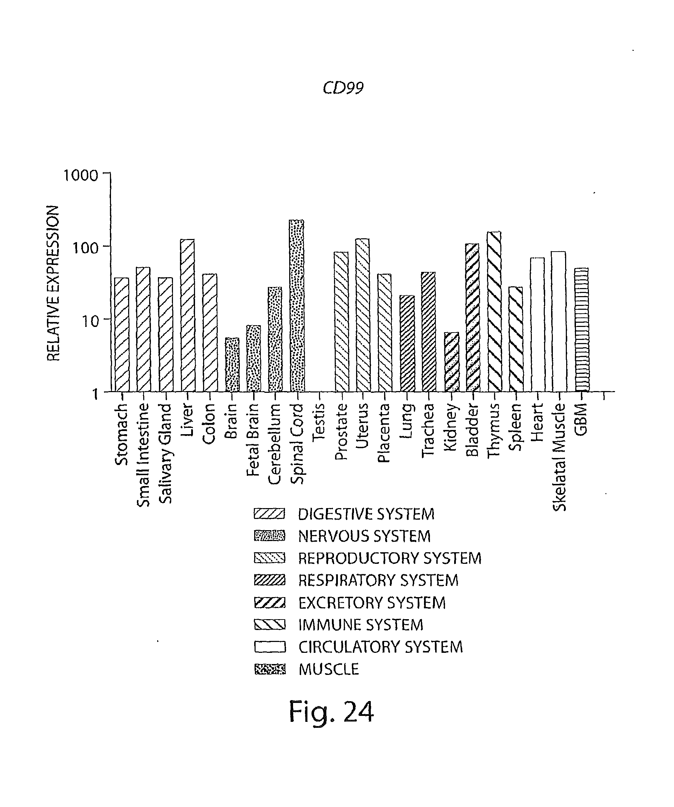

**Figure 24****.** Relative expression levels for *CD99* in normal tissues. Quantitative real time PCRs were performed with cDNAs samples prepared from RNAs from several normal tissues. *GUSB*, *BCRP and HPRT* used as an endogenous control genes. Expression levels (log10 2-ΔΔCt) were calculated based on the mean geometric average of the control genes expression levels of each sample. The expression of each sample was calculated relative to the tissue with the lower gene expression level (testis) and compared to the mean expression level of GBM cases.

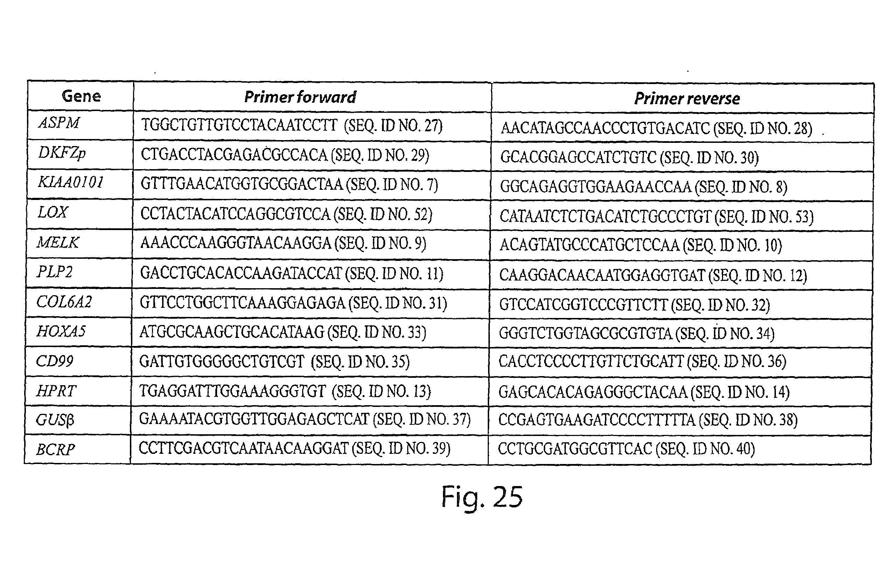

**Figure 25****.** Primer sequences (5'-3') used to perform QT-PCT analysis.

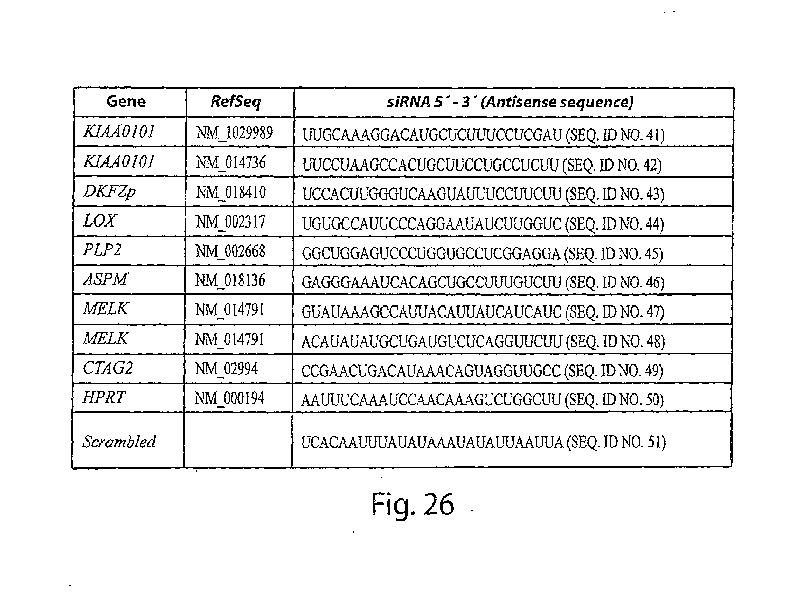

**Figure 26****.** Sequence of 27- mer siRNA duplexes used in functional assays synthesized by Integrated DNA Technologies (IDT, Coralville, IA).

### Detailed Description of the Invention

We have performed cDNA microarray analyses to identify gene expression differences between highly invasive glioblastoma multiforme and typically benign pilocytic astrocytomas. Despite the significant clinical and pathological differences between the two tumor types, only 63 genes were found to exhibit two-fold or greater over-expression in GBM as compared with GI (PA). More than 50% of these genes are related to the regulation of the cell cycle and mitosis. Initial QT-PCR validation of five over-expressed genes: *MELK*, , *ASPM*, *LOX*, *COL6A2* and *KIAA0101*, confirmed at least a 5 fold increase in the average expression levels in GBM compared to GI (PA). Of the initial group of the over expressed genes, the gene that exhibited the most statistically significant difference is *Maternal Embryonic Leucine zipper Kinase* (*MELK*). We undertook a more detailed investigation of the expression of the serine/threonine kinase *MELK* in the light of its role in the regulation of multipotent neural progenitor proliferation and previous suggestions of its oncogenic relevance. In an examination of more than 100 tumors of the central nervous system we found progressively higher expression of *MELK* with astrocytoma grade and a noteworthy uniformity of high level expression in GBM. This latter probably explains the lack of observed association of *MELK* expression with survival of GBM patients. Overexpression at a similar level to GBM was also observed in medulloblastoma. We found neither gene methylation nor amplification to be a factor in *MELK* expression but were able to demonstrate that *MELK* knockdown in malignant astrocytoma cell lines caused a reduction in proliferation and anchorage-independent growth in *in vitro* assays. Our results indicate that GBM and GI (PA) differ by the expression of surprisingly few genes. Among them, *MELK* may play a role in differentiating these two tumor types and represent a therapeutic target for the management of the most frequent brain tumors in adult and children.

Additional genes were subsequently validated as over-expressed using QT-PCR.

The invention provides for the identification, isolation, enrichment, selection and targeting of astrocytoma cells. Astrocytoma cells are identified, isolated, enriched, selected or target by detection (including binding) or use of "astrocytoma markers", which are set forth in part in Table I. Some of the markers shown in Table IX as upregulated in astrocytoma stem cells (i.e, are "astrocytoma stem cell markers") also have been confirmed as upregulated in astrocytoma tissue, and therefore also are astrocytoma markers: CD99, HOXC9 and E2F2. Therefore, astrocytoma stem cell markers are a subset of astrocytoma markers. Preferred markers are those validated using QT-PCR as having statistically significant higher levels of expression in astrocytomas: KIAA0101, ASPM, LOX, HOXA5, COL6A2, PLP2, DKFZp762E1312, MELK, CD99, HOXC9 and E2F2.

Astrocytoma marker gene expression in identified, isolated, enriched or selected astrocytoma cells can be increased in type and/or in amount relative to cells or tissue that are not astrocytoma cells or tissue, e.g., non-neoplastic brain tissue. By "increased type" and the like is meant that different and/or more genes are expressed in astrocytoma cells relative to non-astrocytoma cells. By "increased amount" and the like is meant that higher amounts of gene products of genes upregulated in astrocytoma cells are expressed as measured by amounts of RNA transcripts or protein. Each of these is demonstrated in the Examples below.

The expression of astrocytoma marker gene products can be determined by standard methods known in the art. These methods include the microarray and QT-PCR methods described herein, as well as immunohistochemistry, and quantitation of expression using fluorescence activated cell sorting and other methods that allow for discriminating between cell types in a population without significantly affecting the ability of the cells to survive the quantitation methods.

Astrocytoma cells can be isolated by a variety of methods known in the art. These methods include flow cytometry, such as fluorescence activated cell sorting. One preferred method is to contact a population of astrocytoma cells with an antibody that binds a cell surface astrocytoma marker and isolate the contacted cells by flow cytometry. Typically the antibody used in such a method is detectably labeled, e.g., with a fluorescent molecule, to facilitate sorting of the cells bound by the antibody. Alternatively, the contacted cells are further contacted with a detectably labeled molecule that binds to the antibody. Another method for separating cells to isolate astrocytoma cells or enrich astrocytoma cells in populations of cells is to use magnetic activated cell sorting as described herein.

The astrocytoma markers are particularly useful in determining the presence of astrocytoma cells in a biological sample of a subject. Further description of diagnostic methods is provided elsewhere herein. In addition, as shown herein, the level of expression of astrocytoma markers correlates with astrocytoma stage or grade (i.e., grade I, grade II, grade III or grade IV), which permits accurate staging of disease that does not rely on subjective histological analysis. This also permits the medical practitioner to make decisions as to the most appropriate treatment regimen for subject, including selecting a therapeutic based on the stage of the disease in the subject. It also permits the medical practitioner to follow the course of treatment and to determine if the treatment is effective, or if the disease has progressed to a higher stage despite treatment provided to the subject. In addition, as described further below, the identification of astrocytoma markers provides new therapeutics for treating astrocytomas.

The tumor mass of glioblastoma multiforme (GBM) is comprised of morphologically diverse cells. GBMs contain cells that express neuronal as well as glial markers, suggesting the presence of multipotent neural stem cell (NSC)-like cells. NSCs have the ability to generate three cell lineages: neuron, astrocyte, and oligodendrocyte. Mixed gliomas putatively arise from neural stem cells or from differentiated cell types that acquired stem cell-like properties by either reprogramming or de-differentiation as a consequence of oncogenic mutation. Primary cell cultures obtained from GBM contain a subpopulation of cells that display self-renewal capability (a hallmark of stem-cells) and expresses the NSC markers CD133 (a cell surface marker) and nestin (a cytoskeletal protein), and which grow to form oncospheres (a sphere-like cell structure that grows in suspension). This GBM-derived stem cell population resident in oncospheres can be distinguished from NCS-derived populations that grow into neurospheres when both cultures are grown under conditions that promote cell differentiation. Under these conditions NSC cultures fully differentiate, a state which is irreversible. GBM-derived stem cells however retain the ability to grow into oncospheres and to express NSC markers. CD133-positive GBM-derived stem cells when introduced into mice cause tumor formation in vivo.

The identification of cancer stem cells (CSCs) is important since the stem cell population within a tumor mass is likely to be responsible for the recurrences that occur after conventional cancer treatments. Only a small number of CSCs are required to regenerate the tumor. Thus the cellular heterogeneity of brain tumors may cause conventional bulk therapy to fail to eradicate the tumor-initiating cells, thus allowing for disease relapse and/or progression. It is therefore important for cancer therapy to target and eliminate these stem cells. To date however there is no appropriate marker for isolating or selectively targeting astrocytoma (e.g., GBM) stem cells, or for distinguishing cancer stem cells from NSCs (Yuan X, 2004, Oncogene 23, 9392-9400; Singh SK, 2004, Nature 432, 396-400; Galli R, 2004, Cancer Res. 64, 7011-7021).

The invention provides for the identification, isolation, enrichment, selection and targeting of astrocytoma stem cells. Astrocytoma stem cells are identified, isolated, enriched, selected or targeted through the detection (including binding) or use of "astrocytoma stem cell markers", which are set forth in Table IX: NXPH1, PCDHB14, TMOD1, HOXC9, E2F2, LOC151261 (BX439624), CD99, PDE1A, PPFIBP2, BAIAP2L1, THBS1, SLC45A1, DAAM2, AI272963 (Hs.149361), T03803 (Hs.654945), HSD3B7 and FLJ10489 (AK001351). Some of the astrocytoma stem cell markers are genes that are upregulated in astrocytoma stem cells (NXPH1, PCDHB14, TMOD1, HOXC9, E2F2, LOC151261 (BX439624), CD99), while others are downregulated in astrocytoma stem cells (PDE1A, PPFIBP2, BAIAP2L1, THBS1, SLC45A1, DAAM2, AI272963 (Hs.149361), T03803 (Hs.654945), HSD3B7 and FLJ10489 (AK001351)). The astrocytoma stem cells also are CD133 positive, and thus can be identified, isolated, enriched, selected or targeted by binding of cell-surface antigens, e.g., CD133 found on the surfaces of astrocytoma stem cells, to reagents that specifically bind such antigens.

One of the surface antigens is CD133, which is also known as AC133 and prominin. The AC133 monoclonal antibody is exemplary of antibody embodiments of reagents that recognize CD133 molecules. CD133/AC133/prominin is a membrane protein expressed in various epithelial cells (Weigmann et al., Proc Natl Acad Sci U S A. 94(23):12425-12430, 1997; Corbeil et al., J Cell Sci. 112 (Pt 7):1023-1033, 1999; Corbeil et al., Blood 91(7):2625-2626,1998; Miriglia et al., Blood 91(11):4390-4391, 1998).

Various antibodies that bind AC133 (CD133) are described in U.S. Pat. No. 5,843,333, which is incorporated herein by reference for the teaching of the antibodies and use thereof. The AC133 antigen is a 5-transmembrane cell surface antigen with a molecular weight of 117 kDa. Expression of this, antigen is highly tissue specific, and has been detected on a subset of hematopoietic progenitor cells derived from human bone marrow, fetal bone marrow and liver, cord blood, and adult peripheral blood. These AC133 antibodies are capable of immunoselection for the subset of human cells of interest in this invention. Preferred AC133 monoclonal antibodies can be obtained commercially from Miltenyi Biotec Inc. (Auburn Calif.), including AC133/1-PE antibody (Cat #808-01) and AC133/2-PE antibody (Cat #809-01). For MACS separation, a 50:50 mixture of the monoclonal antibodies is preferred. The high tissue specificity of AC133 expression is particularly advantageous during enrichment for highly purified astrocytoma stem cell populations.

Buck, et al. (U.S. Patent No. 7,037,719) described the isolation of central nervous system (CNS) neural stem cells that express AC133, which can initiate neurospheres (NS-IC) and progenitor cells. The presence of AC133 on cells also has been used to identify, isolate and enrich pancreatic stem cells (Uchida et al., published U.S. Patent Application No. 2006/0205072 A1).

Recently, a novel alternatively spliced variant of human AC133, AC133-2, was reported (Yu et al., J Biol Chem 277:20711-20716, 2002). This novel isoform lacks exon 4, which leads to deletion of 9 amino acids from AC133. According to this report, additional alternative splicing isoforms exist within the open reading frame of human AC133. This report further demonstrates that AC133 transcripts can be alternatively spliced, leading to formation of mRNAs with different 5' untranslated exons. The mouse AC133 homolog prominin similarly has alternative transcripts.

The invention thus provides isolated cell populations that include astrocytoma stem cells, which include glioblastoma multiforme (GBM) stem cells. The astrocytoma stem cells express CD133, i.e., are CD133 positive. Preferably the astrocytoma stem cells express at least one astrocytoma stem cell marker gene product as shown in Example 6. The CD133 expressed by the astrocytoma stem cells can be any of the isoforms of CD133, such as CD133-1 or a splice variant of CD133. A preferred splice variant is CD133-2.

Gene expression in identified, isolated, enriched or selected astrocytoma stem cells can be increased in type and/or in amount relative to astrocytoma cells that are not astrocytoma stem cells. By "increased type" and the like is meant that different and/or more genes are expressed in astrocytoma stem cells relative to non-astrocytoma stem cell astrocytoma cells. By "increased amount" and the like is meant that higher amounts of gene products of genes upregulated in astrocytoma stem cells are expressed as measured by amounts of RNA transcripts or protein. Each of these is demonstrated in the Examples below. Gene expression in astrocytoma stem cells can be decreased in amount relative to astrocytoma cells that are not astrocytoma stem cells. By "decreased amount" and the like is meant that lower amounts of gene products of genes downregulated in astrocytoma stem cells are expressed as measured by amounts of RNA transcripts or protein.

The expression of CD133 and/or one or more astrocytoma gene products, can be determined by standard methods known in the art. These methods include the microarray and QT-PCR methods described herein, as well as immunohistochemistry, and quantitation of expression using fluorescence activated cell sorting and other methods that allow for discriminating between cell types in a population without significantly affecting the ability of the cells to survive the quantitation methods.

In some cases, the astrocytoma stem cells also express one or more additional stem cell markers. Additional stem cell markers that can be used to identify or isolate astrocytoma stem cells include such markers as Stage Specific Embryonic Antigens (SSEAs), ABCG2 and/or Sca-1.

Additional markers can be used, as needed, to further purify or enrich astrocytoma stem cells. Preferably such markers are cell surface markers, such as ABCB5, CD166, integrin β1, integrin α2β1, SOX10, p75NTR, nestin, BCRP1/ABCG2, CD44, CXCR4/CD184, SSEA-1/CD15, and/or CD24.

Additional markers that are not found on astrocytoma stem cells, but are found on astrocytoma cells that are not astrocytoma stem cells, also can be used in enrichment and purification methods, e.g., by removing non-astrocytoma stem cells from a population of astrocytoma cells using various standard methods such as fluorescence activated cell sorting, magnetic activated cell sorting (MACS), killing cells recognized by antibodies against non-astrocytoma markers or astrocytoma stem cell markers, etc.

Astrocytoma stem cells can be isolated by a variety of methods known in the art. These methods include flow cytometry, such as fluorescence activated cell sorting. One preferred method is to contact a population of astrocytoma cells with an antibody that binds CD133 and isolate the contacted cells by flow cytometry. Typically the antibody used in such a method is detectably labeled, e.g., with a fluorescent molecule, to facilitate sorting of the cells bound by the antibody. Alternatively, the contacted cells are further contacted with a detectably labeled molecule that binds to the antibody.

Another method for separating cells to isolate astrocytoma stem cells or enrich astrocytoma stem cells in populations of cells is to use magnetic activated cell sorting. In one such method, astrocytoma stem cells are isolated by contacting a population of astrocytoma cells with immunomagnetic particles (e.g., beads) that include an antibody that binds to CD133, and then isolating the immunomagnetic particles. Still other methods for separating cells are known to the skilled person.

Thus the invention provides methods for isolating astrocytoma stem cells and enriching astrocytoma stem cells in cell populations. The methods generally include contacting a population of astrocytoma cells with one or more labeled agents, such as one or more antibodiesthat bind to CD133 and a gene product upregulated in astrocytoma stem cells, and then isolating the astrocytoma stem cells based on the binding of the labeled antibodies to the astrocytoma stem cells. The population of astrocytoma cells can be any population of cells that is suspected of having astrocytoma stem cells. These may include populations of cells obtained from a tumor sample, such as a biopsy, or a cell line. The astrocytoma stem cells can be isolated for further study, for establishment of cultures, for preparing models of disease, and the like.

The isolated cell populations of the invention preferably are enriched for astrocytoma stem cells, i.e., have a higher proportion of astrocytoma stem cells than a native cell population (taken from a subject or tumor sample) or an unenriched population of cultured cells. In a preferred embodiment, the astrocytoma stem cells are enriched in the cell population by at least 2-fold relative to an unenriched cell population. More preferably, the astrocytoma stem cells are enriched in the cell population by at least 5-fold relative to an unenriched cell population. Still more preferably, the astrocytoma stem cells are enriched in the cell population by at least 10-fold relative to an unenriched cell population. Still more preferably, the astrocytoma stem cells are enriched in the cell population by at least 20-fold relative to an unenriched cell population. Yet more preferably, the astrocytoma stem cells are enriched in the cell population by at least 50-fold relative to an unenriched cell population.

The identification and characterization of astrocytoma cells or astrocytoma stem cells (and optionally the isolation of astrocytoma stem cells and/or enrichment of astrocytoma stem cells in cell populations) by specific markers for the astrocytoma cells ("astrocytoma markers") or astrocytoma stem cells ("astrocytoma stem cell markers") also facilitates therapeutic and diagnostic methods based on the properties of the astrocytoma cells or astrocytoma stem cells. These methods generally utilize the ability to selectively recognize astrocytoma cells or astrocytoma stem cells and optionally isolate the astrocytoma cells or astrocytoma stem cells, kill the astrocytoma cells or astrocytoma stem cells, or inhibit proliferation of the astrocytoma cells or astrocytoma stem cells, thereby permitting treatment or diagnosis of cancer, particularly astrocytoma.

The invention provides for treatment of cancer in a subject and for killing or inhibiting proliferation of astrocytoma cells or astrocytoma stem cells. The terms "therapy", "therapeutic", "treat" or "treatment" are intended to include prophylaxis, amelioration, prevention or cure from the disease. The treatment of cancer in this manner can be a monotherapy, i.e., treating a patient only by using the methods and compositions described herein to kill the astrocytoma cells or astrocytoma stem cells or inhibit proliferation of the astrocytoma cells or astrocytoma stem cells. However, the cancer treatment also can be an adjunct therapy to one or more other therapies, including immune therapies such as vaccination, surgery, radiation therapy, and chemotherapy. Aspects of these methods are described in greater detail elsewhere herein.

Particular methods for killing or inhibiting the proliferation of astrocytoma cells or astrocytoma stem cells, include contacting a population of astrocytoma cells (such as an astrocytoma tumor, cell line or cell sample such as a biopsy) with an agent or combination of agents selectively targeted to astrocytoma cells or astrocytoma stem cells of the population of astrocytoma cells. By "selectively targeted" is meant that the agent or combination of agents selectively recognizes and binds to astrocytoma cells or astrocytoma stem cells as compared to non-astrocytoma stem cells in a tissue or cell population. The agent or combination of agents can effectively kill the astrocytoma cells or astrocytoma stem cells or inhibit the proliferation of astrocytoma cells or astrocytoma stem cells by one of several mechanisms, such as by induction of apoptosis, bringing into close proximity a cytotoxic or cytostatic agent, or attracting other cells such as cytotoxic T lymphocytes or macrophages that can kill or inhibit proliferation of the astrocytoma cells or astrocytoma stem cells. By "cytotoxic or cytostatic agent" is meant an agent (e.g., molecule) that kills or reduces proliferation of cells.

The agent in certain preferred embodiments is an antibody, or antigen-binding fragment thereof, which binds CD133 and/or a gene product of a gene upregulated in astrocytoma cells or astrocytoma stem cells. The antibody or fragment can be a bispecific or multispecific antibody or fragment that binds CD133 and at least one gene product of a gene upregulated in astrocytoma stem cells. The antibody or fragment can be a bispecific or multispecific antibody or fragment that binds at least two gene product of a gene upregulated in astrocytoma cells or astrocytoma stem cells. By "bispecific" is meant an agent (e.g., antibody or antigen-binding fragment thereof) that binds to two different antigens. By "multispecific" is meant an agent (e.g., antibody or antigen-binding fragment thereof) that binds to at least two different antigens, preferably at least three antigens.

In some cases, the labeled antibodies (or other agents known to those of skill in the art to selectively bind to proteins) are labeled with detectable molecules, preferably fluorescent molecules, or magnetic entities, such as magnetic particles. These molecules or entities can be used to facilitate detection and/or separation of the astrocytoma cells or astrocytoma stem cells from other cells of a cell population. As such, astrocytoma cells or astrocytoma stem cells can be isolated by a variety of methods known in the art, including preferred methods such as fluorescence-activated cell sorting and magnetic activated cell sorting.

Labeled agents (e.g., antibodies) can be used sequentially or simultaneously to isolate astrocytoma cells or astrocytoma stem cells. In one exemplary method, astrocytoma stem cells are contacted first with labeled antibodies that bind to CD133, followed by contacting the cells with labeled antibodies that bind to a gene product of a gene upregulated in astrocytoma stem cells. In another exemplary method, astrocytoma stem cells are contacted first with labeled antibodies that bind to a gene product of a gene upregulated in astrocytoma stem cells, followed by contacting the cells with labeled antibodies that bind to CD133. In a third exemplary method, astrocytoma stem cells are contacted simultaneously with labeled antibodies that bind to CD133 and with labeled antibodies that bind to a gene product of a gene upregulated in astrocytoma stem cells. Similar methods can be applied to astrocytoma cells using (for example) antibodies that bind astrocytoma markers. In each of these methods, the cells can be isolated after each labeling step, or can be isolated after labeling with both agents. In a further exemplary method, astrocytoma stem cells are contacted first with labeled antibodies that bind to CD133, followed by isolating the CD133+ cells, then determining the expression of a gene product of a gene upregulated or downregulated in astrocytoma stem cells (e.g., as shown in Example 4).

In the various methods described herein, clones af CD133+ cells can be isolated (e.g., by subjecting the CD133+ cells to limiting dilution) and cultured prior to further analysis (such as gene expression analysis) to provide clonal populations of cells from which the astrocytoma stem cells having specific gene expression profiles can be isolated.

Diagnostic methods based on identification and characterization of astrocytoma cells or astrocytoma stem cells include identifying the presence of astrocytoma cells or astrocytoma stem cells in an animal or subject *in vivo*, *ex vivo* or *in vitro*. For astrocytoma stem cells, such *in vivo* methods include administering to the animal a detectably labeled agent that binds to CD133 and/or a gene product of a gene upregulated in astrocytoma stem cells. For diagnostic procedures on internal cells, the detectably labeled agent circulates and binds to astrocytoma stem cells in the body of the animal or subject, thereby facilitating detection of astrocytoma stem cells. The agent also can be administered intratumorally, i.e., by direct injection into a tumor. Similar methods are useful for identification and characterization of astrocytoma cells using one or more detectably labeled agents that bind to gene products of genes upregulated in astrocytoma cells, i.e., astrocytoma markers.

For *ex vivo* or *in vitro* methods, a cell sample, tissue sample or other population of cells suspected of having astrocytoma stem cells is contacted with a detectably labeled agent that binds to CD133 and a gene product of a gene upregulated in astrocytoma stem cells, and detection of binding of the detectably labeled agent indicates the presence of astrocytoma stem cells in the cell sample or other population of cells. Similar methods are useful for identification and characterization of astrocytoma cells using one or more detectably labeled agents that bind to gene products of genes upregulated in astrocytoma cells, i.e., astrocytoma markers.

Identification of astrocytoma cells or astrocytoma stem cells in the population can contribute to diagnosis or classification of a tumor as an astrocytoma. The methods therefore may be of use in identifying metastatic astrocytoma tumors.

The diagnostic agent in certain preferred embodiments is an antibody, or antigen-binding fragment thereof, which binds CD133 (for astrocytoma stem cells) and/or one or more gene products of genes upregulated in astrocytoma cells or astrocytoma stem cells. The antibody or fragment can be a bispecific or multispecific antibody or fragment that binds CD133 and at least one gene product of a gene upregulated in astrocytoma stem cells, or at least two gene products of genes upregulated in astrocytoma cells.

The invention thus includes diagnosing or monitoring cancer in a subject by determining the presence or amount of astrocytoma stem cells, which express CD133 and preferably one or more gene products of a gene upregulated in astrocytoma stem cells, more preferably CD99, HOXC9 or E2F2. The invention also includes diagnosing or monitoring cancer in a subject by determining the presence or amount of astrocytoma cells, which express one or more gene products of a gene upregulated in astrocytoma cells, such as KIAA0101, ASPM, LOX, HOXA5, COL6A2, PLP2, DKFZp762E1312 and/or MELK.

In preferred embodiments, this determination is performed by assaying a biological sample obtained from the subject, such as a tumor biopsy (preferably a biopsy of an astrocytoma or cells suspected of being astrocytoma cells), cell scraping, serum, blood, or lymph node fluid, for the presence of astrocytoma cells or astrocytoma stem cells as described herein. This determination may also be performed by assaying a tissue or cells from the subject for the presence of one or more cells expressing both CD133 and one or more gene products of genes upregulated or downregulated in astrocytoma stem cells (including nucleic acid molecules) described herein.

For example, the invention permits more accurate determinations of prognosis, based on the existence of astrocytoma stem cells or astrocytoma cells in a tumor. A tumor with fewer or no astrocytoma stem cells or astrocytoma cells would be expected to have less (or no) repopulation capacity, and therefore be less aggressive, less metastatic, and/or less able to withstand cancer therapy.

Measurement of the astrocytoma cells or astrocytoma stem cells in a tumor over time by sequential determinations permits monitoring of the disease and/or the effects of a course of treatment. For example, a cell sample, such as a biopsy, may be obtained from a subject, tested for the existence or quantity of astrocytoma cells or astrocytoma stem cells, and at a second, subsequent time, another sample, may be obtained from the subject and similarly tested. The results of the first and second (or subsequent) tests can be compared as a measure of the onset, regression or progression of cancer, or, if cancer treatment was undertaken during the interval between obtaining the samples, the effectiveness of the treatment may be evaluated by comparing the results of the two tests.

The invention also permits staging of disease based on the differential expression in astrocytoma cells of various astrocytoma markers as shown in the Examples. Measurement of the astrocytoma markers in biological samples (e.g., tumor biopsy samples) over time by sequential determinations permits monitoring of the disease and/or the effects of a course of treatment. For example, a cell sample, such as a biopsy, may be obtained from a subject, tested for the existence or quantity of astrocytoma cells and/or for the level of expression of astrocytoma markers. At a second time, and optionally subsequent times, another sample(s) may be obtained from the subject and similarly tested. The results of the first and second (or subsequent) tests can be compared as a measure of the onset, regression or progression of cancer (for example, by comparing the stage of the astrocytoma in the respective samples), or, if cancer treatment was undertaken during the interval between obtaining the samples, the effectiveness of the treatment may be evaluated by comparing the results of the two tests. In addition, determination of the stage of astrocytoma cells based on analysis of astrocytoma markers permits more accurate determination of prognosis.

Diagnostic methods of the invention may involve determining the expression of one or more of markers of astrocytoma cells or astrocytoma stem cells described herein, such as gene products of genes upregulated or downregulated in astrocytoma cells or astrocytoma stem cells, or the nucleic acid molecules that encode them. Such determinations can be carried out via any standard nucleic acid assay, including the polymerase chain reaction or assaying with hybridization probes, which may be labeled, or by assaying biological samples with binding partners (e.g., antibodies) for genes product of genes upregulated or downregulated in astrocytoma cells or astrocytoma stem cells using standard methodologies.

The diagnostic methods of the invention can be used to detect the presence of a cancer associated with astrocytoma cells or astrocytoma stem cells, such as astrocytomas of grades I-IV, by determining the amount and/or or stage of astrocytoma cells or the amount of astrocytoma stem cells present in a tissue or cell sample, as well as to assess the progression and/or regression of the cancer such as in response to treatment (e.g., chemotherapy, radiation).

It is expected that a typical non-cancer tissue or cell sample will have zero or a very low level of expression of astrocytoma markers, whereas an astrocytoma cancer tissue or cell sample will have a significantly higher level of expression of astrocytoma markers, which can be termed an "aberrant level" of astrocytoma marker expression. The relative levels of expression of astrocytoma markers in astrocytoma cells/tissues (grades I-IV) versus non-neoplastic cells/tissues is shown in the Examples below. As used herein, an aberrant level of astrocytoma marker expression is intended to include any level of astrocytoma marker expression that is different by a statistically significant amount from the expected level of astrocytoma marker expression. For example, astrocytoma marker expression in a tissue that is not expected to have such expression would be an example of an "aberrant level" of astrocytoma marker(s). Likewise, a significantly higher level of astrocytoma marker expression than expected is another example of an "aberrant level" of astrocytoma stem cells. Therefore, a determination of the level of astrocytoma marker expression is diagnostic of cancer if the level of expression is above a baseline level determined for that tissue type. The baseline level of expression can be determined using standard methods known to those of skill in the art. Such methods include, for example, assaying a number of histologically normal tissue samples from subjects that are clinically normal (i.e., do not have clinical signs of cancer in that tissue type) and determining the mean level of astrocytoma marker expression for the samples.

It is expected that a typical non-cancer tissue or cell sample will have zero or a very low number of astrocytoma stem cells, whereas a cancer tissue or cell sample will have a significantly higher number of astrocytoma stem cells, which can be termed an "aberrant number" of astrocytoma stem cells. As used herein, aberrant numbers of astrocytoma stem cells is intended to include any number of astrocytoma stem cells that is different by a statistically significant amount from the expected amount of astrocytoma stem cells. For example, the presence of astrocytoma stem cells in a tissue that is not expected to have such cells would be an example of an "aberrant number" of astrocytoma stem cells. Likewise, a significantly higher number of astrocytoma stem cells than expected is another example of an "aberrant number" of astrocytoma stem cells. Therefore, a determination of the presence and/or amount of astrocytoma stem cells is diagnostic of cancer if the level of expression is above a baseline level determined for that tissue type. The baseline amount of astrocytoma stem cells can be determined using standard methods known to those of skill in the art. Such methods include, for example, assaying a number of histologically normal tissue samples from subjects that are clinically normal (i.e., do not have clinical signs of cancer in that tissue type) and determining the mean number or amount of astrocytoma stem cells and/or the expression of astrocytoma stem cell markers for the samples.

The level of expression of astrocytoma markers can indicate cancer in the tissue when the level of expression of astrocytoma markers is significantly more in the tissue than in a control sample, e.g., a negative control sample. In some embodiments, the level of expression of astrocytoma markers in the tissue or cells being examined that is at least about 5%, 10%, 20%, 30%, 40%, 50%, 60%, 70%, 80%, 90%, 100%, 150%, 200%, 250%, 300%, 400 %, 500% or 1000% more than the level of expression of astrocytoma markers in negative control tissue or cells indicates cancer in the tissue or cells being examined.

As used herein the term "control" typically means samples of materials tested in parallel with the experimental materials, although the control may be tested separately from the experimental materials, and may be an historical control value. Examples include samples from control cell populations or control tissue samples generated through manufacture to be tested in parallel with the experimental samples.

Typically the negative control will be based on apparently healthy individuals in an appropriate age bracket. Positive control cell populations or tissue samples, i.e., that express astrocytoma markers, can be used to verify experimental procedures.

Determining the presence of astrocytoma cells or astrocytoma stem cells can, in some aspects, be performed by determining the level (i.e., presence or amount) of expression of one or more astrocytoma markers or astrocytoma stem cell marker nucleic acid molecules and/or polypeptides (such as one or more gene products of a gene upregulated or down regulated in astrocytoma cells and/or astrocytoma stem cells). The expression of one or more astrocytoma markers or astrocytoma stem cell markers may be determined using routine methods known to those of ordinary skill in the art. These methods include, but are not limited to: direct RNA amplification, reverse transcription of RNA to cDNA, real-time RT-PCR, amplification of cDNA, hybridization, and immunologically based assay methods, which include, but are not limited to immunohistochemistry, antibody sandwich capture assay, ELISA, and enzyme-linked immunospot assay (EliSpot assay). For example, the determination of the presence or level of one or more astrocytoma marker or astrocytoma stem cell marker nucleic acid molecules in a subject or tissue can be carried out via any standard nucleic acid determination assay, including the polymerase chain reaction, or assaying with labeled hybridization probes. Such hybridization methods include, but are not limited to, microarray techniques as described herein.

These methods of determining the presence and/or level of the one or more astrocytoma markers or astrocytoma stem cell markers in cells and tissues may include use of labels to monitor the presence of the astrocytoma cells or astrocytoma stem cells. Such labels may include, but are not limited to radiolabels or chemiluminescent labels, which may be utilized to determine whether one or more astrocytoma markers or astrocytoma stem cell markers is expressed in a cell or tissue, and to determine the level of expression in the cell or tissue. For example, as described elsewhere herein, a fluorescently labeled or radiolabeled antibody that selectively binds to one or more astrocytoma markers or astrocytoma stem cell markers may be contacted with a tissue or cell to visualize the polypeptide *in vitro* or *in vivo*. These and other *in vitro* and *in vivo* imaging methods for determining the presence of the one or more astrocytoma markers or astrocytoma stem cell markers are well known to those of ordinary skill in the art.

In another aspect of the invention methods to predict the likelihood of tumor recurrence are provided. In one embodiment gene expression of one or more biomarker(s) is determined from a tissue sample from a subject obtained from a first surgery and the expression level of the one or more biomarker is compared to the gene expression in non-neoplastic tissue samples. The first surgery may be carried out for diagnostic and/or therapeutical reasons. The expression level of the one or more biomarker can be determined by any method described herein or known in the art, e.g. QT-PCR and/or immunohistochemistry. A level of the one or more biomarker that is higher than the level determined in non-neoplastic tissue indicates that the subject having undergone the first surgery has a higher likelihood that a tumor will recur than when the level of the one or more biomarker is not elevated as compared to non-neoplastic tissue. An elevated level of the one or more biomarker as determined by the method described above also indicates that the subject having a higher likelihood that a tumor will recur may be a candidate for more aggressive treatment than otherwise indicated, according to the current standard of care, to potentially prevent or delay recurrence. Appropriate methods of intervention and levles of aggressivenes may be determined, for example by a physician or specialist, depending on the specific circumstances and may vary. In a preferred embodiment the tissue sample is a brain sample and the tumor is a glioblastoma. In a preferred embodiment the one or more biomarker is MELK and/or PLP2. In another embodiment the one or more biomarker is selected from the group consisting of KIAA0101, ASPM, LOX, HOXA5, COL6A2, DKFZp762E1312, CD99, HOXC9 and E2F2.

The invention includes kits for assaying the presence and/or expression of one or more astrocytoma markers or astrocytoma stem cell markers, preferably antibodies that specifically bind to one or more astrocytoma markers or astrocytoma stem cell markers. An example of such a kit may include an antibody, or antigen-binding fragment thereof, that binds specifically to one or more astrocytoma markers or astrocytoma stem cell markers, such as one or more gene products of a genes upregulated or downregulated in astrocytoma stem cells. The antibody, or antigen-binding fragment thereof, may be applied to a tissue or cell sample from a patient with cancer and the sample then processed to assess whether specific binding occurs between the antibody and one or more astrocytoma markers or astrocytoma stem cell markers. In addition, the antibody or antigen-binding fragment thereof may be applied to a body fluid sample, such as serum, from a subject, either suspected of having cancer, diagnosed with cancer, or believed to be free of cancer. As will be understood by one of skill in the art, such binding assays may also be performed with a sample or object contacted with an antibody and/or gene product of a gene upregulated in astrocytoma cells or astrocytoma stem cells that is in solution, for example in a 96-well plate or applied directly to an object surface.

Another example of a kit of the invention is a kit that provides components necessary to determine the level of expression of one or more astrocytoma markers or astrocytoma stem cell markers. Such components may include primers useful for amplification of one or more astrocytoma markers or astrocytoma stem cell markers and/or other chemicals for PCR amplification. Another example of a kit of the invention is a kit that provides components necessary to determine the level of expression of one or more astrocytoma markers or astrocytoma stem cell markers using a method of hybridization.

The foregoing kits can include instructions or other printed material on how to use the various components of the kits for diagnostic purposes.

As used herein, a "subject" is preferably a human, non-human primate, cow, horse, pig, sheep, goat, dog, cat or rodent. In all embodiments, human subjects are preferred. In some embodiments, the subject is suspected of having cancer or has been diagnosed with cancer.

As used herein, a "biological sample" includes, but is not limited to: tissue, cells and/or body fluid (e.g. serum, blood, lymph node fluid, cerebrospinal fluid, etc.). The fluid sample may include cells and/or fluid. The tissue and cells may be obtained from a subject or may be grown in culture (e.g., from a cell line). As used herein, a biological sample is body fluid, tissue or cells obtained from a subject using methods well-known to those of ordinary skill in the related medical arts.

As used herein, the "nucleic acid molecules that encode" means the nucleic acid molecules that code for the one or more astrocytoma markers or astrocytoma stem cell markers. These nucleic acid molecules may be DNA or may be RNA (e.g. mRNA). The one or more astrocytoma markers or astrocytoma stem cell markers also encompass variants of the molecules described herein. These variants may be splice variants or allelic variants, as are known in the art. Variants of the nucleic acid molecules for use as described herein are intended to include homologs and alleles. In all embodiments, human astrocytoma markers or astrocytoma stem cell markers (including gene products of genes upregulated or downregulated in astrocytoma stem cells) and the encoding nucleic acid molecules thereof, are preferred.

As used herein the term "isolated nucleic acid molecule" means: (i) amplified *in vitro* by, for example, polymerase chain reaction (PCR); (ii) recombinantly produced by cloning; (iii) purified, as by cleavage and gel separation; or (iv) synthesized by, for example, chemical synthesis. An isolated nucleic acid is one which is readily manipulable by recombinant DNA techniques well known in the art. Thus, a nucleotide sequence contained in a vector in which 5' and 3' restriction sites are known or for which polymerase chain reaction (PCR) primer sequences have been disclosed is considered isolated but a nucleic acid sequence existing in its native state in its natural host is not. An isolated nucleic acid may be substantially purified, but need not be. For example, a nucleic acid that is isolated within a cloning or expression vector is not pure in that it may comprise only a tiny percentage of the material in the cell in which it resides. Such a nucleic acid is isolated, however, as the term is used herein because it is readily manipulable by standard techniques known to those of ordinary skill in the art.

Optimal alignment of sequences for comparison may be conducted using programs such as BLAST, publicly available on the National Library of Medicine website. Other programs such as UniGene (The National Library of Medicine website), SAGE Anatomic Reviewer and its Virtual Northern tool, (The Cancer Genome Anatomy Project CGAP website) are also publicly available. Preferably, the "percentage of sequence identity" is determined by comparing two optimally aligned sequences over a window of comparison of at least 20 positions, wherein the portion of the polynucleotide or polypeptide sequence in the comparison window may comprise additions or deletions (i.e., gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases or amino acid residue occurs in both sequences to yield the number of matched positions, dividing the number of matched positions by the total number of positions in the reference sequence (i.e., the window size) and multiplying the results by 100 to yield the percentage of sequence identity.

In general, homologs, alleles and preferred variants of the one or more astrocytoma markers or astrocytoma stem cell markers typically will share at least 90% nucleotide identity and/or at least 95% amino acid identity to the sequences of astrocytoma stem cell marker nucleic acids and polypeptides, respectively, in some instances will share at least 95% nucleotide identity and/or at least 97% amino acid identity, in other instances will share at least 97% nucleotide identity and/or at least 98% amino acid identity, in other instances will share at least 99% nucleotide identity and/or at least 99% amino acid identity, and in other instances will share at least 99.5% nucleotide identity and/or at least 99.5% amino acid identity.

According to yet another aspect of the invention, an expression vector comprising any of the astrocytoma markers or astrocytoma stem cell markers, preferably operably linked to a promoter, is provided. In a related aspect, host cells transformed or transfected with such expression vectors also are provided. As used herein, a "vector" may be any of a number of nucleic acid molecules into which a desired sequence may be inserted by restriction and ligation for transport between different genetic environments or for expression in a host cell. Vectors are typically composed of DNA although RNA vectors are also available. Vectors include, but are not limited to, plasmids, phagemids, and virus genomes. A cloning vector is one which is able to replicate in a host cell, and which is further characterized by one or more endonuclease restriction sites at which the vector may be cut in a determinable fashion and into which a desired DNA sequence may be ligated such that the new recombinant vector retains its ability to replicate in the host cell. In the case of plasmids, replication of the desired sequence may occur many times as the plasmid increases in copy number within the host bacterium or just a single time per host before the host reproduces by mitosis. In the case of phage, replication may occur actively during a lytic phase or passively during a lysogenic phase. An expression vector is one into which a desired DNA sequence may be inserted by restriction and ligation such that it is operably joined to regulatory sequences and may be expressed as an RNA transcript. Vectors may further contain one or more marker sequences suitable for use in the identification of cells which have or have not been transformed or transfected with the vector. Markers include, for example, genes encoding proteins which increase or decrease either resistance or sensitivity to antibiotics or other compounds, genes which encode enzymes whose activities are detectable by standard assays known in the art, e.g., beta-galactosidase or alkaline phosphatase, and genes which visibly affect the phenotype of transformed or transfected cells, hosts, colonies or plaques, e.g., green fluorescent protein. Preferred vectors are those capable of autonomous replication and expression of the structural gene products present in the DNA segments to which they are operably joined.

As used herein, a coding sequence and regulatory sequences are said to be "operably joined" when they are covalently linked in such a way as to place the expression or transcription of the coding sequence under the influence or control of the regulatory sequences. As used herein, "operably joined" and "operably linked" are used interchangeably and should be construed to have the same meaning. If it is desired that the coding sequences be translated into a functional protein, two DNA sequences are said to be operably joined if induction of a promoter in the 5' regulatory sequences results in the transcription of the coding sequence and if the nature of the linkage between the two DNA sequences does not (1) result in the introduction of a frame-shift mutation, (2) interfere with the ability of the promoter region to direct the transcription of the coding sequences, or (3) interfere with the ability of the corresponding RNA transcript to be translated into a protein. Thus, a promoter region is operably joined to a coding sequence if the promoter region is capable of effecting transcription of that DNA sequence such that the resulting transcript can be translated into the desired protein or polypeptide.

The precise nature of the regulatory sequences needed for gene expression may vary between species or cell types, but shall in general include, as necessary, 5' non-transcribed and 5' non-translated sequences involved with the initiation of transcription and translation respectively, such as a TATA box, capping sequence, CAAT sequence, and the like. Often, such 5' non-transcribed regulatory sequences will include a promoter region which includes a promoter sequence for transcriptional control of the operably joined gene. Regulatory sequences may also include enhancer sequences or upstream activator sequences as desired. The vectors of the invention may optionally include 5' leader or signal sequences. The choice and design of an appropriate vector is within the ability and discretion of one of ordinary skill in the art.

It will also be recognized that the invention embraces the use of the one or more astrocytoma markers or astrocytoma stem cell markers and genomic sequences in expression vectors, as well as to transfect host cells and cell lines, be these prokaryotic, e.g., *E. coli,* or eukaryotic, e.g., CHO cells, COS cells, yeast expression systems, and recombinant baculovirus expression in insect cells. Especially useful are mammalian cells such as human, mouse, hamster, pig, goat, primate, etc. They may be of a wide variety of tissue types, including mast cells, fibroblasts, oocytes, and lymphocytes, and may be primary cells and cell lines. Specific examples include dendritic cells, peripheral blood leukocytes, bone marrow stem cells and embryonic stem cells. The expression vectors require that the pertinent sequence, i.e., those nucleic acids described herein, be operably linked to a promoter.

The invention, in one aspect, also permits the construction of "knock-outs" and "knock-ins" in cells and in animals of one or more of the astrocytoma stem cell marker genes, providing materials for studying certain aspects of cancer and immune system responses to cancer.

Expression vectors containing all the necessary elements for expression are commercially available and known to those skilled in the art. Cells are genetically engineered by the introduction into the cells of heterologous DNA or RNA encoding one or more astrocytoma markers or astrocytoma stem cell markers, fragments, or variants thereof. The heterologous DNA or RNA is placed under operable control of transcriptional elements to permit the expression of the heterologous DNA in the host cell.

Preferred systems for mRNA expression in mammalian cells are those such as pcDNA/V5-GW/D-TOPO® and pcDNA3.1 (Invitrogen) that contain a selectable marker (which facilitates the selection of stably transfected cell lines) and contain the human cytomegalovirus (CMV) enhancer-promoter sequences. Additionally, suitable for expression in primate or canine cell lines is the pCEP4 vector (Invitrogen), which contains an Epstein Barr virus (EBV) origin of replication, facilitating the maintenance of plasmid as a multicopy extrachromosomal element.

The invention also involves the use of agents such as polypeptides that bind to one or more astrocytoma markers or astrocytoma stem cell markers, such as gene products of genes upregulated in astrocytoma cells or astrocytoma stem cells. Such agents can be used in methods of the invention including the diagnosis and/or treatment of cancer. Such binding agents can be used, for example, in screening assays to detect the presence or absence of astrocytoma cells or astrocytoma stem cells and can be used in quantitative assays to determine numbers of astrocytoma cells or astrocytoma stem cells in biological samples and cells. Such agents also may be used to inhibit the native activity of the one or more astrocytoma markers or astrocytoma stem cell markers, for example, by binding to such polypeptides.

In preferred embodiments, the binding polypeptide is an antibody or antibody fragment, more preferably, an Fab or F(ab)₂ fragment of an antibody. Typically, the fragment includes a CDR3 region that is selective for the one or more astrocytoma markers or astrocytoma stem cell markers. Any of the various types of antibodies can be used for this purpose, including polyclonal antibodies, monoclonal antibodies, humanized antibodies, and chimeric antibodies.

Thus, the invention provides agents which bind to one or more astrocytoma markers or astrocytoma stem cell markers. Such binding partners can be used in screening assays to detect the presence or absence of astrocytoma cells or astrocytoma stem cells and in purification protocols to isolate such astrocytoma cells or astrocytoma stem cells. Likewise, such binding partners can be used to selectively target drugs, toxins or other molecules (including detectable diagnostic molecules) to cells which express one or more astrocytoma markers or astrocytoma stem cell markers. In this manner, for example, cells present in solid or non-solid tumors that express one or more astrocytoma markers or astrocytoma stem cell markers can be treated with cytotoxic compounds that are selective for the one or more astrocytoma markers or astrocytoma stem cell markers (nucleic acids and/or antigens). Such binding agents also can be used to inhibit the native biological activity of the one or more astrocytoma markers or astrocytoma stem cell markers, for example, to further characterize the functions of these molecules.

The antibodies of the present invention are prepared by any of a variety of methods, including administering a protein, fragments of a protein, cells expressing the protein or fragments thereof and the like to an animal to induce polyclonal antibodies. The production of monoclonal antibodies is well known in the art. As detailed herein, such antibodies may be used, for example, to identify astrocytoma cells or astrocytoma stem cells in tissues or to purify astrocytoma cells or astrocytoma stem cells. Antibodies also may be coupled to specific labeling agents or imaging agents, including, but not limited to a molecule preferably selected from the group consisting of fluorescent, enzyme, radioactive, metallic, biotin, chemiluminescent, bioluminescent, chromophore, or colored, etc. In some aspects of the invention, a label may be a combination of the foregoing molecule types.

Significantly, as is well-known in the art, only a small portion of an antibody molecule, the paratope, is involved in the binding of the antibody to its epitope (see, in general, Clark, W.R. (1986) The Experimental Foundations of Modem Immunology Wiley & Sons, Inc., New York; Roitt, I. (1991) Essential Immunology, 7th Ed., Blackwell Scientific Publications, Oxford). The pFc' and Fc regions, for example, are effectors of the complement cascade but are not involved in antigen binding. An antibody from which the pFc' region has been enzymatically cleaved, or which has been produced without the pFc' region, designated an F(ab')2 fragment, retains both of the antigen binding sites of an intact antibody. Similarly, an antibody from which the Fc region has been enzymatically cleaved, or which has been produced without the Fc region, designated an Fab fragment, retains one of the antigen binding sites of an intact antibody molecule. Fab fragments consist of a covalently bound antibody light chain and a portion of the antibody heavy chain denoted Fd. The Fd fragments are the major determinant of antibody specificity (a single Fd fragment may be associated with up to ten different light chains without altering antibody specificity) and Fd fragments retain epitope-binding ability in isolation.

Within the antigen-binding portion of an antibody, as is well-known in the art, there are complementarity determining regions (CDRs), which directly interact with the epitope of the antigen, and framework regions (FRs), which maintain the tertiary structure of the paratope (see, in general, Clark, 1986; Roitt, 1991). In both the heavy chain Fd fragment and the light chain of IgG immunoglobulins, there are four framework regions (FR1 through FR4) separated respectively by three complementarity determining regions (CDR1 through CDR3). The CDRs, and in particular the CDR3 regions, and more particularly the heavy chain CDR3, are largely responsible for antibody specificity.

It is now well-established in the art that the non-CDR regions of a mammalian antibody may be replaced with similar regions of nonspecific or heterospecific antibodies while retaining the epitopic specificity of the original antibody. This is most clearly manifested in the development and use of "humanized" antibodies in which non-human CDRs are covalently joined to human FR and/or Fc/pFc' regions to produce a functional antibody. See, e.g., U.S. patents 4,816,567, 5,225,539, 5,585,089, 5,693,762, and 5,859,205.

Fully human monoclonal antibodies also can be prepared by immunizing mice transgenic for large portions of human immunoglobulin heavy and light chain loci. Following immunization of these mice (e.g., XenoMouse (Abgenix), HuMAb mice (Medarex/GenPharm)), monoclonal antibodies can be prepared according to standard hybridoma technology. These monoclonal antibodies will have human immunoglobulin amino acid sequences and therefore will not provoke human anti-mouse antibody (HAMA) responses when administered to humans.

Thus, as will be apparent to one of ordinary skill in the art, the present invention also provides for F(ab')₂, Fab, Fv, and Fd fragments; chimeric antibodies in which the Fc and/or FR and/or CDR1 and/or CDR2 and/or light chain CDR3 regions have been replaced by homologous human or non-human sequences; chimeric F(ab')2 fragment antibodies in which the FR and/or CDR1 and/or CDR2 and/or light chain CDR3 regions have been replaced by homologous human or non-human sequences; chimeric Fab fragment antibodies in which the FR and/or CDR1 and/or CDR2 and/or light chain CDR3 regions have been replaced by homologous human or non-human sequences; and chimeric Fd fragment antibodies in which the FR and/or CDR1 and/or CDR2 regions have been replaced by homologous human or non-human sequences. The present invention also includes so-called single chain antibodies (e.g., ScFv), (single) domain antibodies, and other intracellular antibodies. Domain antibodies, camelid and camelized antibodies and fragments thereof, such as those described in patents and published patent applications of Ablynx NV and Domantis also can be used as described herein.

Thus, the invention involves polypeptides of numerous size and type that bind specifically to one or more astrocytoma markers or astrocytoma stem cell markers. These polypeptides may be derived also from sources other than antibody technology. For example, such polypeptide binding agents can be provided by degenerate peptide libraries which can be readily prepared in solution, in immobilized form or as phage display libraries. Combinatorial libraries also can be synthesized of peptides containing one or more amino acids. Libraries further can be synthesized of peptides and non-peptide synthetic moieties.

The one or more astrocytoma markers or astrocytoma stem cell markers can be used to screen peptide libraries, including phage display libraries, to identify and select peptide binding partners of the one or more astrocytoma markers or astrocytoma stem cell markers. Such molecules can be used, as described herein, for screening assays, for diagnostic assays, for purification protocols or for targeting drugs, toxins and/or labeling agents (e.g., radioisotopes, fluorescent molecules, etc.) to astrocytoma cells or astrocytoma stem cells.

Phage display can be particularly effective in identifying binding agents useful according to the invention. Briefly, one prepares a phage library (using e.g. m13, fd, or lambda phage), displaying inserts from 4 to about 80 amino acid residues using conventional procedures. The inserts may represent, for example, a completely degenerate or biased array. One then can select phage-bearing inserts which bind to one or more astrocytoma markers or astrocytoma stem cell markers. This process can be repeated through several cycles of reselection of phage that bind to the one or more astrocytoma markers or astrocytoma stem cell markers. Repeated rounds lead to enrichment of phage bearing particular sequences. DNA sequence analysis can be conducted to identify the sequences of the expressed polypeptides. The minimal linear portion of the sequence that binds to the one or more astrocytoma markers or astrocytoma stem cell markers can be determined. One can repeat the procedure using a biased library containing inserts containing part or all of the minimal linear portion plus one or more additional degenerate residues upstream or downstream thereof. Yeast two-hybrid screening methods also may be used to identify polypeptides that bind to the one or more astrocytoma markers or astrocytoma stem cell markers, and thereby to astrocytoma stem cells.

As detailed herein, the foregoing antibodies, fragments and other binding agents may be used to identify tissues with normal or aberrant expression of gene products of genes upregulated in astrocytoma stem cells. Antibodies also may be coupled to specific diagnostic labeling agents for imaging of astrocytoma stem cells in cells and tissues or to therapeutically useful agents according to standard coupling procedures. As used herein, "therapeutically useful agents" include any therapeutic molecule which desirably is targeted selectively to astrocytoma stem cells.

Diagnostic agents for *in vivo* use include various contrast agents including, but are not limited to, barium sulfate, iocetamic acid, iopanoic acid, ipodate calcium, diatrizoate sodium, diatrizoate meglumine, metrizamide, tyropanoate sodium and radiodiagnostics including positron emitters such as fluorine-18 and carboy-11, gamma emitters such as iodine-123, technitium-99, iodine-131 and indium-111, and nuclides for nuclear magnetic resonance such as fluorine and gadolinium. Other diagnostic agents useful in the invention will be apparent to one of ordinary skill in the art.

The antibodies of the present invention can also be used to therapeutically target astrocytoma cells or astrocytoma stem cells. In a preferred embodiment, antibodies can be used to target one or more astrocytoma markers or astrocytoma stem cell markers expressed on the cell surface. These antibodies can be linked not only to a detectable marker but also an antitumor agent or an immunomodulator. Antitumor agents can include cytotoxic agents and agents that act on tumor neovasculature. Detectable markers include, for example, radioactive or fluorescent markers. Cytotoxic agents include cytotoxic radionuclides, chemical toxins and protein toxins.

The cytotoxic radionuclide or radiotherapeutic isotope preferably is an alpha-emitting isotope such as ²²⁵Ac_{,} ²¹¹At, ²¹²Bi, ²¹³Bi, ²¹²Pb, ²²⁴Ra or ²²³Ra. Alternatively, the cytotoxic radionuclide may a beta-emitting isotope such as ¹⁸⁶Rh, ¹⁸⁸Rh, ¹⁷⁷Lu, ⁹⁰Y, ¹³¹I, ⁶⁷Cu, ⁶⁴Cu, ¹⁵³Sm or ¹⁶⁶Ho. Further, the cytotoxic radionuclide may emit Auger and low energy electrons and include the isotopes ¹²⁵I, ¹²³I or ⁷⁷Br.

Suitable chemical toxins or chemotherapeutic agents include members of the enediyne family of molecules, such as calicheamicin and esperamicin. Chemical toxins can also be taken from the group consisting of methotrexate, doxorubicin, melphalan, chlorambucil, ARA-C, vindesine, mitomycin C, cis-platinum, etoposide, bleomycin and 5-fluorouracil. Other antineoplastic agents that may be conjugated to the antibodies of the present invention include dolastatins (U.S. Patent Nos. 6,034,065 and 6,239,104) and derivatives thereof. Of particular interest is dolastatin 10 (dolavaline-valine-dolaisoleuine-dolaproine-dolaphenine) and the derivatives auristatin PHE (dolavaline-valine-dolaisoleuine-dolaproine-phenylalanine-methyl ester) (Pettit, G.R. et al., Anticancer Drug Des. 13(4):243-277, 1998; Woyke, T. et al., Antimicrob. Agents Chemother. 45(12):3580-3584, 2001), and aurastatin E and the like. Toxins that are less preferred in the compositions and methods of the invention include poisonous lectins, plant toxins such as ricin, abrin, modeccin, botulina and diphtheria toxins. Of course, combinations of the various toxins could also be coupled to one antibody molecule thereby accommodating variable cytotoxicity. Other chemotherapeutic agents are known to those skilled in the art.

Agents that act on the tumor vasculature can include tubulin-binding agents such as combrestatin A4 (Griggs et al., Lancet Oncol. 2:82, 2001), angiostatin and endostatin (reviewed in Rosen, Oncologist 5:20; 2000, incorporated by reference herein) and interferon inducible protein 10 (U.S. Patent No. 5,994,292). A number of antiangiogenic agents currently in clinical trials are also contemplated. Agents currently in clinical trials include: 2ME2, Angiostatin, Angiozyme, Anti-VEGF RhuMAb, Apra (CT-2584), Avicine, Benefin, BMS275291, Carboxyamidotriazole, CC4047, CC5013, CC7085, CDC801, CGP-41251 (PKC 412), CM101, Combretastatin A-4 Prodrug, EMD 121974, Endostatin, Flavopiridol, Genistein (GCP), Green Tea Extract, IM-862, ImmTher, Interferon alpha, Interleukin-12, Iressa (ZD1839), Marimastat, Metastat (Col-3), Neovastat , Octreotide, Paclitaxel, Penicillamine, Photofrin, Photopoint, PI-88, Prinomastat (AG-3340), PTK787 (ZK22584), RO317453, Solimastat, Squalamine, SU 101, SU 5416, SU-6668, Suradista (FCE 26644), Suramin (Metaret), Tetrathiomolybdate, Thalidomide, TNP-470 and Vitaxin. Additional antiangiogenic agents are described by Kerbel, J. Clin. Oncol. 19(18s):45s-51s, 2001, which is incorporated by reference herein. Immunomodulators suitable for conjugation to the antibodies include α-interferon, γ-interferon, and tumor necrosis factor alpha (TNFα).

The coupling of one or more toxin molecules to the antibody is envisioned to include many chemical mechanisms, for instance covalent binding, affinity binding, intercalation, coordinate binding, and complexation. The toxic compounds used to prepare the immunotoxins are attached to the antibodies or antigen-binding fragments thereof by standard protocols known in the art.

As described herein, the one or more astrocytoma markers or astrocytoma stem cell markers and the antibodies and other binding molecules, as described herein, can be used for the diagnosis, determination of prognosis and treatment of disorders. When "disorder" is used herein, it refers to any pathological condition where there are astrocytoma cells or astrocytoma stem cells. An example of such a disorder is cancer specifically astrocytomas, including glioblastomas.

Conventional treatment for cancer may include, but is not limited to: surgical intervention, chemotherapy, radiotherapy, and adjuvant systemic therapies. In one aspect of the invention, treatment may include administering binding polypeptides such as antibodies that specifically bind to the one or more astrocytoma markers or astrocytoma stem cell markers. These binding polypeptides can be optionally linked to one or more detectable markers, antitumor agents or immunomodulators as described above.

Cancer treatment, in another aspect of the invention, includes administering an antisense molecules or RNAi molecules to reduce expression level and/or biological function level of one or more upregulated astrocytoma markers or upregulated astrocytoma stem cell markers in the subject in cancers where astrocytoma cells or astrocytoma stem cells have been identified. The use of RNA interference or "RNAi" involves the use of double-stranded RNA (dsRNA) to block gene expression. (see: Sui, G, et al, Proc Natl. Acad. Sci U.S.A. 99:5515-5520,2002). Methods of applying RNAi strategies in embodiments of the invention would be understood by one of ordinary skill in the art.

Methods in which small interfering RNA (siRNA) molecules are used to reduce the expression of one or more astrocytoma markers or astrocytoma stem cell markers may be used. In one aspect, a cell is contacted with a siRNA molecule to produce RNA interference (RNAi) that reduces expression of one or more astrocytoma markers or astrocytoma stem cell markers. The siRNA molecule is directed against nucleic acids coding for the one or more astrocytoma markers or astrocytoma stem cell markers (e.g. RNA transcripts including untranslated and translated regions). In a preferred aspect of the invention, the astrocytoma markers are KIAA0101, CD99, HOXC9, E2F2, ASPM, LOX, HOXA5, COL6A2, PLP2, DKFZp762E1312 and/or MELK. In another preferred aspect of the invention the astrocytoma stem cell markers are one or more gene products of a gene upregulated in astrocytoma stem cells, preferably CD99, HOXC9 or E2F2. The expression level of the targeted astrocytoma markers or astrocytoma stem cell markers can be determined using well known methods such as FACS or Western blotting for determining the level of protein expression and Northern blotting or RT-PCR for determining the level of mRNA transcript of the target gene.

As used herein, a "siRNA molecule" ("short interfering RNA") is a nucleic acid molecule capable of mediating RNA interference "RNAi" or gene silencing in a sequence-specific manner (Bass, 2001, Nature, 411, 428-429; Elbashir et al, 2001, Nature, 411, 494-498; and Kreutzer et al., International PCT Publication No. WO 00/44895; Zemicka-Goetz et al., International PCT Publication No. WO 01/36646; Fire, International PCT Publication No. WO99/32619; Plaetinck et al., International PCT Publication No. WO 00/O1846; Mello and Fire, International PCT Publication No. WO 01/29058; Deschamps-Depaillette, International PCT Publication No. WO 99/07409; and Li et al. International PCT Publication No. WO 00/44914; Allshire, 2002, Science, 297, 1818-1819; Volpe et al.,2002, Science, 297, 1833-1837; Jenuwein, 2002, Science, 297, 2215-2218; and Hall etal., 2002, Science, 297, 2232-2237; Hutvagner and Zamore, 2002, Science, 297, 2056-60; McManus et al., 2002, RNA, 8, 842-850; Reinhart et al., 2002, Gene & Dev., 16, 16-1626; and Reinhart & Bartel, 2002, Science, 297, 1831; incorporated herein by reference). As used herein,the term RNAi is meant to be equivalent to other terms used to describe sequence specific RNA interference, such as post transcriptional gene silencing, or epigenetics.

Molecules capable of mediating RNA interference include, but are not limited to, short interfering RNA (siRNA), double-stranded RNA (dsRNA), micro-RNA (miRNA), short hairpin RNA (shRNA). As used herein, molecules capable of mediating RNAi need not be limited to those molecules containing only RNA, but further encompasses chemically-modified nucleotides and non-nucleotides, referred to as "siNA" molecules "short interfering nucleic acid". Such siNA molecules that do not require the presence of ribonucleotides within the siNA molecule to support RNAi can however have an attached linker or linkers or other attached or associated groups, moieties, or chains containing one or more nucleotides with 2'-OH groups. Optionally, siNA molecules can comprise ribonucleotides at about 5, 10, 20, 30, 40, or 50% of the nucleotide positions.

In one embodiment the siRNA molecule is a double stranded RNA molecule (dsRNA) consisting of self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof. The siRNA can be assembled from two separate oligonucleotides, where one strand is the sense strand and the other is the antisense strand, wherein the antisense and sense strands are self-complementary (i.e. each strand comprises nucleotide sequence that is complementary to nucleotide sequence in the other strand); the antisense strand comprises nucleotide sequence that is complementary to nucleotide sequence in a target nucleic acid molecule or a portion thereof and the sense strand comprises nucleotide sequence corresponding to the target nucleic acid sequence or a portion thereof.

Alternatively, the siRNA is assembled from a single oligonucleotide, where the self-complementary sense and antisense regions of the siRNA are linked by means of a nucleic acid based or non-nucleic acid-based linker(s). The siRNA can be a polynucleotide with a hairpin secondary structure, having self-complementary sense and antisense regions (Tuschl, T. et al., 1999, Genes & Dev., 13:3191-3197; Elbashir, S.M. et al., 2001, EMBO J., 20:6877-6888; incorporated herein by reference), wherein the antisense region comprises nucleotide sequence that is complementary to nucleotide sequence in a separate target nucleic acid molecule or a portion thereof and the sense region having nucleotide sequence corresponding to the target nucleic acid sequence or a portion thereof. The siRNA can be a circular single-stranded polynucleotide having two or more loop structures and a stem comprising self-complementary sense and antisense regions, wherein the antisense region comprises nucleotide sequence that is complementary the nucleotide sequence in a target nucleic acid molecule or a portion thereof and the sense region having nucleotide sequence corresponding to the target nucleic acid sequence or a portion thereof, and wherein the circular polynucleotide can be processed either in vivo or in vitro to generate an active siRNA molecule capable of mediating RNAi.

In one embodiment the last nucleotide at the 3' end of the antisense strand may be any nucleotide and is not required to be complementary to the region of the target gene. The siRNA molecule may be 19-23 nucleotides in length and form a hairpin structure. In one preferred embodiment the siRNA molecule includes a two nucleotide 3' overhang on the sense strand. In a second preferred embodiment the two nucleotide overhang is thymidine-thymidine (TT). The siRNA molecule corresponds to at least a portion of a target gene. In one embodiment the siRNA molecule corresponds to a region selected from a cDNA target gene beginning between 50 to 100 nucleotides downstream of the start codon (but could be anywhere within the sequence, including the non-coding 5'UTR). In a preferred embodiment the first nucleotide of the siRNA molecule is a purine.

In one embodiment the siRNA molecule may be 27 nucleotides in length or longer, for example, 29 nucleotides in length. 27-mer dsRNA molecules (duplexes) display improved properties over 21-mer siRNAs. 27-mer dsRNAs possess about three to five times higher "long-term" RNAi activity than 21-nt siRNAs and 21-nt dsRNAs. Moreover, 27-mer dsRNAs are approximately 50-100 times more stable than 21-nt siRNA and 21-nt dsRNA in cell-cultured medium supplemented with 10% inactivated serum. These molecules can carry for example a 5' sense modification and can be designed asymmetrically. The 5' sense modifications can be conjugated with small signal molecules to facilitate intracellular delivery of the RNA duplex, e.g. with cholesterol (see, e.g. Kubo T. et al., Oligonucleotides, 17:445-64, 2007; Kim DH et al., Nat. Biotechnol. 23:222-6, 2005).

In one preferred aspect the invention provides 27-mer siRNA duplexes that specifically target markers described herein, such as KIAA0101 (SEQ ID NO. 41 and 42), ASPM (SEQ ID NO. 46), LOX (SEQ ID NO. 44), HOXA5, COL6A2, PLP2 (SEQ ID NO. 45), DKFZp762E1312 (SEQ ID NO. 43) and MELK (SEQ ID NO. 47 and 48). These 27-mer siRNA duplexes can, in one embodiment, be used to target the markers described herein, to reduce the expression of these markers in tumors. In one embodiment the tumor is a glioblastoma, in another embodiment the tumor is a melanoma or is prostate cancer, in yet another embodiment the tumor is any other tumor that overexpresses one or more of the following markers: KIAA0101, ASPM, LOX, HOXA5, COL6A2, PLP2, DKFZp762E1312, MELK, CD99, HOXC9 or E2F2. The 27-mer siRNA duplexes provided herein can be used in vivo and in vitro and can be used in a pharmaceutical composition comprising a pharmaceutically acceptable carrier. The 27-mer siRNA duplexes provided herein can be delivered to a subject by any method known in the art. In one embodiment treatment of a subject with one or more 27-mer siRNA duplexes of the invention, alone or in combination with one or more additional (chemo-)therapeutic agents, such as cytotoxic or anti-angiogenic agents, alleviates or reduces the cancer burden. In another embodiment such treatment prevents the occurrence or recurrence of the cancer or prolongs the period of absence of the tumor.

The siRNA molecules can be plasmid-based. In a preferred method, a nucleic acid sequence that encodes an astrocytoma marker or an astrocytoma stem cell marker is amplified using the well known technique of polymerase chain reaction (PCR). The use of the entire polypeptide encoding sequence is not necessary; as is well known in the art, a portion of the polypeptide encoding sequence is sufficient for RNA interference. The PCR fragment is inserted into a vector using routine techniques well known to those of skill in the art. In one preferred embodiment the nucleotide encoding sequence is the coding sequence of an astrocytoma marker or an astrocytoma stem cell marker. Combinations of the foregoing can be expressed from a single vector or from multiple vectors introduced into cells.

In one aspect of the invention a mammalian vector comprising any of the astrocytoma markers or astrocytoma stem cell markers coding sequences is provided. The mammalian vectors include but are not limited to the pSUPER RNAi vectors (Brummelkamp, T.R. et al., 2002, Science, 296:550-553, incorporated herein by reference). In one embodiment a nucleotide coding sequence can be inserted into the mammalian vector using restriction sites, creating a stem-loop structure. In a second embodiment, the mammalian vector may comprise the polymerase-II H1-RNA gene promoter. The polymerase-II H1-RNA promoter produces a RNA transcript lacking a polyadenosine tail and has a well-defined start of transcription and a termination signal consisting of five thymidines (T5) in a row. The cleavage of the transcript at the termination site occurs after the second uridine and yields a transcript resembling the ends of synthetic siRNAs containing two 3' overhanging T or U nucleotides. The antisense strand of the siRNA molecule hybridizes to the corresponding region of the mRNA of the target gene.

Preferred systems for mRNA expression in mammalian cells are those such as pSUPER RNAi system as described in Brummelkamp et al. (2002, Science, 296:550-553). Other examples include but are not limited to pSUPER.neo, pSUPER.neo+gfp, pSUPER.puro, BLOCK-iT T7-TOPO linker, pcDNA1.2/V5-GW/lacZ, pENTR/U6, pLenti6-GW/U6-laminshrna, and pLenti6/BLOCK-iT-DEST. These vectors are available from suppliers such as Invitrogen, and one of skill in the art would be able to obtain and use them.

Astrocytoma markers or astrocytoma stem cell markers can also be used in one aspect of the invention to induce or enhance an immune response. Some therapeutic approaches based upon the disclosure are premised on a response by a subject's immune system, leading to lysis of antigen presenting cells, such as astrocytoma cells or astrocytoma stem cells which present one or more astrocytoma markers or astrocytoma stem cell markers. One such approach is the administration of autologous CTLs specific to an astrocytoma marker/MHC complex or an astrocytoma stem cell marker/MHC complex to a subject with astrocytoma cells or astrocytoma stem cells. It is within the ability of one of ordinary skill in the art to develop such CTLs *in vitro*. An example of a method for T cell differentiation is presented in International Application number PCT/US96/05607. Generally, a sample of cells taken from a subject, such as blood cells, is contacted with a cell presenting the complex and capable of provoking CTLs to proliferate. The target cell can be a transfectant, such as a COS cell. Alternatively, instead of transfecting COS cells, one might use autologous APCs such as dendritic cells (DCs) purified from peripheral blood mononuclear cells (PBMC). DCs could be transfected or pulsed with antigen, either full length protein or peptide antigens. (Ayyoub, M et al J. Immunol 2004 172:7206-7211, Ayyoub M. et al. J Clin Invest 2004 113:1225-33.) These transfectants present the desired complex of their surface and, when combined with a CTL of interest, stimulate its proliferation. COS cells are widely available, as are other suitable host cells. Specific production of CTL clones is well known in the art. The clonally expanded autologous CTLs then are administered to the subject.

Another method for selecting antigen-specific CTL clones has been described (Altman et al., Science 274:94-96, 1996; Dunbar et al., Curr. Biol. 8:413-416, 1998), in which fluorogenic tetramers or multimers of MHC class I molecule/peptide complexes are used to detect specific CTL clones. Briefly, soluble MHC class I molecules are folded in vitro in the presence of β₂-microglobulin and a peptide antigen which binds the class I molecule. After purification, the MHC/peptide complex is purified and labeled with biotin. Tetramers are formed by mixing the biotinylated peptide-MHC complex with labeled avidin (e.g., phycoerythrin) at a molar ratio or 4:1. Tetramers are then contacted with a source of CTLs such as peripheral blood or lymph node. The tetramers bind CTLs which recognize the peptide antigen/MHC class I complex. Cells bound by the tetramers can be sorted by fluorescence activated cell sorting to isolate the reactive CTLs. The isolated CTLs then can be expanded in vitro for use as described herein.

The use of MHC class II molecules as tetramers was demonstrated by Crawford et al. (Immunity 8:675-682, 1998; see also Dunbar and Ogg, J. Immunol. Methods 268(1):3-7, 2002; Arnold et al., J. Immunol. Methods 271(1-2):137-151, 2002). Multimeric soluble MHC class II molecules were complexed with a covalently attached peptide (which can be attached with or without a linker molecule), but peptides also can be loaded onto class II molecules. The class II tetramers were shown to bind with appropriate specificity and affinity to specific T cells. Thus tetramers can be used to monitor both CD4⁺ and CD8⁺ cell responses to vaccination protocols. Methods for preparation of multimeric complexes of MHC class II molecules are described in Hugues et al., J. Immunological Meth. 268: 83- 92 (2002) and references cited therein, each of which is incorporated by reference for such teachings.

In one therapeutic methodology, referred to as adoptive transfer (Greenberg, J. Immunol. 136(5): 1917, 1986; Riddel et al., Science 257: 238, 1992; Lynch et al, Eur. J. Immunol. 21: 1403-1410,1991; Kast et al., Cell 59: 603-614, 1989), cells presenting the desired complex (e.g., dendritic cells) are combined with CTLs leading to proliferation of the CTLs specific thereto. The proliferated CTLs are then administered to a subject with a cellular abnormality which is characterized by certain of the abnormal cells presenting the particular complex, such as astrocytoma stem cells presenting peptides obtained from a gene product of a gene upregulated in astrocytoma stem cells, e.g., CD99 peptides. The CTLs then lyse the abnormal cells, thereby achieving the desired therapeutic goal.

The foregoing therapy assumes that at least some of the subject's abnormal cells present the relevant HLA/antigen complex. This can be determined very easily, as the art is very familiar with methods for identifying cells which present a particular HLA molecule, as well as how to identify cells expressing DNA of the pertinent sequences, in this case an astrocytoma marker or an astrocytoma stem cell marker sequence. Once cells presenting the relevant complex are identified via the foregoing screening methodology, they can be combined with a sample from a patient, where the sample contains CTLs. If the complex presenting cells are lysed by the mixed CTL sample, then it can be assumed that an astrocytoma marker or an astrocytoma stem cell marker is being presented, and the subject is an appropriate candidate for the therapeutic approaches set forth herein.

Adoptive transfer is not the only form of therapy that is available in accordance with the invention. CTLs can also be provoked *in vivo*, using a number of approaches. One approach is the use of non-proliferative cells expressing the complex. The cells used in this approach may be those that normally express the complex, such as irradiated astrocytoma cells or astrocytoma stem cells isolated as described herein or cells transfected with one or both of the genes necessary for presentation of the complex (i.e. the antigenic peptide of the astrocytoma marker or astrocytoma stem cell marker and the presenting MHC molecule). Chen et al. (Proc. Natl. Acad. Sci. USA 88: 110-114,1991) exemplifies this approach, showing the use of transfected cells expressing HPV E7 peptides in a therapeutic regime. Various cell types may be used. Similarly, vectors carrying one or both of the genes of interest may be used. Viral or bacterial vectors are especially preferred. For example, nucleic acids which encode a gene product of a gene upregulated or downregulated in astrocytoma stem cells may be operably linked to promoter and enhancer sequences which direct expression of the astrocytoma stem cell marker polypeptide in certain tissues or cell types. The nucleic acid may be incorporated into an expression vector.

Expression vectors may be unmodified extrachromosomal nucleic acids, plasmids or viral genomes constructed or modified to enable insertion of exogenous nucleic acids, such as those encoding one or more astrocytoma markers or astrocytoma stem cell markers, as described elsewhere herein. Nucleic acids encoding one or more astrocytoma markers or astrocytoma stem cell markers also may be inserted into a retroviral genome, thereby facilitating integration of the nucleic acid into the genome of the target tissue or cell type. In these systems, the gene of interest is carried by a microorganism, e.g., a Vaccinia virus, pox virus, herpes simplex virus, retrovirus or adenovirus, and the materials de facto "infect" host cells. The cells which result present the complex of interest, and are recognized by autologous CTLs, which then proliferate.

A similar effect can be achieved by combining the astrocytoma marker polypeptide, the astrocytoma stem cell marker polypeptide or a stimulatory fragment thereof with an adjuvant to facilitate incorporation into antigen presenting cells *in vivo.* The astrocytoma marker polypeptide or astrocytoma stem cell marker polypeptide is processed to yield the peptide partner of the MHC molecule while an astrocytoma stem cell marker fragment may be presented without the need for further processing. Generally, subjects can receive an intradermal, intravenous, subcutaneous or intramuscular injection of an effective amount of the one or more astrocytoma markers or astrocytoma stem cell markers, e.g., a gene product of a gene upregulated in astrocytoma cells or astrocytoma stem cells. Initial doses can be followed by bi- or tri-weekly, weekly or monthly booster doses, following immunization protocols standard in the art. Preferred astrocytoma markers or astrocytoma stem cell markers include those where evidence of naturally or spontaneously induced immunity can be observed. This might be the demonstration of antigen-specific CD8 or CD4 T cells in a high frequency of cancer patients with antigen expressing tumors or the presence of autologous antigen-specific antibodies in such cancer patients, preferably both (Jager et al. PNAS 2000 97:4700-5; Gnjatic et al. PNAS 2003 100:8862-7).

The invention involves the use of various materials disclosed herein to "immunize" subjects or as "vaccines". As used herein, "immunization" or "vaccination" means increasing or activating an immune response against an antigen. It does not require elimination or eradication of a condition but rather contemplates the clinically favorable enhancement of an immune response toward an antigen. Generally accepted animal models can be used for testing of immunization against cancer using one or more astrocytoma markers or astrocytoma stem cell markers. For example, human astrocytoma cells or astrocytoma stem cells can be introduced into a mouse to create a tumor, and one or more astrocytoma markers or astrocytoma stem cell markers (or nucleic acids encoding these) can be delivered by the methods described herein. The effect on the cancer cells (e.g., reduction of tumor size) can be assessed as a measure of the effectiveness of the astrocytoma marker or astrocytoma stem cell marker nucleic acid immunization. Of course, testing of the foregoing animal model using more conventional methods for immunization can include the administration of one or more astrocytoma marker or astrocytoma stem cell marker polypeptides or fragments derived therefrom, optionally combined with one or more adjuvants and/or cytokines to boost the immune response.

Methods for immunization, including formulation of a vaccine composition and selection of doses, route of administration and the schedule of administration (e.g. primary and one or more booster doses), are well known in the art. The tests also can be performed in humans, where the end point is to test for the presence of enhanced levels of circulating CTLs against astrocytoma cells or astrocytoma stem cells, to test for levels of circulating antibodies against the one or more astrocytoma markers or astrocytoma stem cell markers, to test for the presence of cells expressing the antigen and so forth.

As part of the immunization compositions, one or more astrocytoma markers or astrocytoma stem cell markers or immunogenic fragments thereof are administered with one or more adjuvants to induce an immune response or to increase an immune response. An adjuvant is a substance incorporated into or administered with antigen which potentiates the immune response. Adjuvants may enhance the immunological response by providing a reservoir of antigen (extracellularly or within macrophages), activating macrophages and stimulating specific sets of lymphocytes. Adjuvants of many kinds are well known in the art. Specific examples of adjuvants include monophosphoryl lipid A (MPL, SmithKline Beecham), a congener obtained after purification and acid hydrolysis of Salmonella minnesota Re 595 lipopolysaccharide; saponins including QS21 (SmithKline Beecham), a pure QA-21 saponin purified from Quillja saponaria extract; DQS21, described in PCT application WO96/33739 (SmithKline Beecham), ISCOMATRIX® (CSL Ltd., Parkville, Victoria, Australia) derived from the bark of the Quillaia saponaria molina tree; QS-7, QS-17, QS-18, and QS-L1 (So et al., Mol. Cells 7:178-186, 1997); incomplete Freund's adjuvant; complete Freund's adjuvant; montanide; alum; CpG oligonucleotides (see e.g. Kreig et al., Nature 374:546-9, 1995; US patent 6,207,646) and other immunostimulatory oligonucleotides; various water-in-oil emulsions prepared from biodegradable oils such as squalene and/or tocopherol; and factors that are taken up by the so-called 'toll-like receptor 7' on certain immune cells that are found in the outside part of the skin, such as imiquimod (3M, St. Paul, Minnesota). Preferably, the antigens are administered mixed with a combination of DQS21/MPL or ISCOMATRIX®. The ratio of DQS21 to MPL typically will be about 1:10 to 10:1, preferably about 1:5 to 5:1 and more preferably about 1:1. Typically for human administration, DQS21 and MPL will be present in a vaccine formulation in the range of about 1 µg to about 100 µg. Other adjuvants are known in the art and can be used in the invention (see, e.g. Goding, Monoclonal Antibodies: Principles and Practice, 2nd Ed., 1986). Methods for the preparation of mixtures or emulsions of polypeptide and adjuvant are well known to those of skill in the art of vaccination.

Other agents which stimulate the immune response of the subject can also be administered to the subject. For example, other cytokines are also useful in vaccination protocols as a result of their lymphocyte regulatory properties. Many other cytokines useful for such purposes will be known to one of ordinary skill in the art, including interleukin-12 (IL-12) which has been shown to enhance the protective effects of vaccines (see, e.g., Science 268: 1432-1434, 1995), GM-CSF, IL-18 and IL-15 (Klebanoff et al. Proc. Natl. Acad. Sci. USA 2004 101:1969-74). Thus cytokines can be administered in conjunction with antigens and adjuvants to increase the immune response to the antigens.

There are a number of immune response potentiating compounds that can be used in vaccination protocols. These include costimulatory molecules provided in either protein or nucleic acid form. Such costimulatory molecules include the B7-1 and B7-2 (CD80 and CD86 respectively) molecules which are expressed on dendritic cells (DC) and interact with the CD28 molecule expressed on the T cell. This interaction provides costimulation (signal 2) to an antigen/MHC/TCR stimulated (signal 1) T cell, increasing T cell proliferation and effector function. B7 also interacts with CTLA4 (CD152) on T cells and studies involving CTLA4 and B7 ligands indicate that the B7-CTLA4 interaction can enhance antitumor immunity and CTL proliferation (Zheng P., et al. Proc. Natl. Acad. Sci. USA 95 (11):6284-6289 (1998)).

B7 typically is not expressed on tumor cells so they are not efficient antigen presenting cells (APCs) for T cells. Induction of B7 expression would enable the tumor cells to stimulate more efficiently CTL proliferation and effector function. A combination of B7/IL-6/IL-12 costimulation has been shown to induce IFN-gamma and a Th1 cytokine profile in the T cell population leading to further enhanced T cell activity (Gajewski et al., J. Immunol, 154:5637-5648 (1995)). Tumor cell transfection with B7 has been discussed in relation to in vitro CTL expansion for adoptive transfer immunotherapy by Wang et al., (J. Immunol., 19:1-8 (1986)). Other delivery mechanisms for the B7 molecule include nucleic acid (naked DNA) immunization (Kim J., et al. Nat. Biotechnol., 15:7:641-646 (1997)) and recombinant viruses such as adeno and pox (Wendtner et al., Gene Ther., 4:7:726-735 (1997)). These systems are all amenable to the construction and use of expression cassettes for the coexpression of B7 with other molecules of choice such as the antigens or fragment(s) of antigens discussed herein (including polytopes) or cytokines. These delivery systems can be used for induction of the appropriate molecules *in vitro* and for *in vivo* vaccination situations. The use of anti-CD28 antibodies to directly stimulate T cells *in vitro* and *in vivo* could also be considered. Similarly, the inducible co-stimulatory molecule ICOS which induces T cell responses to foreign antigen could be modulated, for example, by use of anti-ICOS antibodies (Hutloff et al., Nature 397:263-266, 1999).

Lymphocyte function associated antigen-3 (LFA-3) is expressed on APCs and some tumor cells and interacts with CD2 expressed on T cells. This interaction induces T cell IL-2 and IFN-gamma production and can thus complement but not substitute, the B7/CD28 costimulatory interaction (Parra et al., J. Immunol., 158:637-642 (1997), Fenton et al., J. Immunother., 21:2:95-108 (1998)).

Lymphocyte- function associated antigen-1 (LFA-1) is expressed on leukocytes and interacts with ICAM-1 expressed on APCs and some tumor cells. This interaction induces T cell IL-2 and IFN-gamma production and can thus complement but not substitute, the B7/CD28 costimulatory interaction (Fenton et al., J. Immunother., 21:2:95-108 (1998)). LFA-1 is thus a further example of a costimulatory molecule that could be provided in a vaccination protocol in the various ways discussed above for B7.

Complete CTL activation and effector function requires Th cell help through the interaction between the Th cell CD40L (CD40 ligand) molecule and the CD40 molecule expressed by DCs (Ridge et al., Nature, 393:474 (1998), Bennett et al., Nature, 393:478 (1998), Schoenberger et al., Nature, 393:480 (1998)). This mechanism of this costimulatory signal is likely to involve upregulation of B7 and associated IL-6/IL-12 production by the DC (APC). The CD40-CD40L interaction thus complements the signal 1 (antigen/MHC-TCR) and signal 2 (B7-CD28) interactions.

The use of anti-CD40 antibodies to stimulate DC cells directly, would be expected to enhance a response to tumor antigens which are normally encountered outside of an inflammatory context or are presented by non-professional APCs (tumor cells). In these situations Th help and B7 costimulation signals are not provided.

The invention contemplates delivery of nucleic acids, polypeptides or fragments thereof for vaccination. Delivery of polypeptides and fragments thereof can be accomplished according to standard vaccination protocols which are well known in the art. In another embodiment, the delivery of nucleic acid is accomplished by *ex vivo* methods, i.e. by removing a cell from a subject, genetically engineering the cell to include one or more astrocytoma markers or astrocytoma stem cell markers, and reintroducing the engineered cell into the subject. One example of such a procedure is outlined in U.S. Patent 5,399,346 and in exhibits submitted in the file history of that patent, all of which are publicly available documents. In general, it involves introduction *in vitro* of a functional copy of a gene into a cell(s) of a subject, and returning the genetically engineered cell(s) to the subject. The functional copy of the gene is under operable control of regulatory elements which permit expression of the gene in the genetically engineered cell(s). Numerous transfection and transduction techniques as well as appropriate expression vectors are well known to those of ordinary skill in the art, some of which are described in PCT application WO95/00654. *In vivo* nucleic acid delivery using vectors such as viruses and targeted liposomes also is contemplated according to the invention.

A virus vector for delivering a nucleic acid encoding one or more astrocytoma markers or astrocytoma stem cell markers is selected from the group consisting of adenoviruses, adeno-associated viruses, poxviruses including vaccinia viruses and attenuated poxviruses, Semliki Forest virus, Venezuelan equine encephalitis virus, retroviruses, Sindbis virus, and Ty virus-like particle. Examples of viruses and virus-like particles which have been used to deliver exogenous nucleic acids include: replication-defective adenoviruses (e.g., Xiang et al., Virology 219:220-227, 1996; Eloit et al., J. Virol. 7:5375-5381, 1997; Chengalvala et al., Vaccine 15:335-339, 1997), a modified retrovirus (Townsend et al., J. Virol. 71:3365-3374, 1997), a nonreplicating retrovirus (Irwin et al., J. Virol. 68:5036-5044, 1994), a replication defective Semliki Forest virus (Zhao et al., Proc. Natl. Acad. Sci. USA 92:3009-3013, 1995), canarypox virus and highly attenuated vaccinia virus derivative (Paoletti, Proc. Natl. Acad. Sci. USA 93:11349-11353, 1996), non-replicative vaccinia virus (Moss, Proc. Natl. Acad. Sci. USA 93:11341-11348, 1996), replicative vaccinia virus (Moss, Dev. Biol. Stand. 82:55-63, 1994), Venzuelan equine encephalitis virus (Davis et al., J. Virol. 70:3781-3787, 1996), Sindbis virus (Pugachev et al., Virology 212:587-594, 1995), and Ty virus-like particle (Allsopp et al., Eur. J. Immunol 26:1951-1959, 1996). A preferred virus vector is an adenovirus.

Preferably the foregoing nucleic acid delivery vectors: (1) contain exogenous genetic material that can be transcribed and translated in a mammalian cell and that can induce an immune response in a host, and (2) contain on a surface a ligand that selectively binds to a receptor on the surface of a target cell, such as a mammalian cell, and thereby gains entry to the target cell.

Various techniques may be employed for introducing nucleic acids into cells, depending on whether the nucleic acids are introduced in vitro or in vivo in a host. Such techniques include transfection of nucleic acid-CaPO₄ precipitates, transfection of nucleic acids associated with DEAE, transfection or infection with the foregoing viruses including the nucleic acid of interest, liposome mediated transfection, and the like. For certain uses, it is preferred to target the nucleic acid to particular cells. In such instances, a vehicle used for delivering a nucleic acid into a cell (e.g., a retrovirus, or other virus; a liposome) can have a targeting molecule attached thereto. For example, a molecule such as an antibody specific for a surface membrane protein on the target cell or a ligand for a receptor on the target cell can be bound to or incorporated within the nucleic acid delivery vehicle. Preferred antibodies include antibodies which selectively bind a gene product of a gene upregulated in astrocytoma cells or astrocytoma stem cells, alone or as a complex with a MHC molecule. Especially preferred are monoclonal antibodies. Where liposomes are employed to deliver the nucleic acids, proteins which bind to a surface membrane protein associated with endocytosis may be incorporated into the liposome formulation for targeting and/or to facilitate uptake. Such proteins include capsid proteins or fragments thereof tropic for a particular cell type, antibodies for proteins which undergo internalization in cycling, proteins that target intracellular localization and enhance intracellular half life, and the like. Polymeric delivery systems also have been used successfully to deliver nucleic acids into cells, as is known by those skilled in the art. Such systems even permit oral delivery of nucleic acids.

According to a further aspect of the invention, compositions containing the nucleic acid molecules, proteins, and binding polypeptides are provided. The compositions contain any of the foregoing nucleic acid molecules, proteins, and binding polypeptides (as therapeutic agents) in an optional pharmaceutically acceptable carrier. Thus, in a related aspect, the invention provides a method for forming a medicament that involves placing a therapeutically effective amount of the therapeutic agent in the pharmaceutically acceptable carrier to form one or more doses. The effectiveness of treatment or prevention methods of the invention can be determined using standard diagnostic methods described herein.

When administered, the therapeutic compositions of the present invention are administered in pharmaceutically acceptable preparations. Such preparations may routinely contain pharmaceutically acceptable concentrations of salt, buffering agents, preservatives, compatible carriers, supplementary immune potentiating agents such as adjuvants and cytokines, and optionally other therapeutic agents.

As used herein, the term "pharmaceutically acceptable" means a non-toxic material that does not interfere with the effectiveness of the biological activity of the active ingredients. The term "physiologically acceptable" refers to a non-toxic material that is compatible with a biological system such as a cell, cell culture, tissue, or organism. The characteristics of the carrier will depend on the route of administration. Physiologically and pharmaceutically acceptable carriers include diluents, fillers, salts, buffers, stabilizers, solubilizers, and other materials which are well known in the art. The term "carrier" denotes an organic or inorganic ingredient, natural or synthetic, with which the active ingredient is combined to facilitate the application. The components of the pharmaceutical compositions also are capable of being co-mingled with the molecules of the present invention, and with each other, in a manner such that there is no interaction which would substantially impair the desired pharmaceutical efficacy.

The therapeutics of the invention can be administered by any conventional route, including injection or by gradual infusion over time. The administration may, for example, be oral, intravenous, intratumoral, intraperitoneal, intramuscular, intracavity, subcutaneous, or transdermal. When antibodies are used therapeutically, a preferred route of administration is by pulmonary aerosol. Techniques for preparing aerosol delivery systems containing antibodies are well known to those of skill in the art. Generally, such systems should utilize components which will not significantly impair the biological properties of the antibodies, such as the paratope binding capacity (see, for example, Sciarra and Cutie, "Aerosols," in Remington's Pharmaceutical Sciences, 18th edition, 1990, pp 1694-1712). Those of skill in the art can readily determine the various parameters and conditions for producing antibody aerosols without undue experimentation. When using antisense preparations of the invention, slow intravenous administration is preferred.

The compositions of the invention are administered in effective amounts. An "effective amount" is that amount of a composition that alone, or together with further doses, produces the desired response, e.g. increases an immune response to the one or more astrocytoma markers or astrocytoma stem cell markers, or treats a subject's disease. In the case of treating a particular disease or condition characterized by the presence of astrocytoma cells or astrocytoma stem cells, such as cancer, the desired response is inhibiting the progression of the disease. This may involve only slowing the progression of the disease temporarily, although more preferably, it involves halting the progression of the disease permanently. This can be monitored by routine methods or can be monitored according to diagnostic methods of the invention discussed herein. The desired response to treatment of the disease or condition also can be delaying the onset or even preventing the onset of the disease or condition.

Such amounts will depend, of course, on the particular condition being treated, the severity of the condition, the individual patient parameters including age, physical condition, size and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a patient may insist upon a lower dose or tolerable dose for medical reasons, psychological reasons or for virtually any other reasons.

The pharmaceutical compositions used in the foregoing methods preferably are sterile and contain an effective amount of one or more astrocytoma marker polypeptides or astrocytoma stem cell marker polypeptides or nucleic acids encoding one or more astrocytoma markers or astrocytoma stem cell markers for producing the desired response in a unit of weight or volume suitable for administration to a patient. The response can, for example, be measured by determining the immune response following administration of the astrocytoma marker or astrocytoma stem cell marker composition via a reporter system by measuring downstream effects such as gene expression, or by measuring the physiological effects of the astrocytoma marker or astrocytoma stem cell marker polypeptide composition, such as regression of a tumor, decrease of astrocytoma cells or astrocytoma stem cells or decrease of disease symptoms. Other assays will be known to one of ordinary skill in the art and can be employed for measuring the level of the response.

The doses of one or more astrocytoma marker or astrocytoma stem cell marker compositions (e.g., polypeptide, peptide, antibody, cell or nucleic acid) administered to a subject can be chosen in accordance with different parameters, in particular in accordance with the mode of administration used and the state of the subject. Other factors include the desired period of treatment. In the event that a response in a subject is insufficient at the initial doses applied, higher doses (or effectively higher doses by a different, more localized delivery route) may be employed to the extent that patient tolerance permits.

In general, for treatments for eliciting or increasing an immune response, doses of one or more astrocytoma marker polypeptides or astrocytoma stem cell marker polypeptides are formulated and administered in doses between 1 ng and 1 mg, and preferably between 10 ng and 100 µg, according to any standard procedure in the art. Where nucleic acids encoding one or more astrocytoma markers or astrocytoma stem cell markers or variants thereof are employed, doses of between 1 ng and 0.1 mg generally will be formulated and administered according to standard procedures. Other protocols for the administration of one or more astrocytoma marker or astrocytoma stein cell marker compositions will be known to one of ordinary skill in the art, in which the dose amount, schedule of injections, sites of injections, mode of administration (e.g., intra-tumoral) and the like vary from the foregoing. Administration of astrocytoma marker or astrocytoma stem cell marker polypeptide compositions to mammals other than humans, e.g. for testing purposes or veterinary therapeutic purposes, is carried out under substantially the same conditions as described above.

Where one or more astrocytoma marker or astrocytoma stem cell marker polypeptides are used for vaccination, modes of administration which effectively deliver the astrocytoma marker or astrocytoma stem cell marker polypeptide and adjuvant, such that an immune response to the polypeptide is increased, can be used. For administration of an astrocytoma marker or astrocytoma stem cell marker polypeptide in adjuvant, preferred methods include intradermal, intravenous, intramuscular and subcutaneous administration. Although these are preferred embodiments, the invention is not limited by the particular modes of administration disclosed herein. Standard references in the art (e.g., Remington's Pharmaceutical Sciences, 18th edition, 1990) provide modes of administration and formulations for delivery of immunogens with adjuvant or in a non-adjuvant carrier.

The pharmaceutical compositions may contain suitable buffering agents, including: acetic acid in a salt; citric acid in a salt; boric acid in a salt; and phosphoric acid in a salt.

The pharmaceutical compositions also may contain, optionally, suitable preservatives, such as: benzalkonium chloride; chlorobutanol; parabens and thimerosal.

The pharmaceutical compositions may conveniently be presented in unit dosage form and may be prepared by any of the methods well-known in the art of pharmacy. All methods include the step of bringing the active agent into association with a carrier which constitutes one or more accessory ingredients. In general, the compositions are prepared by uniformly and intimately bringing the active compound into association with a liquid carrier, a finely divided solid carrier, or both, and then, if necessary, shaping the product.

Compositions suitable for oral administration may be presented as discrete units, such as capsules, tablets, lozenges, each containing a predetermined amount of the active compound. Other compositions include suspensions in aqueous liquids or non-aqueous liquids such as a syrup, elixir or an emulsion.

Compositions for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, and lactated Ringer's or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases, and the like.

The pharmaceutical agents of the invention may be administered alone, in combination with each other, and/or in combination with other anti-cancer drug therapies and/or treatments. These therapies and/or treatments may include, but are not limited to: surgical intervention, chemotherapy, radiotherapy, and adjuvant systemic therapies.

The invention also provides a pharmaceutical kit comprising one or more containers comprising one or more of the pharmaceutical compounds or agents of the invention. Additional materials may be included in any or all kits of the invention, and such materials may include, but are not limited to buffers, water, enzymes, tubes, control molecules, etc. The kit may also include instructions for the use of the one or more pharmaceutical compounds or agents of the invention for the treatment of cancer.

### Examples

### Example 1: Identification of astrocytoma markers

### Materials and methods

### Tissue samples

Tumor specimens were obtained from patients with CNS tumors treated by the Neurosurgery Group of the Department of Neurology at Hospital das Clinicas, School of Medicine, University of Sâo Paulo, São Paulo, Brazil, between 2000 and 2005. The tissues were categorized according to the WHO grading system^{1,2} by neuropathologists from the Division of Pathological Anatomy of the same institution. Informed consent was obtained from each patient. Fresh surgical samples of different grades and non-neoplastic tissues of the CNS (temporal lobectomy from epilepsy surgeries) were macrodissected and immediately snap-frozen in liquid nitrogen upon surgical removal. All tumor tissues were microdissected prior to RNA extraction as described previously¹¹.

### Tissue samples for IHC

Tumor specimens were obtained during therapeutic surgical approach of patients with CNS tumors by the Neurosurgery Group of the Department of Neurology at Hospital das Clínicas, School of Medicine, University of São Paulo, São Paulo, Brazil, between 2000 and 2007. The tissues were categorized according to the WHO grading system[i] by neuropathologists from the Division of Pathological Anatomy of the same institution. Informed consent was obtained from each patient. Fresh surgical samples of different grades and non-neoplastic tissues of the CNS (temporal lobectomy from epilepsy surgeries) were macrodissected and immediately snap-frozen in liquid nitrogen upon surgical removal. Before RNA extraction, a 4 µm thick cryosection of each sample was obtained at -25°C for histological assessment under light microscope after hematoxylin-eosin staining. Necrotic, cellular debris and non-neoplastic areas were removed from the frozen block of tumoral tissue by microdissection prior to the RNA extraction procedure.

### Cell lines, cell suspensions, culture and reagents

Human malignant astrocytomas cell lines T98G, U251MG, A172 and U87MG, obtained from the American Type Culture Collection (Manassas, VA) were cultivated in Dulbecco's Modified Eagle's Medium (DMEM), supplemented with 10% fetal calf serum (FCS), in a humidified incubator at 37°C and controlled CO₂ atmosphere.

### Analysis of gene expression using microarrays

Nine primary astrocytomas (three samples of GI/PA, mean age of 19 years old; and six samples of GBM, mean age of 56 years old, and mean survival time of 7.6 months), and a pool of three non-neoplastic brain tissues (mean age of 47 years old) were used for microarray analyses.

Analyses were carried out with oligonucleotide microarrays representing 10,000 human genes (CodeLink Bioarrays-Human Uniset I, GE Healthcare, Piscataway, NJ), following the manufacturer's protocol. The fluorescence was measured using an Axon GenePix 4000B Scanner (Axon Instruments Inc, Sunnyvale, CA), and normalized with the CodeLink Software v.2.3 (GE Healthcare, Piscataway, NJ). Independent hybridizations for each tumor sample were carried out in duplicate.

Individual gene GI (PA) and GBM expression profiles were compared with each other, and with non-neoplastic brain tissue. Differentially expressed genes were identified by calculating the ratio of the mean normalized fluorescence values obtained from each duplicate GBM or GI (PA) sample and non-neoplastic brain tissue, and subsequently between duplicate GBM and GI (PA) samples. Results were expressed as fold variation, and genes displaying greater than 2-fold changes in transcript abundance and p<0.01 were selected.

Differentially expressed genes in astrocytomas were classified according to an ontology term enrichment analysis¹², as defined by the Gene Ontology Consortium, to identify functional classes. In such analysis, the statistical association between being differentially expressed and belonging to a given category was accessed by the Fisher Exact test.

### Total RNA extraction and cDNA synthesis

Total RNA was extracted from tissues or cell lines using Trizol (Invitrogen Inc, Carlsbad, CA) or RNeasy Mini Kit (Qiagen Inc, Hilden, Germany). Synthesis of cDNA was performed by reverse transcription, using oligo(dT) and random hexamers, and SuperScript III (Invitrogen Inc, Carlsbad, CA), according to the manufacturer's recommendations.

### DNA extraction

Frozen tissue DNA was extracted with a standard phenol/chloroform methodology or with Trizol (Invitrogen Inc, Carlsbad, CA), following the manufacturer's instructions.

### Quantitative real time RT-PCR (QT-PCR) from RNA extracted from tissue

The relative expression levels of the genes were analyzed by QT-PCR to validate microarray data. To this end, we used seven non-neoplastic brain tissues, and 18 astrocytic tumor samples (13 GBM and 5 GI (PA)). For a more detailed analysis of relative *MELK* expression we used an independent series of 10 non-neoplastic brain tissues, and 93 astrocytic tumor samples: 12 GI (PA) (median age 22 years), 13 low grade astrocytomas (median age 37 years), 15 anaplastic astrocytomas (median age 31 years), and 53 GBM (median age 52 years). We also examined 26 medulloblastomas (median age 18 years). The median age of epilepsy patients for non-neoplastic brain tissues was 37 years. All QT-PCR assays were carried out in duplicate using the Syber-Green approach. Normalization of quantitative data was based on the housekeeping gene hypoxanthine guanine phosphoribosyltransferase gene (*HPRT*): Primer sequences were as follows (5'-3'):
*AURKB* F: CTGCCATGGGAAGAAG GTGATTCA (SEQ ID NO:1),
*AURKB* R: GATGCGGCGATAGGTCTCGTTG (SEQ ID NO:2),
*ASPMF*: TGTGGAGTGCGCCTTGTG (SEQ ID NO:3),
*ASPM* R: TGCCGGAATCCTGAGTTTCT (SEQ ID NO:4),
*PRC1* F: CCTGCCCTACTGCCAAAGA (SEQ ID NO:5),
*PRC1* R: CAGAACTGAACACAATTTAACAAAGCT (SEQ ID NO:6),
*KIAA0101* F: GTTTGAACATGGTG CGGACTAA (SEQ ID NO:7),
*KIAA0101* R: GGCAGAGGTGGAAGAACCAA (SEQ ID NO:8),
*MELK* F: AAACCCAAGGGTAACAAGGA (SEQ ID NO:9),
*MELK* R: ACAGTATGCCCATGCTCCAA (SEQ ID NO:10),
*PLP2* F: GACCTGCACACCAAGATACCAT (SEQ ID NO:11),
*PLP2* R: CAAGGACAACAATGGAGGTGAT (SEQ ID NO:12),
*HPRT* F: TGAGGATTTGGAAAGGGTGT (SEQ ID NO:13), and
*HPRT* R: GAGCACACAGAGGGCTACAA (SEQ ID NO:14).

Sybr Green I amplification mixtures (12 µL) contained 3 µL of cDNA, 6 µL of 2X Sybr Green I Master Mix (Applied Biosystems, Foster City CA), and forward and reverse primers at a final concentration 200 nM to 400nM. Reactions were run on an ABI 7500 Real-Time PCR System (Applied Biosystems, Foster City CA). The equation 2^{-ΔΔCT} was applied to calculate the relative expression of tumor samples *versus* the median of non-neoplastic tissues. All primers were synthesized by IDT (Integrated DNA Technologies, Inc, Coralville, IA).

### Quantitative real time PCR (QT-PCR) from RNA extracted from cell lines

Forty-eight hours post-transfection, total RNA was extracted, using the RNeasy Mini Kit (Qiagen, Valencia, CA). A total of 0.5-1.0 µg of RNA was reverse transcribed into cDNA by using an Omniscript RT kit according to the manufacturer's protocol using oligo (dT)18 primers. Quantitative data were normalized relative to the geometric mean of the three housekeeping genes. The minimum concentration of primers was determined by the lowest threshold cycle (Ct) and maximum amplification efficiency while minimizing non-specific amplification. Standard curves were established following serial sample dilutions to ensure amplification efficiency within each cycle (data not shown). Analysis of melting curves demonstrated a single peak for the primers. Sybr Green I amplification mixtures (12 µl) contained 3 µl of cDNA, 6 µl of 2x Sybr Green I Master Mix (Applied Biosystems), and forward and reverse primers to a final concentration 200 nM. Reactions were run on ABI 7500 Real-Time PCR Systems (Applied Biosystems). The equation 2-ΔΔCT was applied to calculate the relative expression of tumor samples *versus t*he median of non-neoplastic tissues where ΔCt=Ct target gene - Ct normalizer, and ΔΔCt = ΔCt tumor-mean ΔCt non-neoplastic tissues. The QT-PCR runs for each sample were performed in duplicate and repeated when the Ct values were not similar. The results are presented in log10 scale for better visualization. The expression of each normal tissue sample was calculated relative to the tissue with the lowest gene expression level for each analyzed gene [ii]. All primers were synthesized by IDT (Integrated DNA technologies, Inc, Coralville, IA).

### Immunohistochemistry

For the immunohistochemical analysis, 5 µm paraffin sections were applied to Super Plus slides (Menzel, Braunschweig, Germany), and heated at 60oC for 2 hours to ensure attachment. Sections were deparaffinized, rehydrated in xylene and a series of graded alcohols. Polyclonal antibodies anti-PLP2 (Aviva ARP45348-T100, San Diego, CA, USA), dilution 1:800), and anti-MELK kindly provided by Dr. JP Tassan (Rennes, France)[iii], dilution 1:1000 were used. Heat based antigen retrieval was used for both primary antibodies prior to staining employing citrate (10 mM, pH6.0) buffer. Primary antibodies were incubated overnight at 5oC, and detected with the Visionbiosystems kit (Novocastra, Newcastle Upon Tyne, UK). 3'3'-diaminobenzidine (liquid DAB, Biogenex, San Ramon, CA) was used as a chromogen, and Harris' hematoxylin to counterstain. The extent of immunohistochemical staining was evaluated microscopically according to the amount of immunopositive tumor cells.

### Transfection Experiments

Transfections were performed using LipofectamineTM 2000 (Invitrogen) following the manufacturer's recommended protocols. Briefly, cells were seeded in 60 mm dishes dishes in 4 ml of regular growth media without any antibiotics so the cells would be 50-60% confluent at the time of transfection. For transfection, 40 pmoles of siRNA were diluted in 500 µl Opti-MEM (Invitrogen). Eight µl of LipofectamineTM 2000 were diluted in 500 µl Opti-MEMTM and incubated for 5 min at room temperature before mixing with the diluted siRNA. The siRNA-Lipofectamine 2000 mixture was incubated for 20 min at room temperature and then added to the cells.

### Migration assay

Cells were trypsinized, counted and left 2 hours of calf fetal medium starvation conditions. After 2 hrs, the cells were loaded at the density of 5x104 cells/cm2 onto the Transwell Membrane migration Culture System (BD Falcon, Bedford, NA), a polycarbonate membrane with a pore size of 8 µm in diameter. The bottom chamber had complete growth media as a chemoattractant. After 18 hours of migration, non-migratory cells were wiped away from the upper surface of the membrane, and migratory cells were fixed in phosphate buffered formalin for 20 minutes, and fixed cells were stained with 0.2% crystal violet for 20 minutes. Migrated cells were scored under microscope and the generated data from duplicates were statically analyzed using unpaired t-test and GraphPad Prism software (GraphPad Software Inc. San Diego, CA, USA).

### Colony Formation

For these assays, cells were transfected with different siRNAs for control and target genes and placed in 6-well plates in triplicates and incubated at 37°C for 18 h. 24 hr after transfection 1000 cells were platted in 6 well plate and incubated for 2 weeks.

The number of colonies was determined by counting each plate after crystal violet staining.

### Anchorage-independent growth in soft agar

For these assays, cells transfected with different siRNAs for control and target genes were trypsinized, counted and then resuspended at 5 x 103 cells in 0.35% agar in culture medium over a layer of 0.5% agar/lx DMEM, into 6-well plates and incubated in the presence of 2 ml of DMEM supplemented with 10% FCS. The immobilized cells were grown for 14-21 days in a humidified chamber at 37°C with 5% CO2. Plates were stained with 0.005% crystal violet in phosphate buffered saline for 1 hour.

### Western blot

Protein from conditioned cell culture media was collected and cell lysate samples were extracted using M-PER Mammalian Protein Extraction Reagent (Pierce, Rockford, IL, USA) suplemmented with halt protease inhibitor (Roche, Basel, Switzerland). Protein concentration was determined with Bradford reagent using a Polarstar Optima microplate reader (MBMG Labtechnologies, Drham, NC, USA), and equal amounts of protein samples were used for SDS-PAGE protein assay. The protein samples were size separated on 12% polyacrylamide gel (NuPage LDS Novex Invitrogen, CA, USA) and transferred onto PVDF membrane using semidry Bio-Rad (Hercules, CA, USA) transfer system. The membranes were blocked with blocking system (NuPage, Invitrogen, CA, USA) for 1h at room temperature, and membranes were washed in phosphate buffer saline suplemmented with Tween-20. The primary antibodies diluents were applied on the membranes overnight at 4°C. The following polyclonal antibodies were used: anti-MELK (1:1000, JP Tassan, Rennes, France), anti-PLP2 (1:1000, Aviva ARP45348-T100, San Diego, CA, USA), and anti-β actin (1:5000, Sigma, MO, USA) was used for cellular protein loading control. Following incubation with primary antibodies, membranes were washed in PBST, and incubated with anti-rabbit (Amershan, Pharmacia Biotech Inc. Piscataway, NJ, USA) horseradish peroxidase-conjugated secondary antisera for chemiluminescent detection.

### MELK gene copy number quantification

To determine the gene amplification status *of MELK,* QT-PCR was performed using tissue DNA from the same casuistic used for analysis of relative expression. A single copy gene, hemoglobin beta (*HBB*) gene was used as reference. Primer sequences (IDT) were as follows (5' to 3'): *MELK* intron 7 F: GCTTTGCGACAATTCTGTGA (SEQ ID NO:15) and *MELK* exon 8 R: ACAGTATGCCCATGCTCCAA (SEQ ID NO:16), *HBB* F: GTGAAGGCTCATG GCAAGA (SEQ ID NO:17) and *HBB* R: AGCTCACTCAGTGTGGCAAAG (SEQ ID NO:18). Power Sybr Green I amplification mixtures (12 µL), DNA and primers (600nM) were used for gene amplification analysis in QT-PCR reaction. The relative amount *of MELK* with respect to the control gene DNA yield, *HBB,* was calculated using the equation ΔCt = Ct *HBB -* Ct *MELK* The ΔCt values over 2.0 were considered as having amplification.

### MELKpromoter methylation analyses

Bisulfite DNA treated from breast tumor cell lines (MDA-MB-435, American Type Culture Collection-ATCC, HTB-129, Manassas, VA) without (mock) and with 5-aza-2'deoxycytidine (AZA) treatment and DNA from lymphocyte were used as control for PCR reaction and methylation status. DNA from 2 non-neoplastic brain tissues, 1 GI (PA), 2 low-grade astrocytomas, 1 anaplastic astrocytoma and 5 GBM tissues were analyzed for methylation. Bisulfite modification of genomic DNA was performed as described previously¹³. Selected region containing CpG island (39 CpG count) of *MELK* genomic locus were amplified from bisulfite-modified DNA by nested PCR. To amplify the regions flanking this region, an initial PCR was performed with the following external primers, specific for bisulfite-converted DNA (5'-3'), *MELK* F: GGG tAA Gat tAA GGt tTt AAG TtA (SEQ ID NO:19) and *MELK* R: CTT AaC Cta AAC ACC TCT TC (SEQ ID NO:20). The product of this initial PCR (601bp) was used as template for a second PCR (440bp), using nested primers (5'-3'): *MELK* FN: AGA ttA AGG ttT tAA GTt Att ttT (SEQ ID NO:21) and *MELK* RN: AaA aCC Aaa AaC AAA Cta aaT Tta aaC (SEQ ID NO:22).

Nested PCR products from bisulfite treated control DNAs, were cloned using the pGEM-T Easy Vector System I (Promega, Madison, WI). M13-PCR product was sequenced by the Big Dye^{™} Terminator Cycle Sequencing Ready Reaction Kit version 3.1 (Perkin Elmer Applied Biosystems, USA) on an ABI Prism 3100 automated sequencer.

### MELK RNA interference

siGENOME SMARTpool and single siRNA duplexes were obtained from Dharmacon Research (Lafayette, CO) and diluted according to manufacturer's protocol. Human glioma cell lines were transfected with MELK or non-targeting siRNAs (using Dharmacon duplxes), using Lipofectamine 2000 (Invitrogen Inc, Carlsbad, CA). Specific silencing of target gene was confirmed by QT-PCR, at indicated time points.

### Assessment of apoptosis

Samples of 1 x 10⁵ cells were washed in phosphate-buffered saline and incubated with 5 µl of PI and annexin-V-FITC solutions (BD Farmingen, San Diego, CA) for 15 min. in the dark in order to assess cell membrane integrity and exposure of phosphatidylserine residues respectively. Fluorescence was measured by flow cytometry (FACSCalibur, BectonDickinson, San Jose, CA).

### Cell proliferation assay

For analysis of cell proliferation, cells transfected with *MELK-*specific or non-targeting siRNA were seeded in a 96-well plate (6000 cells/ well). Non-transfected cells were used as a negative control. Proliferative activity was determined by the MTT (3-(4,5-dimethylthiazol-2-yl)-2,5-diphenyltetrazolium bromide) assay. The absorbance of each well was measured at 580 nm on a microplate reader.

### Gene expression in normal tissues and cancer cell lines.

c-DNAs were prepared from RNAs from normal tissues (Ambion, Austin, TX) and BD Biosciences (Palo Alto, CA). For Quantitative real-time reverse transcription-PCR, cDNA samples were run in duplicate for *MELK* and for *ACTB* as the reference gene within the same experiment using Taqman platform in the Applied Biosystem apparatus 7500 Fast Real-Time PCR system and the Taqman fast universal PCR mix (Applied Biosystems, Foster City CA). PCR primers and probes were purchased from Applied Biosystems. PCR conditions were 95°C for 10 minutes followed by 40 cycles at 95°C for 15 seconds and 60°C for 1 minute. Relative quantification of gene expression was determined using the equation 2^{-ΔΔCT}.

### Statistical analysis

The statistical analysis of relative gene expression in different grades of astrocytomas and non-neoplastic tissues was assessed using the Mann-Whitney test and the analyses of anchorage-independent growth in soft agar of malignant astrocytomas cell lines was performed by Student's t-test (non parametric). Differences were considered to be statistically significant at *p*<0.05. Overall survival (OS) was calculated as the interval between day of surgery and day of death, in months. Patients alive on the day of analysis were censored on December 09, 2006. Calculations were performed using SPSS 10.0 software (SPSS, Chicago, IL).

### References

[i] Louis DN, Ohgaki H, Wiestler OD, Cavenee WK, Burger PC, Jouvet A, Scheithauer BW, Kleihues P. The 2007 WHO classification of tumours of the central nervous system. Acta Neuropathol. 2007 Aug;114(2):97-109.
[ii] Livak KJ, Schmittgen TD. Analysis of relative gene expression data using real-time quantitative PCR and the 2(-Delta Delta C(T)) Method. 25(2001):402-408.
[iii] Davezac N, Baldin V, Blot J, Ducommun B, Tassan JP. Human pEg3 kinase associates with and phosphorylates CDC25B phosphatase: a potential role for pEg3 in cell cycle regulation. Oncogene. 2002 Oct 31;21(50):7630-41.

### Results

### Identification of genes exhibiting higher levels of expression in astrocytomas than in non-neoplastic brain tissues by microarray analysis.

Microarray analyses were undertaken using cDNA prepared from three pilocytic astrocytomas (PA, grade I), six glioblastomas multiforme (GBM, grade IV) and a pool of three non-neoplastic brain tissue samples. In all pairwise comparisons, the average of the coefficients of variation for duplicates ranged from 6.5 to 9.0%, with Pearson correlation coefficients being higher than 0.98. For all duplicates, more than 99.7% of the values were within a 2-fold difference.

Similar numbers of genes, 562 and 555, were found to be at least two-fold over-expressed in the GBM and GI (PA) samples as compared to the pooled non-neoplastic tissues, respectively. Of these, 319 were over-expressed genes in both tissues. However in a comparison of each of the GBM and GI (PA) samples in a pair wise fashion, only 63 genes were found to have at least a two-fold higher level of expression in all GBM samples as compared with the GI (PA) samples, as indicated in Table I..

A functional classification of the GBM over-expressed genes revealed that more than 50% are related to the cell cycle and mitosis (Table I). Several of these genes have been reported to be associated with cellular transformation and increased malignancy in a number of different cancer types including gliomas¹⁴⁻¹⁷ as indicated in Table II. It is noteworthy that other microarray studies have consistently identified *CDC20, UBE2C, PCNA, KIAA0101* and *BIRC5* as markers of malignancy.

The microarray data was validated by QT-PCR for five of the genes: *MELK, , ASPM,* LOX, HOXA5, and *KIAA0101* (Figure 1). The finding of the consistent high level of expression *of MELK* in aggressive glioblastomas was of interest given that it has been recently identified as a potential therapeutic target on the basis of its over-expression in a number of cancer types⁹. Moreover, it has been demonstrated to be a key regulator of the proliferation of multipotent neural progenitors¹⁰ We thus elected to characterize the expression of *MELK* in more detail and investigate whether it might be directly related to the malignant properties of GBM as has been shown in cell lines derived from other cancer types.

### MELK expression correlates with tumor grade.

As a first step we measured *MELK* mRNA levels by QT-PCR in an extended series of tumor samples including both grade II and grade III astrocytomas as well as medulloblastomas. We confirmed in this larger data set the consistent and high levels of expression in grade IV GBMs (645-fold that of normal tissues) with just two of the 53 samples studied exhibiting aberrantly low levels of expression similar to that found in normal tissues. The grade II and grade III tumors were found to exhibit progressively higher levels of *MELK* expression (162- and 221-fold, respectively) as compared to non-neoplastic samples. The median expression level *of MELK* was 88.2-fold higher in GI (PA) than normal tissues, thus overall demonstrating an increase in *MELK* expression in parallel to the increase in malignancy (Fig. 1D). *MELK* expression levels in medulloblastoma (medullo) were similar to GBM. There was no direct relationship between *MELK* expression level in GBM and survival time by Kaplan-Meier analysis (log rank *p*=0.455) which probably reflects the relatively uniform expression of the gene in tumors (data not shown).

### Lack of evidence for MELKpromoter methylation or gene amplification.

The phenomenon of very low level expression of embryonic genes in adult tissues and their up regulation in tumors can be due to promoter methylation in normal tissues that is reversed during tumorigenesis. We examined this possibility for *MELK* and identified a CpG island (39CpG count) located at nucleotides -119 to -259 in relation to the transcription start site (+1). Methylation status in this island was measured by sequencing bisulfite-modified DNA but no difference was found in the promoter methylation profile in the bisulfite-treated DNA from astrocytic tumor tissues (one GI (PA), two low grade astrocytomas and five GBM samples) and two non-neoplastic tissues as controls (data not shown). We also asked whether gene up regulation might be accompanied by gene amplification, which is the case for a number of oncogenes. However, no MELK gene amplification was found by QT-PCR in 93 DNA astrocytoma samples with different grades of malignancy as compared with normal tissues (data not shown).

### MELK depletion by siRNA results in apoptosis-dependent growth inhibition in glioma cell lines and reduced clonogenicity.

There is evidence that *MELK* can directly influence proliferation and anchorage-independent growth, but this has not as yet been established in the context of glioblastoma. To investigate this possibility, we used small interference RNAs (21-mer siRNA pool, obtained from Dharmacon) to reduce *MELK* mRNA levels in two malignant astrocytoma cell lines (T98G and U87MG). This procedure had the effect of reducing *MELK* expression levels by up to 95%. This effect on *MELK* mRNA was apparently specific, since the non-targeting control (NT) had no effect and was sustained for at least seven days post-transfection, when approximately a 65% knockdown was still detectable (data not shown). The treatment of both U87MG and T98G with *MELK*-targeting 21-mer siRNA pools resulted in a reduction in cell proliferation (Fig. 14 A). This was accompanied by a modest but significant increase of cleaved caspase-3 positive cells (p < 0.01) or annexin V-stained cells (p < 0.05), as detected by FACS analysis on days 2 and 7 (data not shown), suggesting that the observed alterations in growth kinetics was, at least in part, due to enhanced apoptosis.

Anchorage-independent growth assays were next used to examine the influence of *MELK* on colony formation. Both T98G and U87MG cell lines were transfected with 21-mer siRNA against *MELK* or non-targeting siRNA and, as shown in Fig. 14B. This resulted in reduced colony formation on soft agar by the T98G cells. A reduction in colony number was not observed with the U87MG cells although they did exhibit a 30% relative reduction in size as compared with controls over the time period of the experiment (data not shown). Overall, these results show that this level of inhibition on *MELK* expression is sufficient to partially modulate tumor growth suppression.

### Quantitative analysis of MELK expression in normal tissues.

Given the emerging evidence of the importance *of MELK* in astrocytoma malignancy, and hence its potential utility as a therapeutic target, we assessed its expression in a variety of normal tissues by RT-PCR (Fig. 10). The apparent absence of *MELK* expression in normal brain is consistent with the data presented above and encouraging in terms of its utility as a target. However, there were significant levels of expression detectable in gastrointestinal tissues that would have to be taken into account in the potential utilization of MELK inhibitors.

### Discussion

GBM is resistant to conventional therapies and survival time is usually shorter than 12 months. Therefore, the identification of additional molecular alterations involved in the establishment the aggressive behavior of astrocytic tumors, and that can be targeted by pharmacological agents, is a critical challenge. A number of targetable genetic changes have been described in human gliomas, such as *EGFR* over-expression/amplification and activation of tyrosine kinase receptors, but they are not applicable to all tumors^{17,18}

Genome-wide analysis of gene expression is a powerful approach for identifying therapeutic targets for cancer treatment^{11, 16, 18-22}. However, comparison of global expression profiles of normal and tumor cells usually generates hundreds of differentially expressed genes in cancer relative to normal tissue, most of them probably not playing critical roles in the tumorigenic process. For this reason, we decided to compare GI (PA), a non-invasive indolent tumor with GBM, which is highly invasive and rapidly lethal, with the hope of identifying genes directly involved in malignancy. When either GBM or GI (PA) were compared to non-neoplastic brain tissues, over five hundred genes were found differentially expressed in each case. When GBM was compared to GI (PA), on the other hand only 63 genes were found to have at least a two-fold different level of expression between these tumor types. Several groups have analyzed the profile of proliferation in various cancer types including gliomas and Table II shows a list of genes in common between our array and published data. Rickman et al.¹⁶ found 167 genes over-expressed in a molecular profile study of high-grade (GBM) and low-grade GI (PA) gliomas based on oligonucleotide microarray analysis; among those, we also found seven exhibiting differential expression between GBM and GI (PA): *CKS2, CDC20, IGFBP2, IAP4, UBE2C, PCNA* and *FLNA.* Kathua et al.¹⁷ also compared high-grade astrocytomas versus benign low-grade pediatric astrocytomas using a DNA microarray and found seventeen over-expressed genes, *IGFBP2* among them. Rhodes et al.¹⁵ used a large-scale meta-analysis of several cancer microarrays data and identified *CDC20, UBE2C, CKS2, KIF2C, BIRC5, KIAA0101, CENPA* and *MELK* among 43 genes that form a common transcriptional profile of human cancer. On the other hand, our results showed no overlap with the list of genes generated by Colin et al.²² using Suppression Substractive Hybridization method to compare differentially expressed genes in GBM versus GI (PA), possibly due to significant differences in the methodology.

We noted that in our list of genes with two fold or more over expression in GBM versus GI (PA), most (>50%) were related to cell proliferation. These genes encode proteins that act directly in the cell cycle, such as cyclin B1, cyclin A2, CDC20, CDC2, CDC25c, CDC28, and proliferating cell nuclear antigen, or that function in the regulation or assembly of the mitotic spindle, including centromeric proteins, NIMA-related kinase 2, protein regulator of cytokinesis 1, aurora kinase B, and BIRC5 (survivin). Survivin is also known for its anti-apoptotic activity as a member of the inhibitor of apoptosis (IAP) family. High expression levels of survivin have been reported previously in GBM, and this gene has also been identified as a marker for poor prognosis²³. Survivin interacts with aurora kinase B, a serine- threonine kinase, binding to the catalytic domain and stimulating the kinase activity, thereby promoting cytokinesis. Given their importance in mitosis, aurora kinase and survivin have been selected as a target for a new generation of drugs that inhibit mitosis²⁴⁻²⁶. Our results strengthen the correlation between malignancy grade and the expression level of these proteins. *ASPM* was also over-expressed in our comparison between GBM and GI (PA), and has recently been described as a highly connected 'hub' gene within module of mitosis/cell cycle co-expressed genes in GBM and breast cancer¹⁴. Likewise *PRC1, AURKB* and *ASPM* were described previously as highly expressed in GBM compared to non-neoplastic tissue²⁵ In the present study, these genes were selected among the highly expressed in GBM compared to GI (PA), suggesting that they are related not only to the tumorigenic process itself, but also to the increase of malignancy.

Additionally, among the most highly expressed genes in GBM in relation to GI (PA) we found interleukin-13 receptor alpha-2 (*IL13RA2*) (Table I), which has already been shown to be up-regulated in distinct cancers, including GBM by independent studies (PMID: 10946814). *IL13RA2* is thought to activate transcription (*JAK*/*STAT*) signaling cascades and may contribute to survival and proliferation by stimulation of the phosphatidyl-inositol 3-kinase (*PI3K*) pathway, through recruitment of members of the insulin receptor substrate family²⁷. This protein has been considered as a therapeutic target in this disease²⁸⁻³⁰.

Other putative tumor marker genes found in the present study, such as the *HOX* genes, have only been more recently reported in high-grade astrocytomas³¹. The transcriptome of GBM seems to be enriched for homeobox protein messages, specifically *HOXC9* and *HOXA5,* whose levels were respectively 8- and 16-fold higher in GBM compared with GI (PA). This family of transcription factors regulates a variety of developmental and cellular processes and is required for neuronal differentiation of embryonic stem cells induced by retinoic acid^{32,33}. Dysregulation of homeobox genes has been associated with many different cancer types³⁴, including GBM³².

An ancillary contribution of this study is the generation of functional data for somewhat uncharacterized human genes. Among them, we have detected *KIAA0101* that encodes a proliferating cell nuclear antigen (PCNA)-interacting protein named p15 (PAF), as 13.98-fold increased in GBM compared to GI (PA). Its expression has been described as down-regulated in human hepatocellular carcinoma³⁵ but up-regulated in several other tumors, including thyroid carcinoma, follicular lymphoma, esophageal tumors and non-small-cell lung cancer cell lines^{36,37} The contribution of such uncharacterized proteins to the malignancy of astrocytomas and their relevance to prognosis and therapy is worthy of further investigation.

Among the over-expressed genes in GBM, we selected *MELK* as being of particular interest. Although this gene has received very little attention in the context of human malignancy until recently, it is emerging as an important and possibly ubiquitous contributor to this process. We noted here that its expression correlated with grade of malignancy and exhibited an almost uniformly high level of expression in GBM and medulloblastoma. This stands in contrast to the range of levels of expression of other key genes in GBM such as *EGFR*³⁸*.* Only 2 out of 53 GBM patients presented *MELK* expression overlapping to the expression of non-neoplastic brain tissue. These two patients were submitted to tumor resection surgery at 50 and 71 years of age, and survived for 7 and 2 months, respectively. This homogeneous aspect of expression among GBM and medulloblastoma potentially represents an advantage for the use of *MELK* as a therapeutic target. In this regard, we were able to directly demonstrate that MELK functions in the context of human astrocytomas, by knock down of its expression by RNA interference in malignant astrocytoma cell lines. As a result growth-inhibitory response, as determined by the MTT cell proliferation assay, as well a decrease in anchorage-independent growth were observed.

In addition, a modest but significant increase in both caspase-3 expression and in annexin-V-staining may indicate that *MELK* has a role in inhibiting apoptosis in malignant astrocytoma cells.

*MELK* is a protein Ser/Thr kinase implicated in cell cycle progression, in the context of human cancer, *MELK* expression was found to be significantly lower in growth-arrested non-malignant human mammary epithelial cells that form organized polarized acini *in vitro* than in their proliferating counterparts corroborating its role in the proliferative process. This attribute allowed the use of *MELK* expression as a marker to classify breast cancer patients into groups of differential prognosis³⁹. The results of the present study specifically add GBM and medulloblastoma to the list of tumors for eventual therapeutical trials involving *MELK.*

*MELK* has been implicated in the control of cell proliferation during development⁴⁰ being found enriched in embryonic and adult neural stem/progenitor cells and required for their self-renewal capacity^{10,41}. Deregulation of the molecular pathways governing stem cell-like behavior in cancer cells may be critical for the development of new strategies for cancer prevention and therapy. The increased expression *of MELK* in the CD133 positive compartment of gliomas⁴² may indicate that MELK over-expression is a hallmark of cancer stem cells and that targeting MELK activity in cancer stem cells may thus be useful to increase the efficacy of GBM treatment.

In conclusion, the striking contrast in clinical and pathological behavior between GBM and GI (PA) is associated with a rather limited divergence at the gene expression level, with transcripts for protein involved mainly in cell proliferation accounting for the main differences. These findings provide additional information concerning the molecular nature of astrocytomas of distinct grades of malignancy, which ultimately may contribute towards improving tumor classification and therapeutic strategies. Among these differentially expressed genes, we have found that *MELK* expression correlates with malignant grades of human astrocytic tumors. The effects *of MELK* silencing on proliferation, apoptosis and anchorage independent growth of malignant astrocytic cells suggest that *MELK* deregulation may contribute to the malignant progression of this tumor type. *MELK* over-expression also occurs in the most frequent pediatric brain tumor, medulloblastoma, reinforcing its potential as a candidate for therapeutic approaches.

**Table I. Genes over-expressed in glioblastoma relative to pilocytic astrocytoma, as indicated by microarray analysis. ("Astrocytoma markers")**

| **Accession No.** | **Description** | ***locus*** | **Mean fold change** |
|---|---|---|---|
| *Cell Proliferation* | | | |
| NM_001255 | CDC20 cell division cycle 20 homolog (S. cerevisiae) (CDC20) | 1p34.1 | 20.07 |
| NM_004217 | aurora kinase B (AURKB) | 17p13.1 | 16.1 |
| NM_002497 | NIMA (never in mitosis gene a)-related kinase 2 (NEK2) | 1q32.2-q41 | 15.72 |
| NM_001809 | centromere protein A, 17kDa (CENPA) | 2p24-p21 | 13.68 |
| NM_001657 | amphiregulin (schwannoma-derived growth factor) (AREG) | 4q13-q21 | 11.2 |
| NM_001786 | cell division cycle 2, G1 to S and G2 to M (CDC2) | 10q21.1 | 9.76 |
| NM_006845 | kinesin family member 2C (KIF2C) | 1p34.1 | 8.66 |
| NM_001827 | CDC28 protein kinase regulatory subunit 2 (CKS2) | 9q22 | 7.95 |
| NM_002358 | MAD2 mitotic arrest deficient-like 1 (yeast) (MAD2L1) | 4q27 | 7.77 |
| NM_001813 | centromere protein E, 312kDa (CENPE) | 4q24-q25 | 7.7 |
| NM_031966 | cyclin B1 (CCNB1) | 5q12 | 7.08 |
| NM_016343 | centromere protein F, 350/400ka (mitosin) (CENPF) | 1q32-q41 | 7.06 |
| NM_003981 | protein regulator of cytokinesis 1 (PRC1) | 15q26.1 | 6.66 |
| NM_003318 | TTK protein kinase (TTK) | 6q13-q21 | 6.38 |
| NM_022346 | chromosome condensation protein G (NCAPG) | 4p15.33 | 6.11 |
| NM_012112 | TPX2, microtubule-associated protein homolog (Xenopus laevis) (TPX2) | 20q1.2 | 5.96 |
| NM_003035 | TAL1 (SCL) interrupting locus (STIL) | 1p32 | 4.22 |
| NM_001237 | cyclin A2 (CCNA2) | 4q25-q31 | 3.78 |
| *Cell proliferalion*/*DNA damage* | | | |
| NM_000597 | insulin-like growth factor binding protein 2,36kDa (IGFBP2) | 2q33-q34 | 18.14 |
| NM_001274 | CHK1 checkpoint homolog (S. pombe)(CHK1) | 11q24-q24 | 9.55 |
| NM_199420 | polymerase (DNA directed), theta (POLQ) | 3q13.33 | 8.9 |
| NM_022809 | cell division cycle 25C (CDC25C) | 1p34.1 | 8.61 |
| NM_001827 | CDC28 protein kinase regulatory subunit 2 (CKS2) | 9q22 | 7.95 |
| NM_002875 | RAD51 homolog (RecA homolog, E. coli) (S. cerevisiae) (RAD51) | 15q15.1 | 7.01 |
| NM_002592 | proliferating cell nuclear antigen (PCNA) | 20pter-p12 | 2.72 |
| *DNA and Protein metabolism* | | | |
| NM_007019 | Ubiquitin-conjugating enzyme E2C (UBE2C) | 20q13.12 | 17 |
| NM_014791 | maternal embryonic leucine zipper kinase (MELK) | 9p13.2 | 16.45 |
| NM_002317 | | 5q23.3- | |
| | lysyl oxidase (LOX) | q31.2 | 11.04 |
| NM_003294 | tryptase alpha/beta 1 (TPSAB1) | 16p13.3 | 8.99 |
| NM_014176 | HSPCI50 protein similar to ubiquitin-conjugating enzyme (UBE2T) | 1q32.1 | 7.85 |
| NM_018518 | MCM10 minichromosome maintenance deficient 10 (S. cerevisiae) (MCM10) | 10p13 | 6.3 |
| NM_000943 | peptidylprolyl isomerase C (cyclophilin C) (PPIC) | 5q23.2 | 5.31 |
| NM_001071 | thymidylate synthetase (TYMS) | 18p11.32 | 4.26 |
| NM_030928 | DNA replication factor (CDT1) | 16q24.3 | 3.76 |
| *Transcription* | | | |
| NM_019102 | homeobox protein (HOXA5) | 7p15-p14 | 16.97 |
| NM_006897 | homeobox C9 (HOXC9) | 12q13.3 | 8.29 |
| NM_004237 | thyroid hormone receptor interactor 13 (TRIP13) | 5p13.33 | 7.34 |
| BC033086 | transcription factor 19 (SC1) (TCF19) | 6q21.3 | 6.09 |
| *Cell adhesion* | | | |
| NM_019119 | protocadherin beta 9 (PCDHB9) | 5q31 | 9.81 |
| NM_004369 | collagen, type VI, alpha 3 (COL6A3) | 2q27 | 6.47 |
| *Signal transduction*/*Receptors* | | | |
| NM_000640 | interleukin 13 receptor, alpha 2 (IL13RA2) | Xq13.1-q28 | 32.62 |
| NM_001999 | fibrillin 2 (congenital contractural arachnodactyly) (FBN2) | 5q23-q31 | 13.61 |
| NM_006681 | neuromedin U (NMU) | 4q12 | 11.75 |
| NM_018154 | ASF1 anti-silencing function 1 homolog B (S. cerevisiae) (ASF1B) | 19p13.12 | 9.78 |
| NM_007280 | Opa-interacting protein 5 (OIP5) | 15q15.1 | 7.2 |
| NM_000599 | insulin-like growth factor binding protein 5 (IGFBP5) | 2q33.q36 | 7.16 |
| NM_018098 | | 3q26.1- | 5.8 |
| | epithelial cell transforming sequence 2 oncogene (ECT2) | q26.2 | |
| NM_005497 | gap junction protein, alpha 7, 4SkDa (connexin 45) (GJA7) | 17q21.31 | 5.29 |
| NM_006863 | leukocyte immunoglobulin-like receptor, subfamily A , member I (LILRA1) | 19q13.4 | 3.09 |
| *Angiogenesis*/*Defense* | | | |
| NM_000584 | interleukin 8 (IL8) | 4q13-q21 | 7.11 |
| NM_000636 | superoxide dismutase 2, mitochondrial (SOD2) | 6q25.3 | 5.75 |
| *Cytoskeleton* | | | |
| NM_001456 | filamin A, alpha (actin binding protein 280) (FLNA) | Xq28 | 4.47 |
| NM_002705 | periplakin (PPL) | 16p13.1 | 3.57 |
| *Apoplosis inhibitors* | | | |
| NM_001168 | baculoviral IAP repeat-containing 5 (BIRC5) | 17q25 | 16.46 |
| *Organogenesis* | | | |
| NM_001849 | collagen, type VI, alpha 2 (COL6A2) | 21q22.3 | 11.46 |
| *Unknown function* | | | |
| NM_018136 | asp (abnormal spindle)-like, microcephaly associated (Drosophila) (ASPM) | 1q31 | 16.88 |
| NM_014736 | KIAA0101 gene product (KIAA0101) | 15q22.31 | 13.98 |
| NM_018410 | hypothetical protein (DKFZp762E1312) | 2q37.1 | 9.2 |
| NM_182487 | olfactomedin-like 1A (OLFML2A) | 9q33.3 | 8.02 |
| NM_018455 | uncharacterized bone marrow protein BM039 (CENPN) | 16q23.2 | 4.69 |
| NM_018454 | nucleolar protein ANKT (NUSAP1) | 15q15.1 | 4.56 |
| NM_030804 | hypothetical protein DKFZp434E2135 (FZD5) | 2q33-q34 | 3.88 |
| NM_015426 | DKFZP434C245 protein (WDR51A) | 3p21.1 | 3.56 |

**Table II. Comparison between GBM versus GI (PA) differentially expressed genes**

| **Type of assay** | **Total genes** | **Genes in common to our GBM vs. GI/PA** | **Reference** |
|---|---|---|---|
| c-DNA microarray (6,800 genes, 21 grade IV vs. 9 grade I astrocytoma tissues) | 133 | *CKS2, CDC20, BIRC5. UBE2C, PCNA, FLNA, CENPA, K1AA0101, IL13RA2, CCNB1, KIF2C, CENPF, MELK* | 16 |
| Comparative meta-profiling of 40 microarray data sets (3,762 microarrays) | 43 (*) | *CDC20, UBE2C, PCNA, BIRC5, KIAA0101, CENPA, MELK, KIF2C, CKS2* | 15 |
| c-DNA microarray (12,000 genes, 6 grade IV vs. grade I astrocytoma) | 15 | *IGFBP2* | 17 |
| Suppression Substractive Hybridization (648 clones GBM vs. 648 GI/PA) | 32 | None | 22 |

| | | | |
|---|---|---|---|
| (*) 43 common over-expressed genes comparing high and low grade lung adenocarcinoma, bladder carcinoma, breast ductal carcinoma, glioma, ovarian carcinoma, prostate carcinoma | | | |

### References:

1. Kleihues P, Louis DN, Scheithauer BW, Rorke LB, Reifenberger G, Burger PC, et al. The WHO classification of tumors of the nervous system. J Neuropathol Exp Neurol 2002;61(3):215-25; discussion 26-9.
2. von Deimling A, Louis DN, Schramm J, Wiestler OD. Astrocytic gliomas: characterization on a molecular genetic basis. Recent Results Cancer Res 1994;135:33-42.
3. Korshunov A, Sycheva R, Golanov A. The prognostic relevance of molecular alterations in glioblastomas for patients age < 50 years. Cancer 2005;104(4):825-32.
4. von Deimling A, Louis DN, Wiestler OD. Molecular pathways in the formation of gliomas. Glia 1995;15(3):328-38.
5. Brentani H, Caballero OL, Camargo AA, da Silva AM, da Silva WA, Jr., Dias Neto E, et al. The generation and utilization of a cancer-oriented representation of the human transcriptome by using expressed sequence tags. Proc Natl Acad Sci U S A 2003;100(23):13418-23.
6. Velculescu VE, Zhang L, Vogelstein B, Kinzler KW. Serial analysis of gene expression. Science 1995;270(5235):484-7.
7. Jongeneel CV, Iseli C, Stevenson BJ, Riggins GJ, Lal A, Mackay A, et al. Comprehensive sampling of gene expression in human cell lines with massively parallel signature sequencing. Proc Natl Acad Sci U S A 2003;100(8):4702-5.
8. Grigoriadis A, Mackay A, Reis-Filho JS, Steele D, Iseli C, Stevenson BJ, et al. Establishment of the epithelial-specific transcriptome of normal and malignant human breast cells based on MPSS and array expression data. Breast Cancer Res 2006;8(5):R56.
9. Gray D, Jubb AM, Hogue D, Dowd P, Kljavin N, Yi S, et al. Maternal embryonic leucine zipper kinase/murine protein serine-threonine kinase 38 is a promising therapeutic target for multiple cancers. Cancer Res 2005;65(21):9751-61.
10. Nakano I, Paucar AA, Bajpai R, Dougherty JD, Zewail A, Kelly TK, et al. Maternal embryonic leucine zipper kinase (MELK) regulates multipotent neural progenitor proliferation. J Cell Biol 2005;170(3):413-27.
11. Oba-Shinjo SM, Bengtson MH, Winnischofer SM, Colin C, Vedoy CG, de Mendonca Z, et al. Identification of novel differentially expressed genes in human astrocytomas by cDNA representational difference analysis. Brain Res Mol Brain Res 2005;140(1-2):25-33.
12. Cavalieri D, De Filippo C. Bioinformatic methods for integrating whole-genome expression results into cellular networks. Drug Discov Today 2005;10(10):727-34.
13. Jeronimo C, Usadel H, Henrique R, Silva C, Oliveira J, Lopes C, et al. Quantitative GSTP1 hypermethylation in bodily fluids of patients with prostate cancer. Urology 2002;60(6):1131-5.
14. Horvath S, Zhang B, Carlson M, Lu KV, Zhu S, Felciano RM, et al. Analysis of oncogenic signaling networks in glioblastoma identifies ASPM as a molecular target. Proc Natl Acad Sci U S A 2006;103(46):17402-7.
15. Rhodes DR, Yu J, Shanker K, Deshpande N, Varambally R, Ghosh D, et al. Large-scale meta-analysis of cancer microarray data identifies common transcriptional profiles of neoplastic transformation and progression. Proc Natl Acad Sci USA 2004;101(25):9309-14.
16. Rickman DS, Bobek MP, Misek DE, Kuick R, Blaivas M, Kurnit DM, et al. Distinctive molecular profiles of high-grade and low-grade gliomas based on oligonucleotide microarray analysis. Cancer Res 2001;61(18):6885-91.
17. Khatua S, Peterson KM, Brown KM, Lawlor C, Santi MR, LaFleur B, et al. Overexpression of the EGFR/FKBP12/HIF-2alpha pathway identified in childhood astrocytomas by angiogenesis gene profiling. Cancer Res 2003;63(8):1865-70.
18. van den Boom J, Wolter M, Kuick R, Misek DE, Youkilis AS, Wechsler DS, et al. Characterization of gene expression profiles associated with glioma progression using oligonucleotide-based microarray analysis and real-time reverse transcription-polymerase chain reaction. Am J Pathol 2003;163(3):1033-43.
19. Sallinen SL, Sallinen PK, Haapasalo HK, Helin HJ, Helen PT, Schraml P, et al. Identification of differentially expressed genes in human gliomas by DNA microarray and tissue chip techniques. Cancer Res 2000;60(23):6617-22.
20. Gutmann DH, Hedrick NM, Li J, Nagarajan R, Perry A, Watson MA. Comparative gene expression profile analysis of neurofibromatosis 1-associated and sporadic pilocytic astrocytomas. Cancer Res 2002;62(7):2085-91.
21. Mischel PS, Cloughesy TF, Nelson SF. DNA-microarray analysis of brain cancer: molecular classification for therapy. Nat Rev Neurosci 2004;5(10):782-92.
22. Colin C, Baeza N, Bartoli C, Fina F, Eudes N, Nanni I, et al. Identification of genes differentially expressed in glioblastoma versus pilocytic astrocytoma using Suppression Subtractive Hybridization. Oncogene 2006;25(19):2818-26.
23. Haberler C, Slavc I, Czech T, Gelpi E, Heinzl H, Budka H, et al. Histopathological prognostic factors in medulloblastoma: high expression of survivin is related to unfavourable outcome. Eur J Cancer 2006;42(17):2996-3003.
24. Jiang N, Wang X, Yang Y, Dai W. Advances in mitotic inhibitors for cancer treatment. Mini Rev Med Chem 2006;6(8):885-95.
25. Warner SL, Bashyam S, Vankayalapati H, Bearss DJ, Han H, Mahadevan D, et al. Identification of a lead small-molecule inhibitor of the Aurora kinases using a structure-assisted, fragment-based approach. Mol Cancer Ther 2006;5(7):1764-73.
26. Hou JQ, He J, Wang XL, Wen DG, Chen ZX. Effect of small interfering RNA targeting survivin gene on biological behaviour of bladder cancer. Chin Med J (Engl) 2006;119(20):1734-9.
27. Kelly-Welch AE, Hanson EM, Boothby MR, Keegan AD. Interleukin-4 and interleukin-13 signaling connections maps. Science 2003;300(5625):1527-8.
28. Kawakami M, Leland P, Kawakami K, Puri RK. Mutation and functional analysis ofIL-13 receptors in human malignant glioma cells. Oncol Res 2001;12(11-12):459-67.
29. Mintz A, Gibo DM, Madhankumar AB, Debinski W. Molecular targeting with recombinant cytotoxins of interleukin-13 receptor alpha2-expressing glioma. J Neurooncol 2003;64(1-2):117-23.
30. Husain SR, Puri RK. Interleukin-13 receptor-directed cytotoxin for malignant glioma therapy: from bench to bedside. J Neurooncol 2003;65(1):37-48.
31. Abdel-Fattah R, Xiao A, Bomgardner D, Pease CS, Lopes MB, Hussaini IM. Differential expression of HOX genes in neoplastic and non-neoplastic human astrocytes. J Pathol 2006;209(1):15-24.
32. Mavilio F, Simeone A, Boncinelli E, Andrews PW. Activation of four homeobox gene clusters in human embryonal carcinoma cells induced to differentiate by retinoic acid. Differentiation 1988;37(1):73-9.
33. Simeone A, Acampora D, D'Esposito M, Faiella A, Pannese M, Scotto L, et al. Posttranscriptional control of human homeobox gene expression in induced NTERA-2 embryonal carcinoma cells. Mol Reprod Dev 1989;1(2):107-15.
34. Maroulakou IG, Spyropoulos DD. The study of HOX gene function in hematopoietic, breast and lung carcinogenesis. Anticancer Res 2003;23(3A):2101-10.
35. Guo M, Li J, Wan D, Gu J. KIAA0101 (OEACT-1), an expressionally down-regulated and growth-inhibitory gene in human hepatocellular carcinoma. BMC Cancer 2006;6:109.
36. Mizutani K, Onda M, Asaka S, Akaishi J, Miyamoto S, Yoshida A, et al. Overexpressed in anaplastic thyroid carcinoma-1 (OEATC-1) as a novel gene responsible for anaplastic thyroid carcinoma. Cancer 2005;103(9):1785-90.
37. Bjorck E, Ek S, Landgren O, Jerkeman M, Ehinger M, Bjorkholm M, et al. High expression of cyclin B1 predicts a favorable outcome in patients with follicular lymphoma. Blood 2005; 105(7):2908-15.
38. Scrideli CA, Carlotti CG, Jr., Mata JF, Neder L, Machado HR, Oba-Sinjo SM, et al. Prognostic significance of co-overexpression of the EGFR/IGFBP-2/HIF-2A genes in astrocytomas. J Neurooncol 2007.
39. Fournier MV, Martin KJ, Kenny PA, Xhaja K, Bosch I, Yaswen P, et al. Gene expression signature in organized and growth-arrested mammary acini predicts good outcome in breast cancer. Cancer Res 2006;66(14):7095-102.
40. Badouel C, Komer R, Frank-Vaillant M, Couturier A, Nigg EA, Tassan JP. M-phase MELK activity is regulated by MPF and MAPK. Cell Cycle 2006;5(8):883-9.
41. Easterday MC, Dougherty JD, Jackson RL, Ou J, Nakano I, Paucar AA, et al. Neural progenitor genes. Germinal zone expression and analysis of genetic overlap in stem cell populations. Dev Biol 2003;264(2):309-22.
42. Liu G, Yuan X, Zeng Z, Tunici P, Ng H, Abdulkadir IR, et al. Analysis of gene expression and chemoresistance of CD 13 3+ cancer stem cells in glioblastoma. Mol Cancer 2006;5:67.

### Example 2: Validation of additional astrocytoma marker gene expression

Gliomas of astrocytic origin are the most common type of primary brain tumors in the nervous system. The use of oligonucleotide microarrays to compare the expression pattern of genes between grade IV (glioblastoma) and grade I (pilocytic astrocytoma) astrocytic tumors resulted in the identification of genes involved in the progression of the disease and possible novel therapeutic targets. In further experiments, the expression of additional genes in astrocytomas, glioma cell lines and non-neoplastic tissues was analyzed.

### Quantitative real time RT-PCR (QT-PCR)

The relative expression levels of the listed genes were analyzed by QT-PCR to validate the microarray data. To this end, we used ten non-neoplastic brain tissues, and ninety astrocytic tumor samples: twelve GI (PA) (median age 22 years), thirteen low grade astrocytomas (median age 37 years), fifteen anaplastic astrocytomas (median age 31 years), and fifty three GBM (median age 52 years). All QT-PCR assays were carried out in duplicates by the Syber-Green approach, and normalization of quantitative data was based on the housekeeping gene hypoxanthine guanine phosphoribosyltransferase gene (HPRT):
HPRT F: TGAGGATTTGGAAAGGGTGT (SEQ ID NO:13) and HPRT R:
GAGCACACAGAGGGCTACAA (SEQ ID NO:14). The QT-PCR primer sequences for the other genes are shown in Figure 25.

Sybr Green I amplification mixtures (12 µL) contained 3 µL of cDNA, 6 µL of 2X Sybr Green I Master Mix (Applied Biosystems), and forward and reverse primers to a final concentration 200 nM to 400nM. Reactions were run on ABI 7500 Real-Time PCR Systems (Applied Biosystems). The equation 2-^{ΔΔCT} was applied to calculate the relative expression of tumor samples versus the median of non-neoplastic tissues. All primers were synthesized by IDT (Integrated DNA technologies, Inc, Coralville, IA).

For all genes analyzed we used Universal cycling conditions for Real time PCR (7500 Real Time PCR System). PCR conditions consisted in 50°C for 2 min, 95°C for 2 min followed by 40 cycles of 95°C for 15 sec and 60°C for 1 min. Power Syber Green I amplification mixtures (12 µL) contained 3 µL of cDNA, 6 µL of Power Syber Green Master Mix (Applied Biosystems), and forward and reverse primers. The final concentration of primers used for ASPM, KIAA0101, LOX and COL6A2 was 200nM, PLP2, DKFZp762E1312 was 400nM and for HOXA5 was 600nM.

### Relative expression of genes in stages of astrocytomas and in glioma cell lines

The relative expression of the genes identified above in astrocytomas of different stages, in glioma cell lines and in non-neoplastic tissue was tested using QT-PCR as described above. Figure 1 shows the correlation of malignancy with expression of the following genes: KIAA0101 (Fig. 1A), PLP2 (Fig. 1B), HOXA5 (Fig. 1C), MELK (Fig. 1D), LOX (Fig. 1E), DKFZp762E1312 (Fig. 1F), COL6A2 (Fig. 1G), and ASPM (Fig. 1H) and Table III lists the accompanying p values. It is clear that all of these genes are markers for the disease, exhibiting a low p value of GIV/N, and there is a significant increase in gene expression in GIV compared to N. It is also apparent that in addition to MELK (Fig. 1D, see Example 1) the following genes are markers useful for determining the staging of the disease: KIAA0101 (Fig. 1A), LOX (Fig. 1E), COL6A2 (Fig. 1G), and ASPM (Fig. 1H), in that the exhibit low p values of GIV/GI (see Table III). Figure 2 shows the relative expression of the marker genes identified here in addition to *MELK*: HOXA5 (Fig. 2A), KIAA0101 (Fig. 2B), LOX (Fig. 2C), DKFZp762E1312 (Fig. 2D), COL6A2 (Fig. 2E), PLP2 (Fig. 2F), and ASPM (Fig. 2G). The results depicted in Figure 2 that for these markers progressively higher levels of gene expression can be detected that parallels the increase in malignancy, i.e. the tumor grade.

**Table III: Statistical analysis (Mann Whitney test) comparing gene expression in Grade IV astrocytomas (GIV) vs. Grade I astrocytomas (GI) of non-neoplastic samples (N)**

| **Genes** | *p* value (Mann **GIV x N** | Whitney test) **GIV x GI** |
|---|---|---|
| **KIAA0101** | 0.0007 | 0.0031 |
| *ASPM* | 0.0006 | 0.0001 |
| *LOX* | <0.0001 | 0.006 |
| *DKFZp762E1312* | 0.0046 | 0.1161 |
| *COL6A2* | <0.0001 | <0.0001 |
| *HOXA5* | 0.0017 | 0.0425 |
| *PLP2* | 0.0002 | 0.1589 |
| *MELK* | 0.0004 | 0.0023 |
| *CD99* | <0.0001 | <0.0001 |

Figures 3-9 show the expression profiles for the markers ASPM (Fig. 3), COL6A2 (Fig. 4), DKFZp762E1312 (Fig. 5), KIAA0101 (Fig. 6), LOX (Fig. 7), HOXA5 (Fig. 8), PLP2 (Fig. 9) in normal tissue and cancer cell lines, measure by QT-PCR. ASPM (Fig. 3), KIAA0101 (Fig. 6), and HOXA5 (Fig. 8) showed restricted expression in normal tissue. ASPM (Fig. 3), KIAA0101 (Fig. 6), HOXA5 (Fig. 8), and PLP2 (Fig. 9) showed high expression levels in several distinct cancer types. DKFZp762E1312 (Fig. 5) and LOX (Fig. 7) expression is more cancer type restricted.

### Example 3: Both MELK and PLP2 are markers for the recurrence of glioblastoma

Figure 11 shows that *MELK* and *PLP2* are markers for invasiveness. Gene expression level in recurrence was measured by QT-PCR. The relative expression of PLP2 (A) and MELK (B) in sequential samples of nine patients who were submitted to two surgeries are shown. The first surgery was diagnostic and therapeutical (white) and the second intervention was because of recurrence of tumor (black). The expression level of each gene was compared to the gene expression in non-neoplastic brain samples. As shown in Figure 11 *MELK* and *PLP2* can be detected highly expressed in the second operation compared to the first, which implies that they are good invasiveness markers. This suggests that a physician operating for the first time could take samples to perform a QT-PCR to detect *MELK* and *PLP2* levels from the tissue and compare it with non-neoplastic tissue. In the case that the levels are high, the physician could then decide to subject the patient to a more aggressive treatment to potentially prevent or delay recurrence.

To confirm the elevated expression of *MELK* and *PLP2* on the protein level immunohistochemistry was performed on cancer tissue, Figure 12 shows *MELK* protein staining in recurrence using immunohistochemistry of a GIV case with polyclonal anti-MELK antibody. Panels A and B show a tumor sample from the first surgery and Panels C and D show a tumor sample from the second surgery. Panels C and D show an increase of MELK positive staining in the recurrence of the disease. Figure 13. shows *PLP2* protein staining in recurrence using immunohistochemistry of a GIV case with polyclonal anti-PLP2 antibody. Panels A and B show a tumor sample from the first surgery, and panels C and D from the second surgery. Samples A and C are from border zone of the tumor whereas B and D are from the bulk of the tumor. An increase of PLP2 positive staining can be observed in the sample from the border zone of the tumor extracted in the second surgery, C.

### Example 4: Additional experiments with the astrocytoma markers and development of 27-mer·siRNA duplexes

To test the potential oncogenic role of *ASPM*, *COL6A2*, *DKFZp762E1312, KIAA0101, LOX, HOXA5, PLP2* we studied the biological consequences of down-regulating the protein levels of these markers in glioma cell lines by siRNA-mediated knock down. For increased specificity and stability we designed highly target-specific siRNA molecules for each of the markers. The sequences are shown in Figure 26.

To test the quality of the knock down we tested our 27-mer-siRNAs to deplete marker gene expression in the U251MG glioma cell line. Figure 15A shows relative gene expression measured by QT-PCR after treating the cells with siRNAs targeting *ASPM*, *Melk*, *DKFZp762E1312, KIAA0101, LOX and PLP2.* Control siRNA used CTAG2. As can be seen from Figure 15A gene expression is significantly reduced indicating an efficient knock down using the 27-mer siRNAs. To confirm knock down on the protein level, western blot analysis of U251MG cell line treated with non-target and *MELK* (Fig. 15 B) and *PLP2* (Fig.15 C) siRNAs were performed. The numbers below the blots represent the relative fold *MELK* or *PLP2* mRNA expression, respectively, in comparison to the control CTAG2. Significant knock down can be detected also on the protein level.

To test whether gene knock down of the marker genes directly influence cellular characteristics implicated in tumorigenicity, such as migration and colony formation, in the context of glioblastoma, we used our 27-mer siRNAs to knock down several markers in glioma cell lines. Figure 16 shows the effect of targeted genes knock-down on migration using U251MG (A) and U87MG (B) glioma cell lines. The analysis and quantification of the *in vitro* migratory phenotype of U251MG and U87MG glioma cells transfected with each targeted gene or non-targeting siRNA (CTAG2, and EGFP) indicates that knock down of *LOX* and *MELK* significantly reduce the ability of these cells to migrate.

To test the effect of targeted gene knock-down on colony formation we used U251MG and A172 glioma cell lines. Figure 17 shows that knock down of *Melk, DKFZp762E1312, LOX,* and *PLP2* reduce the ability of these cells to form colonies in this clonogenic survival assay in comparison to non-targeting siRNA.

We further tested the effect of targeted gene knock-down on anchorage independent growth in soft-agar assay in the U87MG glioma cell line. Figure 18 shows that *Melk, DKFZp762E1312, LOX knockdown* significantly reduces the ability of these cells to grow in an anchorage independent manner when compared to control siRNA. Taken together these data suggest that the identified markers are important for tumorigenicity in the context of glioblastoma and provide potential therapeutic targets to reduce of suppress tumor development and tumor growth.

### Example 5: The 27-mer siRNA duplexes are useful for gene knock down in non-glioblastoma cancer cell lines

We wanted to test whether our 27-mer siRNA duplexes can be used to knock down MELK in cell lines other than those of glioblastoma. MELK, as described in Example 1, directly influences proliferation and anchorage-independent growth in other tumor contexts, and it has been recently identified as a potential therapeutic target on the basis of its over-expression in a number of cancer types⁹. Our specific 27-mer siRNA could potentially be useful as a therapeutic MELK targeting agent. To test whether the MELK-specific 27-mer siRNA was efficient to knock down *MELK* gene expression we used SK-MEL-37 and LNCaP cell lines (melanoma and prostate cancer cell lines, respectively). Figure 20 shows the knock down achieved by the MELK-specific 27-mer siRNA in SK-MEL-37 (A) and LNCaP (B) as measured by QT-PCR after treating the cells with siRNA relative to a scrambled universal negative control RNA duplex (Scrambled) which is absent in human, mouse, and rat genomes, and a positive control Dicer-Substrate RNA duplex (HPRT1) which targets a site in the *HPRT* (hypoxanthine guanine phosphoribosyltransferase 1) and is prevalidated to give >90% knockdown of *HPRT* when transfected at 10 nM concentration. Very efficient knock down could be observed.

### Example 6: Expression of HOXC9 and E2F2 is up-regulated in brain cancer stem cells and correlate with malignancy of human astrocytomas

One fundamental issue in cancer therapeutics is the elucidation of the cellular basis of the disease. Although high-throughput gene expression profiling of whole tumors and corresponding normal tissues have been applied in the search for new biomarkers for diagnosis, prognosis, and development of smart drugs, in only a few recent cases have this strategy been explored in tumorigenic stem-like cells present in solid tumors, known as cancer stem cells^{48, 49}. Nevertheless, molecular characterization of subpopulations of solid tumor cells based on their tumorigenic capabilities is lacking. Cells responsible for tumor development and recurrence were first identified in acute myeloid leukemia, considered a stem cell disease⁵⁰. More recently, subsets of cells displaying stem cell characteristics and tumorigenic capability were reported in breast⁵¹ and brain tumors^{52, 53}. In brain tumors, cancer stem cells represent 1- 25% of the tumor cell mass and are characterized by expression of the stem cell marker CD133. Hundreds of CD133+ cells are able to disseminate the tumor while tens of thousands of CD133- cells are not⁵³.

Emergent therapeutic strategies in cancer have been focusing on molecular pathways involved in neoplastic transformation. Potentially curative approaches, however, must target pathways altered in cancer stem cells so that any chance of tumor recurrence could be minimized. Here, we performed a whole genome survey to search for genes differentially expressed in CD133+ GBM cells, as compared with their non-tumorigenic CD133-counterparts, in attempt to identify molecular events associated with tumorigenesis. All brain samples used in this study were obtained after informed consent from patients who underwent surgery for tumor resection or for epilepsy (non-neoplastic controls), as approved by the institutional review boards. Tumors were classified in accordance with the World Health Organization (WHO) guidelines.

Five human GBM specimens were removed within half an hour of surgical resection and suspended in culture medium according to Yuan et al.⁵⁴, with some modifications. Tumor tissues were washed, minced, and suspended in DMEN medium containing 10% fetal calf serum and antibiotics (streptomycin, penicillin and gentamicin). Non-adherent growing cells were maintained up to three days in culture, at 37°C and 5% CO₂. Under these conditions, some GBM cells exhibited clonal expansion *in vitro* and formed small colonies known as oncospheres (Fig. 20). Such suspensions of human GBM contained 5% of viable CD133+ cells in average, as indicated by immunophenotype analysis for CD133 expression by flow cytometry (FACSAria instrument and FACSDiva software, Becton Dickinson, CA, USA).

GBM stem cells were purified from these suspensions based on positive selection of CD133 expressing cells by magnetic activated cell sorting. Thus, oncospheres were collected by centrifugation, incubated with the AC133/1 monoclonal antibody linked to magnetic microbeads, filtered through a 50-µm nylon mesh and sorted on a column subjected to a magnetic field (MiniMACS kit, Miltenyi Biotech, CA, USA). Cell viability was assessed by Trypan Blue dye exclusion. Non-neoplastic cells (PV) were also recovered from the periventricular region (temporal horn) from three patients subjected to epilepsy surgery and cultivated as for CD133+ cells.

Subsequently, gene expression profiling were carried out with CD133+, CD133-, and PV cells using high-density oligonucleotide microarrays representing 55,000 human transcripts, virtually the whole human transcriptome, following the manufacturer's protocol (CodeLink™ Human Whole Genome Bioarrays - GE Health Care, USA). Detailed descriptions of this Bioarray platform is publicly available, according to MIAME guidelines, at the GEO database under the accession number GPL2891.

Due to low yield, total RNA of CD133+ cells recovered from distinct GBM specimens were pooled to generate cRNA, as were total RNAs from distinct non-neoplastic brain tissues used as controls. All other cRNAs were individually synthesized from single total RNA samples. Prior to the microarray experiment, data reproducibility and rate of false positives were assessed by calculating the mean coefficient of variation (< 16%) and Pearson correlation coefficient (>0.98) for all data points in 30 independent hybridizations with alternative tumor and cell culture samples, each conducted in duplicates. After staining with streptavidin-conjugated Cyanine-5 dye, fluorescence was measured by an Axon GenePix 4000B Scanner, using GenePix Pro v.4.1 (Axon Instruments Inc). Light intensities were quantified, and normalized with CodeLink Software v.4.1 (GE Health care, USA). Spots with any background contamination, shape irregularity, or pixel saturation were filtered out.

Genes with similar expression profiles across samples were grouped by hierarchical clustering with VectorXpression (Informax Inc, USA), using an unsupervised analysis and correlation coefficient metrics. Differentially expressed genes were identified according to the HTself method⁵⁵, which takes advantage of self-self experiments to define an intensity-dependent fold-change cutoff. Since our microarray system is a one-color platform, self-self hybridizations were built by combining two replicates of actual experimental hybridizations. Therefore, genes were considered as differentially expressed if all its replicates were above (up-regulated) or below (down-regulated) the dynamic cutoff level defined by these self-self experiments, with a 99% credibility level.

Under these conditions, microarray analysis comparing directly the transcriptomes of CD133+ (tumorigenic) and CD133- (non-tumorigenic) cells against each other revealed a group of seven genes consistently up-regulated and 10 genes consistently down-regulated in the CD133+ subset. This group of 17 genes might be considered as a molecular signature for CD133+ GBM cells since all of them were also found as differentially expressed, in the same fashion, when comparing CD133+ cells against non-neoplastic controls, whereas similar differences in gene expression were not observed in the CD133- cells versus non-neoplastic control comparisons (Fig. 21). The description, corresponding Genbank accession numbers, and average differential expression values of these 17 genes are summarized in Table IX. Based on their current functional classification, no particular biological process was found over-represented and five genes are of unknown function.

**Table IX. Genes differentially expressed in CD133+ cells relative to CD133- cells from human glioblastomas.¹ ("Astrocytoma stem cell markers")**

| **Accession #** | **Description** | **Function** | **Fold change 133+ vs. 133-** |
|---|---|---|---|
| ***ClusterA*** | | | |
| NM_152745 | neurexophilin 1 (NXPH1) | signal transduction | 4.70 |
| NM_018934 | protocadherin beta 14 (PCDHB14) | cell-cell adhesion / synaptogenesis | 13.84 |
| NM_003275 | tropomodulin 1 (TMOD1) | cell organization and biogenesis | 49.72 |
| NM_006897 | homeo box C9 (HOXC9) | regulation of transcription / development | 69.37 |
| NM_004091 | E2F transcription factor 2 (E2F2) | regulation of transcription / cell cycle | 47.61 |
| BX439624 | hypothetical LOC151261 | unknown | 9.26 |
| NM_002414.3 | CD99 antigen (CD99) | cell adhesion | 3.95 |
| ***Cluster B*** | | | |
| NM_005019 | phosphodiesterase 1a, calmodulin-dependent | signal transduction | -25.70 |
| W72042 | ptprf interacting protein, binding protein 2 | nucleic acid metabolism | -8.69 |
| NM_018842 | bai1-associated protein 2-like 1 | unknown | -10.16 |
| NM_003246 | thrombospondin 1 | cell adhesion and motility | -48.16 |
| BM805926 | solute carrier family 45, member 1 dishevelled associated activator of | ion transport | -25.65 |
| NM_015345 | morphogenesis 2 NCI_CGAP_Ut1 cDNA clone | cytoskeleton organization and biogenesis | -10.07 |
| AI272963 | IMAGp998M154881 | unknown | -7.18 |
| T03803 | similar to galectin-9 | unknown | -7.40 |
| NM_025193 | hydroxy-delta-5-steroid dehydrogenase | steroid hormone biosynthesis | -7.91 |
| AK001351 | hypothetical protein flj10489 | unknown | -6.28 |

| | | | |
|---|---|---|---|
| ¹Cluster A and B genes refer to those up regulated or down regulated in CD133+ cells, respectively, as indicated in Figure 8. Functional classification of genes was based on the Gene Ontology annotation. Values represent mean fold changes (P < 0.01). | | | |

Different groups have identified CD133+ cells as a sub-population of multipotent GBM cells capable of self-renewal and differentiation into neuronal and glial cell lineages *in vitro*^{54, 56}. Interestingly, after serial transplantation *in vivo*, these cells behave as regenerating cells, establishing and propagating tumors with phenotypic and histologic features closely resembling the original tumor^{53, 56} Overall, these findings have supported the hypothesis of normal adult stem cells as targets for neoplastic transformation⁵⁷.

Indeed, adult stem cells are primitive long-lived cells that may well accumulate stepwise deleterious genetic alterations leading to cancer. In addition, gliomas are often localized in the subventricular and periventricular zones of the brain, germinal regions containing neural stem cells and progenitors⁵⁸. As shown in Fig. 21, the fact that none of the 17 genes comprising the CD133+ GBM cell molecular signature displayed consistent differential expression in normal periventricular cells, compared to non-neoplastic controls, strengthens a possible involvement of such genes with the tumorigenic process rather than with stem/progenitor properties.

Two genes specifically up-regulated in CD133+ cells, *HOXC9* and *E2F2,* encode transcription factors belonging to conserved gene families with well known roles in the control of cell proliferation, differentiation, apoptosis, and development^{59, 60}. Yet, specific information on the expression of these two members in gliomas is limited. Due to their regulatory function, constitutive alterations in *HOXC9* and *E2F2* expression could directly affect cellular processes implicated in the malignancy of astrocytomas.

To address this issue, a quantitative real-time PCR analysis was performed in a set of 54 primary astrocytomas of different grades (16 pilocytic astrocytomas - WHO grade I, 19 anaplastic astrocytomas - WHO grade II, and 19 glioblastomas - WHO grade IV) and 16 non-neoplastic brain samples. After surgical removal, samples were snap-frozen and stored in liquid nitrogen for RNA extraction by the Trizol method (Invitrogen). Tissue sections (5 µm thick) were obtained with a cryostat at -25°C for histological assessment by light microscopy after hematoxylin-eosin staining. Areas of necrosis or adjacent non-neoplastic tissue were removed from the frozen block. Normal tissues were carefully analyzed to avoid gray matter and, consequently, neuron-related expressing genes.

The RNA integrity of samples was evaluated based on the intensity of 28S and 18S rRNA bands on agarose gels and by RT-PCR of beta-actin, *ACTB*, and *GAPDH* genes. Quantitative real-time PCR assays were carried out in duplicates by the SYBR-Green approach in the ABI 7000 Sequence Detection System. Standard curves were established following serial sample dilutions and data normalized based on expression of the housekeeping gene hypoxanthine phosphoribosyltransferase (*HPRT*). The sequences of primers were: *HOXC9* (Forward: 5' CCGGCAGCAAGCACAAAGA 3' (SEQ ID NO: 23); Reverse: 5' CGGTGAGATTGAGAACCCG 3' (SEQ ID NO: 24)), *E2F2* (Forward: 5'
GTCCTTCCGAGGAGCCTCT 3' (SEQ ID NO: 25); Reverse: 5'
GGGACAGGAACTGGTCCTC 3' (SEQ ID NO: 26)), and *HPRT* (Forward: 5'
TGAGGATTTGGAAAGGGTGT 3' (SEQ ID NO: 13); Reverse: 5'
GAGCACACAGAGGGCTACAA 3' (SEQ ID NO: 14)).

Higher expression levels of *HOXC9* and *E2F2* were found for both GBM and grade II astrocytomas, relative to grade I astrocytomas. However, none of the 16 non-neoplastic control samples tested had detectable expression of *HOXC9*, while a lower expression of *E2F2* could be detected in only one control specimen (Fig. 22).

Notably, apart from the magnitude of expression, when analyzing the frequency of detectable gene expression per tumor sample (presence or absence), we found that *HOXC9* and *E2F2* expression are not ubiquitous in astrocytomas grades I or II. Conversely, expression of both genes could be consistently detected, in high levels, in 100% of the GBM samples tested. A strong association between detection of *HOXC9* and *E2F2* expression with malignancy of astrocytomas was indicated by the Goodman and Kruskal's gamma test. In this statistical test, perfect association of two parameters is given by a gamma value equal to 1. As indicated in Fig. 22, the gamma values obtained for *HOXC9* and *E2F2* were 0.818 and 0.798, respectively.

These findings are consistent with the involvement of *E2F* and *HOX* gene families with carcinogenesis and suggest a potential relevance of these particular members in the development of high-grade astrocytomas. Indeed, the pRb/E2F pathway is deregulated in many cancers. In human glioblastoma cell lines, E2F2 has been shown to transactivate promoter elements and induce *Bcl-2* expression⁶¹. E2F2 is also capable of transcriptionally up-regulate the expression of *survivin* and *MYCN* genes, which are associated with tumors of poor prognosis^{62, 63}. These antiapoptotic and proliferative effects of E2F2 might contribute to the highly aggressive and invasive growth characteristic of GBM tumors.

Likewise, transcription factors of the class I homeobox family have been found overexpressed in many solid tumors and implicated in cancer development⁶⁴. In brain cancers, aberrant expression of *HOXB3*, *B4* and *C6* occur in childhood medulloblastomas and primitive neuroectodermal tumors⁶⁵ while *HOXA6*, *A7*, *A9*, *A13*, *B13*, *D4*, *D9*, *D10*, and *D13* are overexpressed in GBM⁶⁶. Information on *HOXC9* in human cancers is rather poor and association of its expression with malignancy of astrocytomas is first reported here. Similar to our findings, in cervical cancer, *HOXC9* expression was reported to be detected in tumors but not in non-neoplastic tissue⁶⁷, suggesting that this particular *HOX* family member may be relevant to the process of cellular transformation.

In conclusion, our comparative gene expression profiling of cellular subpopulations purified from human GBM identified many genes not previously associated with astrocytomas, with an aberrant expression in CD133+ cells, but not in CD133- cells, when compared with corresponding non-neoplastic controls. Two of such genes, *E2F2* and *HOXC9*, encode transcription factors with established roles in cell proliferation and differentiation. While normal brain tissues virtually lack expression of *E2F2* and *HOXC9,* up-regulation of both genes was detected in astrocytomas of different grades, predominantly in GBM. Frequency of *HOXC9* and *E2F2* expression correlated significantly with malignancy of astrocytomas, indicative of putative roles in GBM development. Further functional studies are currently under investigation to address the relevance of these genes to the tumorigenic properties of brain cancer stem cells and their usefulness as targets for the development of new anticancer therapies.

The expression of CD99 antigen in astrocytomas was confirmed by QT-PCR as described herein. To determine the relationship between expression level of CD99 protein and malignancy a total of 19 non-neoplastic brain tissue (N), and 97 astrocytic tumor samples were used (18 pilocytic astrocytomas (GI), 18 low grade astrocytomas (GII), 18 anaplastic astrocytomas (GIII), and 46 Glioblastomas (GIV) were analyzed by QT-PCR. Normalization of quantitative data was based on the housekeeping genes: hypoxanthine guanine phosphoribosyl transferase gene (*HPRT*), glucuronidase beta gene (*GUSB*), and breast cancer resistance protein gene (*BCRP*). The results are depicted in Figure 23. Just like the markers described earlier, CD99 is a good stem cell marker for glioblastoma and the expression profile changes according the tumor grade, suggesting that *CD99* can also be used to determine the tumor stage. Figure 24 shows the relative expression levels for *CD99* in normal tissues.

### References

43. A.K. Karak, R. Singh, P.N. Tandon, C. Sarkar, A comparative survival evaluation and assessment of interclassification concordance in adult supratentorial astrocytic tumors, Pathol. Oncol. Res. 6 (2000), pp. 46-52.
44. K. Boon, J.B. Edwards, C.G. Eberhart, G.J. Riggins, Identification of astrocytoma associated genes including cell surface markers, BMC Cancer 4 (2004), pp. 39.
45. K.K. Wong, Y.M. Chang, Y.T. Tsang, L. Perlaky, J. Su, A. Adesina, D.L. Armstrong, M . Bhattacharjee, R. Dauser, S.M. Blaney, M. Chintagumpala, C.C. Lau, Expression analysis of juvenile pilocytic astrocytomas by oligonucleotide microarray reveals two potential subgroups, Cancer Res. 65 (2005), pp. 76-84.
46. M.G. McCabe, K. Ichimura, L. Liu, K. Plant, L.M. Backlund, D.M. Pearson, V.P. Collins, High-resolution array-based comparative genomic hybridization of medulloblastomas and supratentorial primitive neuroectodermal tumors, J. Neuropathol. Exp. Neurol. 65 (2006), pp. 549-561.
47. R. Yamanaka, T. Arao, N. Yajima, N. Tsuchiya, J. Homma, R. Tanaka, M. Sano, A. Oide, M. Sekijima, K. Nishio, Identification of expressed genes characterizing long-term survival in malignant glioma patients, Oncogene 44 (2006), pp. 5994-6002.
48. S.K. Kang, J.B. Park, S.H. Cha, Multipotent, dedifferentiated cancer stem-like cells from brain gliomas, Stem Cells Dev. 15 (2006), pp. 423-435.
49. J. Lee, S. Kotliarova, Y. Kotliarov, A. Li, Q. Su, N.M. Donin, S. Pastorino, B.W. Purow, N. Christopher, W. Zhang, J.K. Park, H.A. Fine, Tumor stem cells derived from glioblastomas cultured in bFGF and EGF more closely mirror the phenotype and genotype of primary tumors than do serum-cultured cell lines, Cancer Cell 9 (2006), pp. 391-403.
50. D. Bonnet, J.E. Dick, Human acute myeloid leukemia is organized as a hierarchy that originates from a primitive hematopoietic cell, Nat. Med. 3 (1997) (7), pp. 730-737.
51. M. Al-Hajj, M.S. Wicha, A. Benito-Hernandez, S.J. Morrison, M.F. Clarke, Prospective identification of tumorigenic breast cancer cells, Proc. Natl. Acad. Sci. USA 100 (2003), pp. 3983-3988.
52. H.D. Hemmati, 1. Nakano, J.A. Lazareff, M. Masterman-Smith, D.H. Geschwind, M. Bronner-Fraser, H.I. Kornblum, Cancerous stem cells can arise from pediatric brain tumors, Proc. Natl. Acad. Sci. USA 100 (2003), pp.:15178-15183.
53. S.K. Singh, I.D. Clarke, M. Terasaki, V.E. Bonn, C. Hawkins, J. Squire, P.B. Dirks, Identification of a cancer stem cell in human brain tumors, Cancer Res. 15 (2003) (18), pp. 5821-5828.
54. X. Yuan, J. Curtin, Y. Xiong, G. Liu, S. Waschsmann-Hogiu, D.L. Farkas, K.L. Black, J.S. Yu, Isolation of cancer stem cells from adult glioblastoma multiforme, Oncogene 16 (2004), pp. 9392-9400.
55. R.Z. Vencio, T. Koide, HTself: self-self based statistical test for low replication microarray studies. DNA Res. 12 (2005), pp. 211-214.
56. R. Galli, E. Binda, U. Orfanelli, B. Cipelletti, A. Gritti, S. De Vitis, R. Fiocco, C. Foroni, F. Dimeco, A. Vescovi, Isolation and characterization of tumorigenic, stem-like neural precursors from human glioblastoma, Cancer Res. 64 (2004) (19), pp. 7011-7021.
57. R.J. Gilbertson, Brain tumors provide new clues to the source of cancer stem cells: does oncology recapitulate ontogeny? Cell Cycle 5 (2006), pp. 135-137.
58. P. Taupin, Neurogenesis in the adult central nervous system. C. R. Biol. 329 (2006) (7), pp. 465-475.
59. R. Krumlauf, Hox genes in vertebrate development, Cell 2 (1994), pp. 191-201.
60. H. Muller, A.P. Bracken, R. Vemell, M.C. Moroni, F. Christians and E. Grassilli et al., E2Fs regulate the expression of genes involved in differentiation, development, proliferation, and apoptosis, Genes Dev. 3 (2001), pp. 267-285.
61. C. Gomez-Manzano, P. Mitlianga, J. Fueyo, H.Y. Lee, M. Hu, K.B. Spurgers, T.L. Glass, D. Koul, T.J. Liu, T.J. McDonnell, W.K. Yung, Transfer of E2F-1 to human glioma cells results in transcriptional up-regulation of Bcl-2. Cancer Res. 18 (2001), pp. 6693-6697.
62. V. Strieder, W. Lutz, E2F proteins regulate MYCN expression in neuroblastomas, J. Biol. Chem. 278 (2003), pp. 2983-2989.
63. Y. Jiang, H.I. Saavedra, M.P. Holloway, G. Leone, R.A. Altura, Aberrant regulation of survivin by the RB/E2F family of proteins, J. Biol. Chem. 279 (2004), pp. 40511-40520.
64. R.W. Redline, P. Hudock, M. MacFee, P. Patterson, Expression of AbdB-type homeobox genes in human tumors, Lab. Invest. 71 (1994), pp. 663-670.
65. B. Bodey, B. Bodey Jr, S.E. Siegel, H.E. Kaiser, Immunocytochemical detection of the homeobox B3, B4, and C6 gene products in childhood medulloblastomas/primitive neuroectodermal tumors, Anticancer Res. 20 (2000), pp.1769-1780.
66. R. Abdel-Fattah, A. Xiao, D. Bomgardner, C.S. Pease, M.B. Lopes, I.M., Hussaini, Differential expression of HOX genes in neoplastic and non-neoplastic human astrocytes. J. Pathol. 209 (2006), pp. 15-24.
67. R. Lopez, E. Garrido, G. Vazquez, P. Pina, C. Perez, I. Alvarado, M. Salcedo, A subgroup of HOX Abd-B gene is differentially expressed in cervical cancer, Int. J. Gynecol. Cancer 16 (2006), pp. 1289-1296.

Those skilled in the art will recognize, or be able to ascertain using no more than routine experimentation, many equivalents to the specific embodiments of the invention described herein. Such equivalents are intended to be encompassed by the following claims.

All references disclosed herein are incorporated by reference in their entirety for the purposes specified above.

## Claims

1. A method of diagnosing an astrocytoma in a subject, comprising:
determining a level of a LOX gene product in a biological sample isolated from the subject, and
comparing the level in the sample to a reference/control level, wherein if the level in the sample is higher than the reference/control level, then the subject is indicated as having an astrocytoma.

2. A method of staging an astrocytoma in a subject, comprising:
determining a level of a LOX gene product in a sample of the astrocytoma tumor isolated from the subject, and
comparing the level of the gene product in the sample to a reference/control level to determine that the tumor is an astrocytoma of a particular stage.

3. The method of claim 1 or claim 2, wherein the gene product is a mRNA.

4. The method of claim 3, wherein the level of gene product in the sample is determined by nucleic acid amplification, or by a nucleic acid hybridization method.

5. The method of claim 4, wherein the nucleic acid amplification is polymerase chain reaction (PCR), reverse transcribed PCR (RT-PCR), real-time RT-PCR, or quantitative real-time RT-PCR.

6. The method of claim 4, wherein the nucleic acid hybridization method is a microarray or a membrane hybridization method.

7. The method of claim 1 or claim 2, wherein the gene product is a polypeptide.

8. The method of claims 1, 2 or 7, wherein the level of gene product in the sample is determined by a polypeptide binding method.

9. The method of claim 8, wherein the polypeptide binding method is an immunological detection method.

10. The method of claim 9, wherein the immunological detection method uses an antibody or an antigen binding fragment thereof.

11. The method of any of claims 1-10, wherein the difference between the level of the gene product in the sample and the reference/control level is at least about 1%, 2%, 3%, 5%, 10%, 15%, 20%, 25%, 30%, 35%, 40%, 50%, 60%, 70%, 80%, 90%, 100%, 110%, 120%, 130%, 140%, 150%, 175%, 200%, 250%, 300%, 350%, 400%, 450%, 500%, 600%, 700%, 800%, 900%, or 1000%.

12. The method of any of claims 1-11, wherein the sample is a body fluid, a body effusion, cell or a tissue.

13. A compound or composition for use in treating astrocytoma, wherein the compound or composition reduces the level or biological activity of a LOX gene product, or binds a LOX polypeptide.

14. The compound or composition of claim 13, wherein the astrocytoma is a pilocytic astrocytoma (PA; grade I), low grade astrocytoma (grade II), anaplastic astrocytoma (grade III) and glioblastoma multiforme (GBM; grade IV).

15. The compound or composition of claim 13, wherein the compound or composition is an antibody or an antigen-binding fragment thereof.

16. The compound or composition of any of claims 13-15, wherein, when the compound or composition binds a LOX polypeptide, the compound or composition reduces the activity or function of the polypeptide.

17. The compound or composition of claim 13, wherein, when the compound or composition reduces the level or biological activity of a LOX gene product, the compound or composition is a RNAi molecule or antisense molecule.

18. The compound or composition of claims 13, 14 or 17, wherein, when the compound or composition reduces the level or biological activity of a LOX gene product, the gene product is a mRNA or a polypeptide.


SEQUENCE LISTING

<110>  Ludwig Institute for Cancer Research

<120>  METHODS FOR DIAGNOSING AND TREATING ASTROCYTOMAS

<130>  L0461.70171WO00

<150>  US 60/926263
<151>  2007-04-26

<160>  53

<170>  PatentIn version 3.3

<210>  1
<211>  24
<212>  DNA
<213>  Artificial Sequence

<220>
<223>  Synthetic Oligonucleotide

<400>  1
ctgccatggg aagaaggtga ttca                                              24


<210>  2
<211>  22
<212>  DNA
<213>  Artificial Sequence

<220>
<223>  Synthetic Oligonucleotide

<400>  2
gatgcggcga taggtctcgt tg                                                22


<210>  3
<211>  18
<212>  DNA
<213>  Artificial Sequence

<220>
<223>  Synthetic Oligonucleotide

<400>  3
tgtggagtgc gccttgtg                                                     18


<210>  4
<211>  20
<212>  DNA
<213>  Artificial Sequence

<220>
<223>  Synthetic Oligonucleotide

<400>  4
tgccggaatc ctgagtttct                                                   20


<210>  5
<211>  19
<212>  DNA
<213>  Artificial Sequence

<220>
<223>  Synthetic Oligonucleotide

<400>  5
cctgccctac tgccaaaga                                                    19


<210>  6
<211>  27
<212>  DNA
<213>  Artificial Sequence

<220>
<223>  Synthetic Oligonucleotide

<400>  6
cagaactgaa cacaatttaa caaagct                                           27


<210>  7
<211>  22
<212>  DNA
<213>  Artificial Sequence

<220>
<223>  Synthetic Oligonucleotide

<400>  7
gtttgaacat ggtgcggact aa                                                22


<210>  8
<211>  20
<212>  DNA
<213>  Artificial Sequence

<220>
<223>  Synthetic Oligonucleotide

<400>  8
ggcagaggtg gaagaaccaa                                                   20


<210>  9
<211>  20
<212>  DNA
<213>  Artificial Sequence

<220>
<223>  Synthetic Oligonucleotide

<400>  9
aaacccaagg gtaacaagga                                                   20


<210>  10
<211>  20
<212>  DNA
<213>  Artificial Sequence

<220>
<223>  Synthetic Oligonucleotide

<400>  10
acagtatgcc catgctccaa                                                   20


<210>  11

<211>  22
<212>  DNA
<213>  Artificial Sequence

<220>
<223>  Synthetic Oligonucleotide

<400>  11
gacctgcaca ccaagatacc at                                                22


<210>  12
<211>  22
<212>  DNA
<213>  Artificial Sequence

<220>
<223>  Synthetic Oligonucleotide

<400>  12
caaggacaac aatggaggtg at                                                22


<210>  13
<211>  20
<212>  DNA
<213>  Artificial Sequence

<220>
<223>  Synthetic Oligonucleotide

<400>  13
tgaggatttg gaaagggtgt                                                   20


<210>  14
<211>  20
<212>  DNA
<213>  Artificial Sequence

<220>
<223>  Synthetic Oligonucleotide

<400>  14
gagcacacag agggctacaa                                                   20


<210>  15
<211>  20
<212>  DNA
<213>  Artificial Sequence

<220>
<223>  Synthetic Oligonucleotide

<400>  15
gctttgcgac aattctgtga                                                   20


<210>  16
<211>  20
<212>  DNA
<213>  Artificial Sequence

<220>
<223>  Synthetic Oligonucleotide

<400>  16

acagtatgcc catgctccaa                                                    20

<210>  17
<211>  19
<212>  DNA
<213>  Artificial Sequence

<220>
<223>  Synthetic Oligonucleotide

<400>  17
gtgaaggctc atggcaaga                                                     19

<210>  18
<211>  21
<212>  DNA
<213>  Artificial Sequence

<220>
<223>  Synthetic Oligonucleotide

<400>  18
agctcactca gtgtggcaaa g                                                  21

<210>  19
<211>  24
<212>  DNA
<213>  Artificial Sequence

<220>
<223>  Synthetic Oligonucleotide

<400>  19
gggtaagatt aaggttttaa gtta                                               24

<210>  20
<211>  20
<212>  DNA
<213>  Artificial Sequence

<220>
<223>  Synthetic Oligonucleotide

<400>  20
cttaacctaa acacctcttc                                                    20

<210>  21
<211>  24
<212>  DNA
<213>  Artificial Sequence

<220>
<223>  Synthetic Oligonucleotide

<400>  21
agattaaggt tttaagttat tttt                                               24

<210>  22
<211>  27
<212>  DNA
<213>  Artificial Sequence

<220>
<223>  Synthetic Oligonucleotide

<400>  22
aaaaccaaaa acaaactaaa tttaaac                                          27


<210>  23
<211>  19
<212>  DNA
<213>  Artificial Sequence

<220>
<223>  Synthetic Oligonucleotide

<400>  23
ccggcagcaa gcacaaaga                                                   19


<210>  24
<211>  19
<212>  DNA
<213>  Artificial Sequence

<220>
<223>  Synthetic Oligonucleotide

<400>  24
cggtgagatt gagaacccg                                                   19


<210>  25
<211>  19
<212>  DNA
<213>  Artificial Sequence

<220>
<223>  Synthetic Oligonucleotide

<400>  25
gtccttccga ggagcctct                                                   19


<210>  26
<211>  19
<212>  DNA
<213>  Artificial Sequence

<220>
<223>  Synthetic Oligonucleotide

<400>  26
gggacaggaa ctggtcctc                                                   19


<210>  27
<211>  22
<212>  DNA
<213>  Artificial Sequence

<220>
<223>  Synthetic Oligonucleotide

<400>  27
tggctgttgt cctacaatcc tt                                               22


<210>  28

<211>  23
<212>  DNA
<213>  Artificial Sequence

<220>
<223>  Synthetic Oligonucleotide

<400>  28
aacatagcca accctgtgac atc                                              23


<210>  29
<211>  20
<212>  DNA
<213>  Artificial Sequence

<220>
<223>  Synthetic Oligonucleotide

<400>  29
ctgacctacg agacgccaca                                                  20


<210>  30
<211>  17
<212>  DNA
<213>  Artificial Sequence

<220>
<223>  Synthetic Oligonucleotide

<400>  30
gcacggagcc atctgtc                                                     17


<210>  31
<211>  22
<212>  DNA
<213>  Artificial Sequence

<220>
<223>  Synthetic Oligonucleotide

<400>  31
gttcctggct tcaaaggaga ga                                               22


<210>  32
<211>  19
<212>  DNA
<213>  Artificial Sequence

<220>
<223>  Synthetic Oligonucleotide

<400>  32
gtccatcggt cccgttctt                                                   19


<210>  33
<211>  20
<212>  DNA
<213>  Artificial Sequence

<220>
<223>  Synthetic Oligonucleotide

<400>  33

atgcgcaagc tgcacataag                                                20

<210>  34
<211>  19
<212>  DNA
<213>  Artificial Sequence

<220>
<223>  Synthetic Oligonucleotide

<400>  34
gggtctggta gcgcgtgta                                                 19

<210>  35
<211>  18
<212>  DNA
<213>  Artificial Sequence

<220>
<223>  Synthetic Oligonucleotide

<400>  35
gattgtgggg gctgtcgt                                                  18

<210>  36
<211>  21
<212>  DNA
<213>  Artificial Sequence

<220>
<223>  Synthetic Oligonucleotide

<400>  36
cacctcccct tgttctgcat t                                              21

<210>  37
<211>  25
<212>  DNA
<213>  Artificial Sequence

<220>
<223>  Synthetic Oligonucleotide

<400>  37
gaaaatacgt ggttggagag ctcat                                          25

<210>  38
<211>  22
<212>  DNA
<213>  Artificial Sequence

<220>
<223>  Synthetic Oligonucleotide

<400>  38
   ccgagtgaag atcccctttt ta                                            22

<210>  39
<211>  23
<212>  DNA
<213>  Artificial Sequence

<220>
<223>  Synthetic Oligonucleotide

<400>  39
ccttcgacgt caataacaag gat                                              23


<210>  40
<211>  17
<212>  DNA
<213>  Artificial Sequence

<220>
<223>  Synthetic Oligonucleotide

<400>  40
cctgcgatgg cgttcac                                                     17


<210>  41
<211>  27
<212>  RNA
<213>  Artificial Sequence

<220>
<223>  Synthetic Oligonucleotide

<400>  41
uugcaaagga caugcucuuu ccucgau                                          27


<210>  42
<211>  27
<212>  RNA
<213>  Artificial Sequence

<220>
<223>  Synthetic Oligonucleotide

<400>  42
uuccuaagcc acugcuuccu gccucuu                                          27


<210>  43
<211>  27
<212>  RNA
<213>  Artificial Sequence

<220>
<223>  Synthetic Oligonucleotide

<400>  43
uccacuuggg ucaaguauuu ccuucuu                                          27


<210>  44
<211>  27
<212>  RNA
<213>  Artificial Sequence

<220>
<223>  Synthetic Oligonucleotide

<400>  44
ugugccauuc ccaggaauau cuugguc                                          27


<210>  45

<211>  27
<212>  RNA
<213>  Artificial Sequence

<220>
<223>  Synthetic Oligonucleotide

<400>  45
ggcuggaguc ccuggugccu cggagga                                          27


<210>  46
<211>  27
<212>  RNA
<213>  Artificial Sequence

<220>
<223>  Synthetic Oligonucleotide

<400>  46
gagggaaauc acagcugccu uugucuu                                          27


<210>  47
<211>  27
<212>  RNA
<213>  Artificial Sequence

<220>
<223>  Synthetic Oligonucleotide

<400>  47
guauaaagcc auuacauuau caucauc                                          27


<210>  48
<211>  27
<212>  RNA
<213>  Artificial Sequence

<220>
<223>  Synthetic Oligonucleotide

<400>  48
acauauaugc ugaugucuca gguucuu                                          27


<210>  49
<211>  27
<212>  RNA
<213>  Artificial Sequence

<220>
<223>  Synthetic Oligonucleotide

<400>  49
ccgaacugac auaaacagua gguugcc                                          27


<210>  50
<211>  27
<212>  RNA
<213>  Artificial Sequence

<220>
<223>  Synthetic Oligonucleotide

<400>  50

aauuucaaau ccaacaaagu cuggcuu 27

<210> 51
<211> 27
<212> RNA
<213> Artificial Sequence

<220>
<223> Synthetic Oligonucleotide

<400> 51
ucacaauuua uauaaauaua uuaauua 27

<210> 52
<211> 21
<212> DNA
<213> Artificial Sequence

<220>
<223> Synthetic Oligonucleotide

<400> 52
cctactacat ccaggcgtcc a 21

<210> 53
<211> 24
<212> DNA
<213> Artificial Sequence

<220>
<223> Synthetic Oligonucleotide

<400> 53
cataatctct gacatctgcc ctgt 24